# FASCICULE DE BREVET EUROPEEN

(11) **EP 2 382 215 B1**
(45) Date de publication et mention de la délivrance du brevet: **16.07.2014**
(21) Numéro de dépôt: 09805790.4
(22) Date de dépôt: 24.12.2009
(51) Int. Cl.: C07D 471/04, C07D 491/04, C07D 513/04, A61K 31/4162, A61P 27/06, C07D 213/72, C07D 213/77, C07D 401/12, C07D 405/12, C07D 409/12, C07D 403/04, C07D 495/04

(54) **DÉRIVÉS DE 2-PYRIDIN-2-YL-PYRAZOL-3(2H)-ONE, LEUR PRÉPARATION ET LEUR APPLICATION EN THÉRAPEUTIQUE**
DERIVATE VON 2-PYRIDIN-2-YLPYRAZOL-3(2H)-ON, DEREN HERSTELLUNG UND DEREN THERAPEUTISCHE VERWENDUNG
DERIVATIVES OF 2-PYRIDIN-2-YL-PYRAZOL-3(2H)-ONE, PREPARATION AND THERAPEUTIC USE THEREOF

(30) Priorité: 29.12.2008 FR 0807475; 28.08.2009 FR 0904091
(43) Date de publication de la demande: 02.11.2011
(73) Titulaire: SANOFI, 75008 Paris (FR)
(72) Inventeur: ALTENBURGER, Jean-Michel, F-75013 Paris (FR); FOSSEY, Valérie, F-75013 Paris (FR); ILLIANO, Stéphane, F-75013 Paris (FR); MANETTE, Géraldine, F-75013 Paris (FR)
(74) Mandataire: Romanowski, Caroline
(86) Numéro de dépôt international: PCT/FR2009/052692
(87) Numéro de publication internationale: WO 2010/076525

(56) Documents cités:
- WO-A-2007/103905
- WO-A-2008/047198
- WO-A-2008/141731
- US-A- 5 750 088
- US-A1- 2005 187 276

## Description

La présente invention a pour objet des nouveaux dérivés de dihydropyrazolone substituées, leur préparation et leur application en thérapeutique en tant qu'activateurs du facteur de transcription HIF.

Le facteur induit par l'hypoxie (HIF pour « Hypoxia Inducible Factor ») (HIF1-alpha) est un facteur de transcription exprimé de façon constitutive dans tous les tissus. Cette protéine a été découverte en 1994 par Gregg Semenza lors de l'étude des séquences régulatrices du gène de l'EPO. Il a identifié une séquence située en position 3' non-codante dans le promoteur de l'EPO, qui porte le nom d'« hypoxia response element » (HRE) et qui est un site de fixation de la protéine HIF1-alpha permettant l'activation transcriptionnelle de l'EPO. Par la suite, la séquence HRE a aussi été localisée sur plus de 70 autres gènes, tels que le VEGF (vascular endothelial growth factor) ou Glut1 (glucose transporter 1) Le complexe transcriptionnel HIF-1 est à minima un hétérodimère constitué de la protéine HIF1-alpha ou HIF2-alpha et d'un autre facteur de transcription ARNT (anciennement nommé HIF1-alpha). ARNT est exprimé de façon constitutive et stable dans les cellules et l'essentiel de la régulation du complexe transcriptionnel est lié à la quantité de HIF1-alpha présent dans les cellules et qui est donc le facteur limitant.

Dans des conditions normales en oxygène la protéine HIF1-alpha est rapidement dégradée (demi-vie de 5 minutes). Cette dégradation est consécutive à l'hydroxylation de HIF1-alpha ou de HIF2-alpha respectivement sur les Prolines 402 et 563 et les Prolines 405 et 531 pour les formes humaines par les HIF Prolyl Hydroxylase (HIF_PHDs ou EGLNs). Cette hydroxylation permet la liaison de la protéine de Von Hippell Lindau (pVHL) associée à une ubiquitine ligase, ce qui va entrainer la dégradation de HIF1-alpha ou HIF2-alpha par le système ubiquitine protéasome. Lorsque la cellule ou le tissu sont soumis à une hypoxie/ischémie, HIF1-alpha ou HIF2-alpha ne sont plus dégradés par le système ubiquitine-protéasome et peuvent alors s'associer avec les autres facteurs de transcription du complexe HIF pour basculer dans le noyau et activer leurs gènes cibles.

Bien que l'hypoxie soit la principale cause de l'activation des protéines HIF1-alpha et HIF2-alpha d'autres inducteurs, tels que l'insuline, les facteurs de croissance peuvent aussi jouer un rôle dans leur stabilisation notamment via la phosphorylation sur les Serines 641 et 643.

Un criblage phénotypique visant à mesurer la stabilisation de la protéine HIF1-alpha et/ou HIF2-alpha a donc été établi pour identifier les composés de la présente invention.

WO2008/047198 décrit des dérivés pyrazol-pyridines utilisés dans le traitement inhibant TGF-beta et notamment pour inhiber la formation d'escarres. US5,750,088 décrit des dérivés d'hydrazone liés à un groupe biologique peptidique actif utilisés en radiopharmacologie. WO2007/103905 décrit des dérivés bicycliques hétéroaromatiques N-glycine substitués utilisés dans le traitement de maladies nécessitant l'inhibition de l'enzyme prolyl hydroxylase régulant HIF.

Les composés selon la présente invention répondent à la formule (I) suivante: 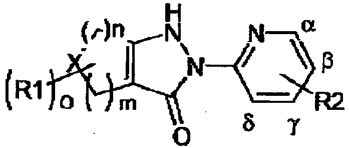 dans laquelle
**n** est égal à 0, 1, 2, 3 ou 4;
**m** est égal à 0, 1 ou 2 ;
**o** est égal à 0 ou 1 ;
**X** représente un groupe -CH₂, -CH(R')-, -N(R')- ou un hétéroatome choisi parmi un atome d'oxygène et un atome de soufre, étant entendu que R' représente un groupe - (C1-C5)alkyle, -(C1-C5)alcoxy, -CH₂-aryle, -C(O)R5 ou -COOR5 avec R5 tel que défini ci-dessous ;
**R1** représente un groupe oxo, un groupe -COOR5, un groupe -W-OH ou un groupe-W-NR5R6, avec W, R5 et R6 tels que définis ci-dessous ; et
**R2** représente un atome d'hydrogène ou un groupe choisi parmi (i) les groupes -(C1-C5)alkyle, (ii) les groupes -(C1-C5)alcoxy, (iïi) les groupes -COOR5, (iv) les groupes-NR5R6, (v) les groupes -C(O)-NR5R6, (vi) les groupes -SO₂-NR3R4, (vii) les groupes hétéroaryle eventuellement substitués par un groupe -(C1-C5)alkyle, (viii) les groupes-W-aryle, (ix) les groupes -W-hétéroaryle, (x) les groupes -O-W-aryle, (xi) les groupes -O-W-hétéroaryle et (xii) les groupes -O-W-NR5R6, avec W, R3, R4, R5 et R6 tels que définis ci-dessous ;
Etant entendu que :
**R3** et **R4**,
   (i) qui peuvent être identiques ou différents, représentent, indépendamment l'un de l'autre, un atome d'hydrogène, un groupe -(C1-C5)alkyle, un groupe -(C3-C6)cycloalkyle, un groupe aryle, un groupe hétéroaryle, un groupe -CH₂-hétéroaryle, un groupe -(C1-C5)alkyl-NR5R6, un groupe -W-OH ou un groupe -W-NR5R6 ; ou
   (ii) forment ensemble avec l'atome d'azote qui les porte un groupe hétérocycloalkyle éventuellement substitué par un ou plusieurs groupes choisis parmi les groupes -(C1-C5)alkyle et les groupes -CH₂-aryle ;
**W** est un groupe -(C1-C5)alkylène, éventuellement substitué par un ou plusieurs groupes hydroxy; et
**R5** et **R6**, qui peuvent être identiques ou différents, représentent, indépendamment l'un de l'autre, un atome d'hydrogène ou un groupe choisi parmi les groupes -(C1-C5)alkyle et les groupes -(C3-C6)cycloalkyle.

Les composés de formule (I) peuvent exister à l'état de bases ou de sels, les composés de formule (I) ayant été dans ce cas salifiés par des acides ou des bases, notamment des acides ou des bases pharmaceutiquement acceptables. On parle alors de sels d'addition, en particulier des sels d'addition à un acide ou à une base, qui font partie de l'invention. Ces sels sont avantageusement préparés avec des acides pharmaceutiquement acceptables, mais les sels d'autres acides utiles, par exemple, pour la purification ou l'isolement des composés de formule (I), font également partie de l'invention. On peut citer les sels de chlorhydrate, les sels d'acide trifluoroacétique et les sels de sodium.

Les composés de formule (I) peuvent également exister sous forme de solvates, à savoir sous forme d'associations ou de combinaisons avec une ou plusieurs molécules de solvant. De tels solvants font également partie de l'invention.

Quand o est égal à 0, alors le cycle ne comporte que des atomes d'hydrogène.

Les différentes formes tautomériques des composés de formule (t) font également partie de l'invention :

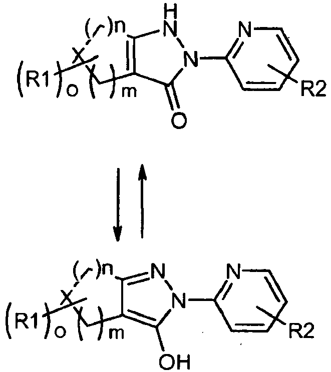

De plus, l'invention a également pour objet un test homogène de mesure directe par complémentation beta galactosidase de la quantité de la protéine HIF1-alpha dans le noyau des cellules, de préférence des cellules HEK, après traitement des dites cellules par un ou des composés à tester consistant à :
(a) Ensemencer, de préference en plaques 384 puits, lesdites cellules dans un milieu de culture approprié, de préférence du sérum de veau foétal à 1 % (abrégé SVF);
(b) Ajouter le ou les composés à tester à une concentration appropriée dans un solvant approprié aux cellules précédemment ensemencées dans ledit milieu de culture, de préférence les composés à tester sont dilués dans le SVF à 0,1%;
(c) Incuber lesdites cellules ainsi préparées dans un incubateur à environ 37°C, avantageusement pendant environ 6h;
(d) Lyser les cellules avec un tampon de lyse contenant un substrat chemiluminescent de la beta galactosidase ;
(e) Incuber à l'abri de la lumière avant lecture et mesure de la luminescence qui est fonction de l'activité de la beta galactosidase.

Les composés selon l'invention ont subi un test de screening selon le test tel que défini ci-dessus.

Dans le cadre de la présente invention, et sauf mention différente dans le texte, on entend par :
- un atome d'halogène : un atome de fluor, de chlore, de brome ou d'iode ;
- un groupe alkyle : un groupe aliphatique saturé, linéaire ou ramifié, pouvant comporter 1, 2, 3, 4 ou 5 atomes de carbone (abrégé par -*(C1-C5)alkyle)*. A titre d'exemples, on peut citer comme (i) groupe *-C1alkyle*, le groupe méthyle, comme (ii) groupe *-C2alkyle*, le groupe éthyle, comme (iii) groupe *-C3alkyle*, le groupe propyle, isopropyle, comme (iv) groupe *-C4alkyle*, le groupe butyle, isobutyle, tertbutyle, comme (v) groupe *-C5alkyle* le groupe pentyle, isopentyle;

- un groupe alkylène : un groupe alkyle tel que précédemment défini, divalent saturé, linéaire ou ramifié, pouvant comporter 1, 2, 3, 4 ou 5 atomes de carbone (abrégé *-(C1-C5)alkylène-*). A titre d'exemple, on peut citer les radicaux méthylène (ou -CH₂-), éthylène (ou -CH₂-CH₂-), propylène (-CH₂-CH₂-CH₂-) ;
- un groupe cycloalkyle : un groupe alkyle cyclique pouvant comporter 3, 4, 5 ou 6 atomes de carbone, abrégé également *-(C3-C6)cycloalkyle.* A titre d'exemples, on peut citer les groupes cyclopropyle, cyclobutyle, cyclopentyle, cyclohexyle ;
- un groupe alcoxy : un radical *-O-alkyle* où le groupe alkyle est tel que précédemment défini. A titre d'exemples, on peut citer les groupes *-O-(C1-C5)alkyle* ou-*(C1-C5)alcoxy,* et en particulier comme (i) groupe *-O-C1alkyle,* le groupe -Ométhyle, comme (ii) groupe *-O-C2alkyle*, le groupe -Oéthyle, comme (iii) groupe *-O-C3alkyle,* le groupe -Opropyle, -Oisopropyle, comme (iv) groupe *-O-C4alkyle,* le groupe -Obutyle,-Oisobutyle, -Otertbutyle, comme (v) groupe *-O-C5alkyle* le groupe -Opentyle,-Oisopentyle;
- un groupe alcoxy-alkyle : un radical de formule *-alkylène-O-alkyle,* où les groupes alkyle et alkylène, comprenant le même nombre de carbone ou ne comprenant pas le même nombre de carbone, sont tels que définis précédemment. A titre d'exmples, on peut citer les groupes *-(C1-C5)alkyléne-O-(Cl-C5)alkyle,* avec -(C1-C5)alkylène- et-(C1-C5)alkyle tels que définis ci-dessus ;
- un groupe halogénoalkyle : un groupe alkyle tel que défini ci-dessus substitué par 1, 2, 3, 4 ou 5 atomes d'halogène, tels que définis précédemment. On citera par exemple les groupes *-halogéno(C1-C5)alkyle,* avec (C1-C5)alkyle tel que défini ci-dessus, et en particulier le groupe trifluorométhyle (abrégé -CF₃) ;
- un groupe aryle : un groupe aromatique cyclique comprenant 5 ou 6 atomes de carbone. A titre d'exemples de groupes aryles, on peut citer le groupe phényle ;
- un groupe hétéroaryle : un groupe aromatique cyclique comprenant 2, 3, 4 ou 5 atomes de carbone et comprenant 1 à 3 hétéroatomes, qui peu(ven)t être choisi(s) parmi l'atome d'azote, l'atome d'oxygène et l'atome de soufre, indépendemment l'un de l'autre, de manière à être identiques ou différents, lorsqu'ils sont au nombre de 2 ou indépendemment les uns des autres, de manière à être identiques ou différents, lorsqu'ils sont au nombre de 3. On peut citer les groupes pyridyle, pyrrole, furanyle ;
- un hétérocycloalkyle : un groupe alkyle cyclique, éventuellement ponté, comprenant 5, 6 ou 7 atomes de carbone et comprenant 1, 2 ou 3 hétéroatomes qui peu(ven)t être choisi(s), indépendemment l'un de l'autre, de manière à être identiques ou différents, lorsqu'ils sont au nombre de 2 ou indépendemment les uns des autres, de manière à être identiques ou différents, lorsqu'ils sont au nombre de 3, parmi l'atome d'azote, l'atome d'oxygène et l'atome de soufre. On peut notamment citer les groupes pipéridinyle, pipérazinyle, pyrrolidinyle, hexaméthylèneimino, morpholinyle, 1,1-dioxydotetrahydrothiényle;
- les lettres α, β, γ et δ autour de la pyridine des composés de formule (I) permettent d'identifier les positions des différents atomes de carbone.

Parmi les composés décrits dans la présente invention, on peut citer un premier groupe de composés répondant à la formule (I) dans laquelle :
**n** est égal à 0, 1, 2, 3 ou 4,
et/ou
**m** est égal à 0, 1 ou 2,
et/ou
**o** est égal à 0 ou 1,
et/ou
**X** représente un groupe -CH₂-, -CH(R')-, -N(R')- ou un hétéroatome choisi parmi l'atome d'oxygène et l'atome de soufre,
et/ou
**R'** représente un groupe -(C1-C5)alkyle, un groupe -(C1-C5)alcoxy, un groupe -CH₂-aryle, un groupe -C(O)R5 ou un groupe -COOR5 ;
et/ou
**R1** représente un groupe oxo, un groupe -COOR5, un groupe -W-OH ou un groupe -W-NR5R6 ;
et/ou
**R2** représente un atome d'hydrogène, un groupe -(C1-C5)alkyle, un groupe -(C1-C5)alcoxy, un groupe -COOR5, un groupe -NR5R6, un groupe -C(O)-NR5R6 ou un groupe -SO₂-NR3R4;
et/ou
**R3** et **R4**
   (i) représentent, indépendamment l'un de l'autre, un atome d'hydrogène, un groupe -(C1-C5)alkyle, un groupe -(C3-C6)cycloalkyle, un groupe aryle, un groupe hétéroaryle, un groupe -CH₂-hétéroaryle ou un groupe -(C1-C5)alkyl-NR5R6 ; ou
   (ii) forment ensemble avec l'atome d'azote qui les porte un groupe hétérocycloalkyle éventuellement substitué par un groupe -(C1-C5)alkyle ou par un groupe aryle,
et/ou
**W** représente un groupe -(C1-C5)alkylène, éventuellement substitué par un ou plusieurs groupes hydroxy;
et/ou
**R5** et **R6** représentent indépendamment l'un de l'autre un atome d'hydrogène ou un groupe -(C1-C5)alkyle.

Un premier sous-groupe de composés de l'invention est constitué par les composés de formule (I) dans laquelle **n** est égal à 0, 1, 2, 3 ou 4.

Un deuxième sous-groupe de composés de l'invention est constitué par les composés de formule (I) dans laquelle **m** est égal à 0, 1 ou 2.

Un troisième sous-groupe de composés de l'invention est constitué par les composés de formule (I) dans laquelle **o** est égal à 0.

Un quatrième sous-groupe de composés de l'invention est constitué par les composés de formule (I) dans laquelle **R1** représente un groupe oxo, un groupe -CH₂-aryle, un groupe - C(O)R5- ou un groupe -COOR5, ledit groupe R1 pouvant être lié à un atome de carbone ou un hétéroatome. Avantageusement, le groupe aryle représente un groupe phényle.

Un cinquième sous-groupe de composés de l'invention est constitué par les composés de formule (I) dans laquelle **R2** représente (i) un atome d'hydrogène, (ii) un groupe- (C1-C5)alkyle, (iii) un groupe -(C1-C5)alcoxy, (iv) un groupe -COOR5, (v) un groupe -NR5R6, (vi) un groupe -C(O)-NR5R6, (vii) un hétéroaryle substitué par un groupe -(C1-C5) alkyle, (viii) un groupe -O-W-aryle ou (ix) un groupe -O-W-heteroaryle.

Un sixième sous-groupe de composés de l'invention est constitué par les composés de formule (I) dans laquelle **R2** représente un groupe -SO₂-NR3R4.

Un septième sous-groupe de composés de l'invention est constitué par les composés de formule (I) dans laquelle **R2** est un substituant de l'atome situé en position beta de la pyridine.

Un huitième sous-groupe de composés de l'invention est constitué par les composés de formule (I) dans laquelle **R2** est un substituant de l'atome situé en position gamma de la pyridine.

Un neuvième sous-groupe de composés de l'invention est constitué par les composés de formule (I) dans laquelle **R3** et **R4** représentent indépendamment l'un de l'autre un atome d'hydrogène, un groupe -(C1-C5)alkyle, un groupe -(C3-C6)cycloalkyle, un groupe aryle, un groupe hétéroaryle, un groupe -CH₂-hétéroaryle ou un groupe -(C1-C5)alkyl-NR5R6. Avantageusement, le groupe aryle représente un groupe phényle et le groupe hétéroaryle représente un groupe pyridinyle ou un groupe furanyle.

Un dixième sous-groupe de composés de l'invention est constitué par les composés de formule (I) dans laquelle **R3** et **R4** forment ensemble avec l'atome d'azote qui les porte un groupe hétérocycloalkyle éventuellement substitué par un ou plusieurs groupe(s) - (C1-C5)alkyle et/ou aryle. Avantageusement, le groupe hétérocycloalkyle représente un groupe pipéridinyle, un groupe pyrrolidinyle ou un groupe hexaméthylèneimino et le groupe aryle représente un groupe phényle.

Un onzième sous-groupe de composés de l'invention est constitué par les composés de formule (I) dans laquelle **R5** représente un groupe -(C1-C5)alkyle ou un groupe -(C1-C5)cycloalkyle.

Un douzième sous-groupe de composés de l'invention est constitué par les composés de formule (I) dans laquelle **R6** représente un atome d'hydrogène ou un groupe (C1-C5)alkyle.

Les sous-groupes définis ci-dessus pris séparément ou en combinaison font également partie de l'invention.

Parmi les composés décrits dans la présente invention, on peut encore citer un sous- groupe de composés répondant à la formule (I) dans laquelle :
- **n** est égal à 1, 2, 3 ou 4 ;
- **m** est égal à 0, 1 ou 2 ;
- **o** est égal à 0 ou 1 ;
- **X** représente un groupe -CH₂-, un groupe -CH(R')-, un groupe -N(R')- ou un hétéroatome choisi parmi l'atome d'oxygène et l'atome de soufre ;
- **R'** représente un groupe -(C1-C5)alkyle, un groupe -(C1-C5)alcoxy, un groupe-CH₂-aryle, un groupe -C(O)R5 ou un groupe -COOR5 ;
- **R1** représente un groupe oxo, un groupe COOR5, un groupe -W-OH ou un groupe -W-NR5R6 ;
- **R2** représente un groupe -SO₂-NR3R4;
- **R3** et **R4** représentent, indépendamment l'un de l'autre, un atome d'hydrogène, un groupe -(C1-C5)alkyle, un groupe -(C3-C6)cycloalkyle, un groupe aryle, un groupe hétéroaryle ou un groupe -CH₂-hétéroaryle, ou **R3** et **R4** forment ensemble avec l'atome d'azote qui les porte un groupe hétérocycloalkyle ; et
- **R5** et **R6** représente un groupe -(C1-C5)alkyle.

Parmi les composés décrits dans la présente invention, on peut enfin citer un sous- groupe de composés répondant à la formule (I) dans laquelle :
- **n** est égal à 1, 2, 3 ou 4 ;
- **m** est égal à 0, 1 ou 2 ;
- **o** est égal à 0 ou 1 ;
- **X** représente un groupe -CH₂-, un groupe -CH(R')-, un groupe -N(R')- ou un hétéroatome choisi parmi l'atome d'oxygène et l'atome de soufre ;
- **R'** représente un groupe -(C1-C5)alkyle, un groupe -(C1-C5)alcoxy, un groupe-CH₂-aryle, un groupe -C(O)R5 ou un groupe -COOR5 ;
- **R1** représente un groupe oxo ;
- **R2** représente un atome d'hydrogène, un groupe -(C1-C5)alkyle, un groupe -(C1-C5)alcoxy, un groupe -COOR5, un groupe -NR5R6 ou -C(O)-NR5R6 ; et
- **R5** et **R6** représentent, indépendamment l'un de l'autre, un atome d'hydrogène, un groupe -(C1-C5)alkyle ou un groupe -(C1-C5)cycloalkyle.

Parmi les composés de formule (I) objets de l'invention, on peut citer notamment les composés suivants :
∘ 2-[5-(pipéridin-1-ylsulfonyl)pyridin-2-yl]-1,2,4,5,6,7-hexahydro-3H-indazol-3-one ;
∘ 6-méthyl-2-[5-(piperidin-1-ylsulfonyl)pyridin-2-yl]-1,2,4,5,6,7-hexahydro-3H-indazol-3-one ;
∘ 2-[5-(pipéridin-1-ylsulfonyl)pyridin-2-yl]-1,4,5,6,7,8-hexahydrocyclohepta[c]pyrazol-3(2H)-one ;
∘ N-éthyl-6-(3-oxo-1,3,4,5,6,7-hexahydro-2H-indazol-2-yl)-N-phénylpyridine-3-sulfonamide;
∘ 6-(5-benzyl-3-oxo-1,3,4,5,6,7-hexahydro-2H-pyrazolo[4,3-c]pyridin-2-yl)-N-éthyl-N-phénylpyridine-3-sulfonamide;
∘ (+/-)2-(5-{[(3R,5S)-3,5-diméthylpipéridin-1-yl]sulfonyl}pyridin-2-yl)-1,2,4,5,6,7-hexahydro-3H-indazol-3-one;
∘ 2-(4-méthoxypyridin-2-yl)-1,2,4,6-tetrahydro-3H-thieno[3,4-c]pyrazol-3-one;
∘ 2-(pyridin-2-yl)-1,4,5,6-tétrahydrocyclopenta[c]pyrazol-3(2H)-one;
∘ (+/-)5-benzyl-2-(5-{[(3R,5S)-3,5-diméthylpiperidin-1-yl]sulfonyl}pyridin-2-yl)-1,2,4,5,6,7-hexahydro-3H-pyrazolo[4,3-c]pyridin-3-one;
∘ (+/-)2-(5-{[(3R,5S)-3,5-dimethylpiperidin-1-yl]sulfonyl}pyridin-2-yl)-6-méthyl-1,2,4,5,6,7-hexahydro-3H-indazol-3-one;
∘ 6-(5-benzyl-3-oxo-1,3,4,5,6,7-hexahydro-2H-pyrazolo[4,3-c]pyridin-2-yl)-N, N-diéthylpyridine-3-sulfonamide;
∘ N-éthyl-6-(3-oxo-1,3,4,5,6,7,8,9-octahydro-2H-cycloocta[c]pyrazol-2-yl)-N-phenylpyridine-3-sulfonamide;
∘ N-éthyl-6-(3-oxo-4,6-dihydro-1H-thieno[3,4-c]pyrazol-2(3H)-yl)-N-phénylpyridine-3-sulfonamide;
∘ 6-(5-benzyl-3-oxo-1,3,4,5,6,7-hexahydro-2H-pyrazolo[4,3-c]pyridin-2-yl)-N,N-di(propan-2-yl)pyridine-3-sulfonamide;
∘ 6-méthoxy-2-(pyridin-2-yl)-1,4,5,6-tétrahydrocyclopenta[c]pyrazol-3(2H)-one;
∘ 2-(pyridin-2-yl)-1,4,6,7-tétrahydrothiopyrano[4,3-c]pyrazol-3(2H)-one;
∘ N-éthyl-6-(3-oxo-1,4,6,7-tétrahydrothiopyrano[4,3-c]pyrazol-2(3H)-yl)-N-phenylpyridine-3-sulfonamide;
∘ N-éthyl-6-(3-oxo-3,5,6,7-tétrahydrothiopyrano[3,2-c]pyrazol-2(1H)-yl)-N-phénylpyridine-3-sulfonamide;
∘ N,N-diéthyl-6-(3-oxo-4,6-dihydro-1H-thieno[3,4-c]pyrazol-2(3H)-yl)pyridine-3-sulfonamide;
∘ N,N-diméthyl-6-(3-oxo-4,6-dihydro-1H-thièno[3,4-c]pyrazol-2(3H)-yl)pyridine-3-sulfonamide;
∘ 2-[5-(pyrrolidin-1-ylsulfonyl)pyridin-2-yl]-1,2,4,6-tétrahydro-3H-thièno[3,4-c]pyrazol-3-one;
∘ N-cyclopropyl-6-(3-oxo-4,6-dihydro-1H-thieno[3,4-c]pyrazol-2(3H)-yl)pyridine-3-sulfonamide;
∘ 6-(3-oxo-4,6-dihydro-1H-thieno[3,4-c]pyrazol-2(3H)-yl)-N-(propan-2-yl)pyridine-3-sulfonamide;
∘ N-tert-butyl-6-(3-oxo-4,6-dihydro-1H-thieno[3,4-c]pyrazol-2(3H)-yl)pyridine-3-sulfonamide;
∘ N-(furan-2-ylmethyl)-6-(3-oxo-4,6-dihydro-1H-thieno[3,4-c]pyrazol-2(3H)-yl)pyridine-3-sulfonamide;
∘ N-cyclopentyl-6-(3-oxo-4,6-dihydro-1H-thieno[3,4-c]pyrazol-2(3H)-yl)pyridine-3-sulfonamide;
∘ N-methyl-6-(3-oxo-4,6-dihydro-1H-thieno[3,4-c]pyrazol-2(3H)-yl)-N-(pyridin-2-yl)pyridine-3-sulfonamide;
∘ 2-(pyridin-2-yl)-1,2,4,6-tetrahydro-3H-thieno[3,4-c]pyrazol-3-one ;
∘ 2-[4-(dimethylamino)pyridin-2-yl]-1,2,4,6-tetrahydro-3H-thieno[3,4-c]pyrazol-3-one ;
∘ 2-{5-[(4-benzylpiperidin-1-yl)sulfonyl]pyridin-2-yl}-1,2,4,6-tetrahydro-3H-thieno[3,4-c]pyrazol-3-one ;
∘ 6-(3-oxo-4,6-dihydro-1H-thieno[3,4-c]pyrazol-2(3H)-yl)-N-(pyridin-2-yl)pyridine-3-sulfonamide;
∘ N-ethyl-6-(3-oxo-1,4,6,7-tetrahydropyrano[4,3-c]pyrazol-2(3H)-yl)-N-phenylpyridine-3-sulfonamide;
∘ 2-(4-ethylpyridin-2-yl)-2,6-dihydro-4H-thieno[3,4-c]pyrazol-3-olate de sodium;
∘ 2-[5-(azepan-1-ylsulfonyl)pyridin-2-yl]-1,2,4,6-tetrahydro-3H-thieno[3,4-c]pyrazol-3-one;
∘ 4-benzyl-2-{5-[ethyl(phenyl)sulfamoyl]pyridin-2-yl}-5-oxo-4,5,6,7-tetrahydro-2H-pyrazolo[4,3-b]pyridin-3-olate de sodium;
∘ N-methyl-6-(3-oxo-4,6-dihydro-1H-thieno[3,4-c]pyrazol-2(3H)-yl)-N-(pyridin-2-ylmethyl)pyridine-3-sulfonamide;
∘ 2-{5-[ethyl(phenyl)sulfamoyl]pyridin-2-yl}-3-oxo-1,2,3,4,6,7-hexahydro-5H-pyrazolo[4,3-c]pyridine-5-carboxylate de tert-butyle;
∘ 6-(5-acetyl-3-oxo-1,3,4,5,6,7-hexahydro-2H-pyrazolo[4,3-c]pyridin-2-yl)-N-ethyl-N-phenylpyridine-3-sulfonamide;
∘ 6-(3-oxo-4,6-dihydro-1H-thieno[3,4-c]pyrazol-2(3H)-yl)pyridine-3-carboxylate tert-butyle;
∘ 2-(4-methylpyridin-2-yl)-2,6-dihydro-4H-thieno[3,4-c]pyrazol-3-olate de sodium;
∘ 2-{5-[tert-butyl(methyl)sulfamoyl]pyridin-2-yl}-2,6-dihydro-4H-thieno[3,4-c]pyrazol-3-olate de sodium;
∘ 2-{5-[tert-butyl(ethyl)sulfamoyl]pyridin-2-yl}-2,6-dihydro-4H-thieno[3,4-c]pyrazol-3-olate de sodium;
∘ 2-(5-methylpyridin-2-yl)-2,6-dihydro-4H-thieno[3,4-c]pyrazol-3-olate de sodium;
∘ 2-[5-(tert-butylcarbamoyl)pyridin-2-yl]-2,6-dihydro-4H-thieno[3,4-c]pyrazol-3-olate de sodium;
∘ 2-(5-methoxypyridin-2-yl)-2,6-dihydro-4H-thieno[3,4-c]pyrazol-3-olate de sodium;
∘ N-methyl-6-(3-oxo-4,6-dihydro-1H-thieno[3,4-c]pyrazol-2(3H)-yl)-N-(pyridin-4-yl)pyridine-3-sulfonamide;
∘ 2-{5-[cyclopentyl(ethyl)sulfamoyl]pyridin-2-yl}-2,6-dihydro-4H-thieno[3,4-c]pyrazol-3-olate de sodium;
∘ 2-[4-(pyridin-3-ylmethoxy)pyridin-2-yl]-2,6-dihydro-4H-thieno[3,4-c]pyrazol-3-olate de sodium;
∘ 2-{5-[ethyl(phenyl)sulfamoyl]pyridin-2-yl}-3-oxo-1,2,3,4,6,7-hexahydro-5H-pyrazolo[4,3-c]pyridine-5-carboxylate de méthyle;
∘ cyclopentyl 6-(3-oxo-4,6-dihydro-1H-thieno[3,4-c]pyrazol-2(3H)-yl)pyridine-3-carboxylate de cyclopentyle;
∘ 6-(3-oxo-4,6-dihydro-1H-thieno[3,4-c]pyrazol-2(3H)-yl)pyridine-3-carboxylate de2-méthylpropyle;
∘ 2-[4-(propan-2-yl)pyridin-2-yl]-1,2,4,6-tetrahydro-3H-thieno[3,4-c]pyrazol-3-one;
∘ 2-[5-(propan-2-yl)pyridin-2-yl]-2,6-dihydro-4H-thieno[3,4-c]pyrazol-3-olate de sodium;
∘ methyl2-{5-[ethyl(phenyl)sulfamoyl]pyridin-2-yl}-3-oxo-2,3,4,6-tetrahydro-1H-thieno[3,4-c]pyrazole-4-carboxylate de méthyle;
∘ 6-(3-oxo-4,6-dihydro-1H-thieno[3,4-c]pyrazol-2(3H)-yl)pyridine-3-carboxylate de propan-2-yle;
∘ 2-{5-[ethyl(phenyl)sulfamoyl]pyridin-2-yl}-6-methoxy-2,4,5,6-tetrahydrocyclopenta[c]pyrazol-3-olate de sodium;
∘ N-ethyl-6-(3-oxo-3,4,5,6-tetrahydrocyclopenta[c]pyrazol-2(1H)-yl)-N-phenylpyridine-3-sulfonamide;
∘ 2-[4-(pyridin-3-ylmethoxy)pyridin-2-yl]-2,6-dihydro-4H-thieno[3,4-c]pyrazol-3-olate de sodium;
∘ 6-(3-oxo-4,6-dihydro-1H-thieno[3,4-c]pyrazol-2(3H)-yl)pyridine-3-carboxylate 2,2-diméthylpropyle;
∘ 2-[5-(5-tert-butyl-1,2,4-oxadiazol-3-yl)pyridin-2-yl]-1,2,4,6-tetrahydro-3H-thieno[3,4-c]pyrazol-3-one;
∘ N-cyclopentyl-N-methyl-6-(3-oxo-4,6-dihydro-1H-thieno[3,4-c]pyrazol-2(3H)-yl)pyridine-3-sulfonamide;
∘ N-cyclopentyl-N-ethyl-6-(3-oxo-3,4,5,6-tetrahydrocyclopenta[c]pyrazol-2(1 H)-yl)pyridine-3-sulfonamide;
∘ N-cyclopentyl-N-(2,3-dihydroxypropyl)-6-(3-oxo-4,6-dihydro-1H-thieno[3,4-c]pyrazol-2(3H)-yl)pyridine-3-sulfonamide;
∘ 6-[5-(methylsulfonyl)-3-oxo-1,3,4,5,6,7-hexahydro-2H-pyrazolo[4,3-c]pyridin-2-yl]pyridine-3-carboxylate 2,2-dimethylpropyle;
∘ 2-[5-(3-tert-butyl-1,2,4-oxadiazol-5-yl)pyridin-2-yl]-1,2,4,6-tetrahydro-3H-thieno[3,4-c]pyrazol-3-one.

Dans ce qui suit, on entend par groupe protecteur (Pg) un groupe qui permet, d'une part, de protéger une fonction réactive telle qu'un alcool ou une amine pendant une synthèse. Des exemples de groupes protecteurs ainsi que des méthodes de protection et de déprotection sont données dans « Protective Groups in Organic Synthesis », Green et al., 3rd Edition (John Wiley & Sons, Inc., New York).

On entend par groupe partant (Lg), dans ce qui suit, un groupe nucléofuge pouvant être facilement clivé d'une molécule par rupture d'une liaison hétérolytique, avec départ d'une paire électronique. Ce groupement peut ainsi être remplacé facilement par un autre groupement nucléophile lors d'une réaction de substitution, par exemple. De tels groupes partants sont, par exemple, les halogènes ou un groupe hydroxyle activé tel qu'un mésyle, tosyle, triflate, acétyle, etc. Des exemples de groupes partants ainsi que des références pour leur préparation sont donnés dans « Advances in Organic Chemistry », J. March, 3rd Edition, Wiley Interscience, p. 310-316.

Conformément à l'invention, on peut préparer les composés de formule générale (I) selon le procédé qui suit, illustré dans le schéma 1.

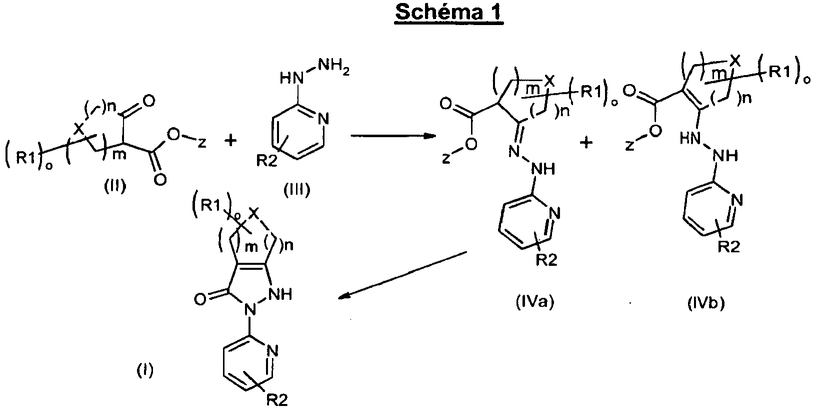

La synthèse des composés de formule générale (I) est faite à partir des composés de formule (II), dans lesquels R1, X, n et m, o sont tels que décrits précédemment et z représente un groupe alkyle, préférentiellement méthyle ou éthyle, qu'on condense dans un solvant protique de type alcool, de préférence le méthanol, à une température comprise entre 20 et 60°C, avec un composé de formule (III) dans laquelle R2 est tel que décrit précédemment, pour conduire à un intermédiaire de formule (IV) qui se présente sous la forme d'un composé de formule (IVa) ou (IVb), ou le mélange des deux, et qui est cyclisé en présence d'une base organique, de préférence le méthylate de sodium, dans un solvant protique tel que le méthanol, à une température comprise entre 20 et 50°C.

Les composés (I) obtenus sont optionnellement convertis avec l'acide ou la base correspondantes en sel.

Les composés de formule (II) quand leur mode de préparation n'est pas décrit, sont disponibles dans le commerce ou décrits dans la littérature, ou bien peuvent être préparés selon des méthodes qui sont connues de l'homme de métier.

Les schémas 2a, 2b et 2c décrivent la préparation des composés de formule (III).
Les composés de formule (III) sont obtenus à partir du composé de formule (V), avec Lg et R2 tels que définis précédemment, par addition d'hydrazine hydrate, de préférence dans un solvant protique tel que l'éthanol, à une température comprise entre 60 et 80°C (schéma 2a).

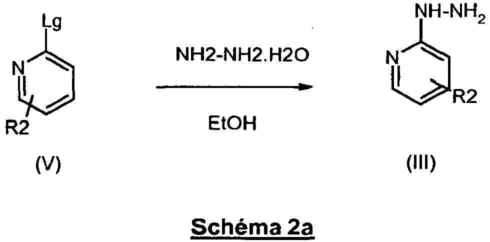

Les composés de formule (III) peuvent être aussi obtenus en deux étapes à partir du composé de formule (V). La fonction hydrazine est alors introduite, par une réaction de couplage entre la benzophénone hydrazone de formule (VI) et le composé de formule (V) en présence d'une quantité catalytique de palladium pour aboutir à l'intermédiaire de formule (VII), dont la fonction hydrazine est libérée par traitement acide tel que l'acide chlorhydrique, de préférence à une concentration comprise entre 6 et 12 N, dans un mélange binaire de solvants non miscibles tels que le toluène et l'eau à une température de 100°C (schéma 2b).

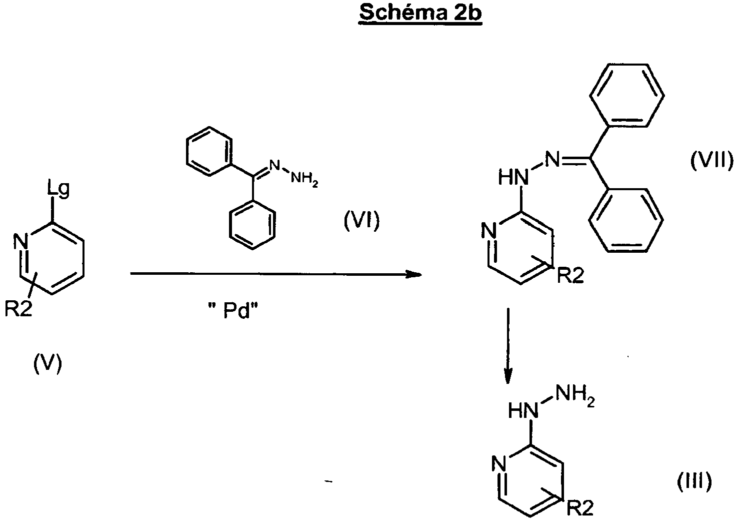

Alternativement, les composés de formule (III) peuvent être synthétisés à partir d'une amino pyridine de formule (VIII) avec R2 tel que défini précédemment, par action d'un mélange d'acide nitrique et d'acide sulfurique concentré à 0°C pour obtenir l'intermédiaire N-nitroamine qui est réduit dans de la soude 10N en présence de zinc (Schéma 2c).

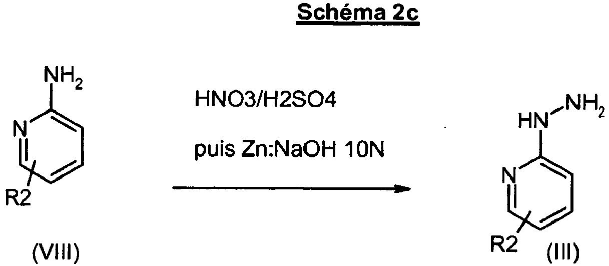

Dans les schémas précédents, les composés de départ, les intermédiaires et les réactifs, quand leur mode de préparation n'est pas décrit, sont disponibles dans le commerce ou décrits dans la littérature, ou bien peuvent être préparés selon des méthodes qui sont connues de l'homme de métier.

L'invention, selon un autre de ses aspects, a également pour objet les composés de formules (IVa), (IVb). Ces composés sont utiles comme intermédiaires de synthèse des composés de formule (I).

### EXEMPLES :

Les exemples suivants illustrent la préparation de certains composés conformes à l'invention. Les numéros des composés exemplifiés renvoient à ceux du tableau donné plus loin qui illustre les structures chimiques et les propriétés physiques de quelques composés selon l'invention.

Les abréviations et formules semi-développées suivantes sont utilisées :
- AcOEt: Acétate d'éthyle
- AcOH: Acide acétique
- anh.: Anhydre
- CCM: Chromatographie sur couche mince
- CL: Chromatographie liquide
- Cs₂CO₃: Carbonate de césium
- DCM: Dichlorométhane
- DMF: Diméthylformamide
- DMSO: Diméthylsulfoxide
- DME: 1,2 diméthoxyéthane
- EtOH: Ethanol
- MeOH: Méthanol
- h: Heure(s)
- HCl: Acide chlorhydrique
- K₂CO₃: Carbonate de potassium
- NH₄Cl: Chlorure d'ammonium
- NaHCO₃: Hydrogénocarbonate de sodium
- Na₂SO₄: Sulfate de sodium
- SM: Spectrométrie de masse
- TEA: Triéthylamine
- TFA: Acide trifluoroacétique
- THF: Tétrahydrofurane
- TA: Témperature ambiante
- HNO₃: Acide nitrique
- H₂SO₄: Acide sulfurique
- Conc: Concentré
- racBINAP: (+/-)-2,2-Bis(diphenylphosphino)-1,1'binaphthalene
- TBTU: 2-(1-H-Benzotriazole-1-yl)-1,1,3,3-tetramethyluronium tetrafluoroborate
- DIEA: Diisopropyléthylamine
- NMO: 4-méthylmorpholine N-oxide
- OsO4: Tétroxide d'osmium
- pTsOH: acide paratoluenesulfonique
- Tr: temps de rétention
- T: temps
- T°C: température en °C
- Min: minute
- PF: point de fusion

Les spectres de résonnance magnétique du proton (RMN1H), tels que décrits ci-dessous, sont enregistrés à 400MHz dans du DMSO-d6, en utilisant le pic du DMSO-d5 comme référence. Les déplacements chimiques δ sont exprimés en partie par million (ppm). Les signaux observés sont exprimés ainsi : s = singulet; sl = singulet large; d = doublet ; dd = doublet de doublet ;dt = doublet de triplet ; t = triplet ; m = massif ; H = proton

Les spectres de masse sont obtenus dans les conditions de couplage LC/MS suivantes :
**Méthode 1:** Colonne Jsphere33*2 mm ;4µM ;
   Eluants : A = H2O +0.05%TFA ; B = CH₃CN +0.05 %TFA
   T0 :98%A ;T1,0 à T5,0min :95%B ;
**Méthode 2:** Colonne :Acquity BEH C18 (50*2,1 mm ; 1,7 µM) ;220nm
   Eluants : A = H2O +0.05% TFA ; B = CH₃CN +0.035TFA.
   **T0** :98%A ;T1,6 à T2,1min :100%B ; T2,5 à T3min: 98%A
   Débit 1.0mL/min- T°C=40°C, injection 2µL

### Exemple 1 : 2-pyridin-2-yl-1,2,4,6-tétrahydro-3H-thiéno[3,4-c]pyrazol-3-one (Composé 1 du Tableau I)

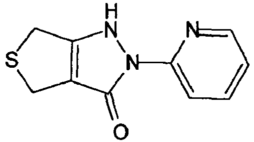

Un mélange de 7 g (64,1 mmoles) de 2-hydrazinopyridine et de 10,3 g (64,1 mmoles) de 4-oxotétrahydrothiophéne-3- carboxylate de méthyle dans 130 mL de MeOH est chauffé 12 h à 80°C. Le milieu est ensuite concentré sous pression réduite et le résidu obtenu est purifié par chromatographie sur colonne de gel de silice en éluant avec un gradient DCM / MeOH de 0 à 10 % de MeOH. Après concentration sous pression réduite, 8,7 g (34,5 mmoles) d'intermédiaire hydrazone, sous forme de poudre jaune, sont isolés et sont ensuite ajoutés,par petites portions, à TA, à une solution de 0,8 g (34,5 mmoles) de sodium dans 46 mL de MeOH anh. Le milieu réactionnel est agité 2 h à TA, puis le précipité formé est filtré, lavé successivement avec 10 mL de MeOH et 20 mL de pentane. Le résidu obtenu est dissous dans 20 mL d'eau et on ajoute 10 mL d'acide acétique. Le précipité obtenu est filtré, lavé avec 10 mL d'eau, séché sous vide et recristallisé dans l'EtOH. On obtient 4,2g de 2-pyridin-2-yl-1,2,4,6-tétrahydro-3H-thiéno[3,4-c]pyrazol-3-one sous forme d'une poudre blanchâtre.
Rendement = 56%
PF(°C) = 152
M = C₁₀H₉N₃OS = 219 ; M+H = 220 ; Méthode 2: Tr= 0,81 min.
RMN ¹H, d6-DMSO, 400MHz, δ (ppm) : 8,45 (d,1H); 8,2 (d,1H); 7,95 (m,1H); 7,3 (t,1H); 4,0 (s,2H); 3,8 (s,2H).

### Exemple 2: Chlorhydratecle2-[4-(diméthylamino)pyridin-2-yl]-1,2,4,6-tétrahydro-3H-thiéno[3,4-c]pyrazol-3-one (Composé 56 du tableau I)

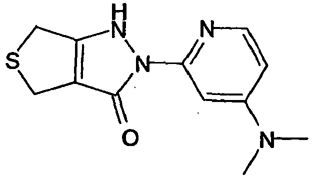

### 2.1. 2-bromo-N,N-diméthylpyridin-4-amine

A une solution de 12,6 mL (125 mmoles) de N, N-diméthyléthanolamine dans 160 mL d'hexane anh., on additionne, sous atmosphère d'argon, à -5°C, en 2 h 30, 100 mL (250 mmoles) de nbutyllithium 2,5 M dans l'hexane puis on agite le milieu réactionnel 0,5h à 0°C et on ajoute ensuite 7,6 g (62,5 mmoles) de 4-diméthylaminopyridine. Après 1 h d'agitation à 0°C, le milieu réactionnel est refroidi à - 78°C et 51,8 g (156,2 mmoles) de tétrabromure de carbone en solution dans 250 mL d'hexane anh. sont ajoutés en 2 h 30 à - 78°C. On laisse ensuite remonter la température jusqu'à 0°C puis l'agitation est poursuivie 1 H 30.

Le milieu est ensuite hydrolysé avec de l'eau (400mL), extrait successivement avec de l'Et2O (400mL) et du DCM (2X400mL). Les phases organiques rassemblées sont séchées sur Na₂SO₄, filtrées et concentrées sous pression réduite. On obtient 6,4 g de 2-bromo-N,N-diméthylpyridin-4-amine sous forme d'un solide marron qui est utilisé tel quel à l'étape suivante.
Rendement = 51 %
RMN ¹H, (CDCl₃, 400 MHz, δ (ppm) : 7,9 (d,1H); 6,6 (s,1H); 6,4 (d,1H); 2,9 (s,6H).

### 2.2. 2-[2-(diphénylmethylidène)hydrazino]-N,N-diméthylpyridin-4-amine

A un mélange de 3,3g (16,41 mmoles) de 2-bromo-N,N-diméthylpyridin-4-amine dans 40 mL de toluène anh, de 3,5 g (18,05 mmoles) de benzophénone hydrazone, de 2,2 g (23 mmoles) de tertbutoxide de sodium anh. et de 100 mg (0,82 mmoles) d'acide benzène boronique dans 40 mL de toluène, on ajoute,sous argon , à T.A, après avoir dégazé le milieu réactionnel sous argon, 74 mg (0,33 mmoles) de palladium acétate et 205 mg (0,33 mmoles) de rac BINAP. Le milieu réactionnel est ensuite chauffé 4h à 80°C. Le milieu réactionnel est repris par 200 mL d'AcOEt, lavé successivement, avec de l'eau (3x30 mL), avec une solution saturée en NaHCO₃ (30 mL) et de la saumure (30 mL) puis séché sur Na₂SO₄, filtré et concentré sous pression réduite. Le résidu obtenu est purifié par chromatographie sur colonne de gel de silice en éluant avec un gradient DCM / MeOH de 0 à 10 % MeOH. Après concentration sous pression réduite, on obtient 4,5 g de 2-[2-(diphénylméthylidène)hydrazino]-N,N-diméthylpyridin-4-amine sous forme d'un solide rouge.
Rendement = 87 %

### 2.3. 2-hydrazino-N,N-diméthylpyridin-4-amine

Un mélange de 4,5 g (14,22 mmoles) de 2-[2-(diphénylméthylidène)hydrazino]-N,N-diméthylpyridin-4-amine, dans 300 mL de toluène et de 80 mL d'acide chlorhydrique 37% aqueux est chauffé 4h à 110°C. Après retour à TA, le milieu réactionnel est extrait avec du toluène (3 x300 mL). La phase aqueuse est diluée par 200 mL d'eau, neutralisée à 0°C par addition d'une solution de soude 12N, puis extraite avec du DCM (3X150 mL). Les phases organiques sont rassemblées, lavées avec de la saumure (300 mL), puis séchées sur Na₂SO₄, filtrées, et concentrées sous pression réduite. On obtient 1,87 g de 2-hydrazino-N,N-diméthylpyridin-4-amine sous forme d'un solide marron qui est engagé tel quel à l'étape suivante.
Rendement =87%
RMN 1H (ppm, d6-DMSO, 400 MHz) : 7,6 (d,1H); 6,9 (s,1H); 6,1 (dd,1H); 5,9 (d,1H); 4 (sl,2H); 2,9 (s,6H).

### 2.4. Chlorhydrate de 2-[4-(diméthylamino)pyridin-2-yl]-1,2,4,6-tétrahydro-3H-thiéno[3,4-c]pyrazol-3-one

Selon le procédé décrit dans l'exemple 1, on obtient, à partir de 1,87g de 2-hydrazino-N,N-diméthylpyridin-4-amine et de 1,97g de 4-oxotétrahydrothiophène-3- carboxylate de méthyle, 35 mg de 2-[4-(diméthylamino)pyridin-2-yl]-1,2,4,6-tétrahydro-3H-thiéno[3,4-c]pyrazol-3-one. On prépare le chlorhydrate en lyophilisant les 35 mg précédemment obtenus en solution dans 1 mL d'HCl 0,1 N.

On obtient ainsi 36 mg de Chlorhydrate de 2-[4-(diméthylamino)pyridin-2-yl]-1,2,4,6-tétrahydro-3H-thiéno[3,4-c]pyrazol-3-one sous forme d'un lyophilisat blanc.
Rendement = 1%
PF(°C) = 182
M=C₁₂H₁₄N₄OS =262; M+H=263; Méthode 2 : Tr= 0,59min.
RMN ¹H, d6-DMSO, 400MHz, δ (ppm) : 7,7 (d,1 H); 6,9 (s,1 H); 6,6 (d,1H); 3,2 (s,2H); 3,5 (s,2H); 2,9 (s,6H).

### Exemple 3 : 2-(4-méthoxypyridin-2-yl)-1,2,4,6-tétrahydro-3H-thièno[3,4-c]pyrazol-3-one (Composé 55 du Tableau I)

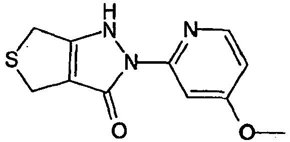

### 3.1. 2 -[2-(diphénylméthylidène)hydrazino]-4-méthoxypyridine

Selon le procédé décrit dans l'exemple 2.2, on obtient, à partir de 0,5 g, de 2-chloro-4-méthoxypyridine, et de 0,75 g de benzophénone hydrazone, 0,76 g de 2-[2-(diphénylméthylidène)hydrazino]-4-méthoxypyridine sous forme d'un solide jaune qui est engagé tel quel à l'étape suivante.

Rendement =73%
RMN ¹H, CDCl₃, 400MHz, δ (ppm) : 8,2 (s,1H); 7,9 (d,1H); 7,7-7,5 (m,5H); 7,4-7,2 (m,5H); 7,1 (s,1H); 6,4 (d,1H); 3,9 (s,3H).

### 3.2. 2-hydrazino-4-méthoxypyridine

Selon le procédé décrit dans l'exemple 2.3, on obtient, à partir de 0,76g de 2-[2-(diphénylméthylidène)hydrazino]-4-méthoxypyridine, 0,24 g de 2-hydrazino-méthoxypyridine sous forme de lyophilisat jaune qui est engagé tel quel à l'étape suivante.
Rendement =69%
RMN ¹H, d6-DMSO, 400MHz, δ (ppm) : 9,7 (sl,1H); 7,7 (d,1H); 6,5(d,1H); 6,4 (s,1 H); 4,8 (sl,2H); 3,9 (s,3H).

### 3.3. 2-(4-méthoxypyridin-2-yl)-1,2,4,6-tétrahydro-3H-thiéno[3,4-c]pyrazol-3-one

Selon le procédé décrit dans l'exemple 1, on obtient, à partir de 243 mg de 2-hydrazino-4-méthoxypyridine et de 280 mg de 4-oxotétrahydrothiophène-3-carboxylate de méthyle, 82 mg de 2-(4-méthoxypyridin-2-yl)-1,2,4,6-tétrahydro-3H-thiéno[3,4-c]pyrazol-3-one sous forme d'un solide blanc.
Rendement = 40%
PF(°C) = 254
M=C₁₁H₁₁N₃O₂S =249; M+H=250; Méthode 2: Tr= 0,8min.
RMN ¹H, d6-DMSO, 400MHz, δ (ppm) : 8,1 (d,1H); 8,05 (d,1H); 6,05 (d,1H); 3,8 (s,3H); 3,7 (s,2H); 3,6 (s,2H)

### Exemple 4 : N-méthyl-6-(3-oxo-4,6-dihydro-1H-thiéno[3,4-c]pyrazol-2(3H)-yl)-N-pyridin-2-ylpyridine-3-sulfonamide (Composé 21 du Tableau I)

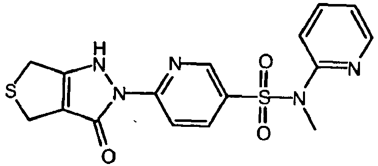

### 4.1. 6-chloro-N-méthyl-N-pyridin-2-ylpyridine-3-sulfonamide

A une solution de 1,3 g (11,8 mmoles) de N-méthylpyridin-2-amine dans 25 mL de DCM, on ajoute à 0°C, 3,3 mL (23,6 mmoles) de triéthylamine puis, par petites portions, 2,5 g (11,8 mmoles) de chlorure de 6-chloropyridine-3-sulfonyle (préparé selon le document WO9840332). Après 12 h d'agitation à TA, le milieu réactionnel est repris par 100 mL de DCM, lavé successivement avec 100 mL d'eau et 30 mL de saumure, séché sur Na₂SO₄, filtré et concentré sous pression réduite. Le résidu obtenu est purifié par chromatographié sur colonne de gel de silice en éluant avec un mélange cyclohexane / AcOEt 4 :1. Après concentration sous pression réduite, on obtient 2,8 g de 6-chloro-N-méthyl-N-pyridin-2-ylpyridine-3-sulfonamide sous forme d'un solide marron.
Rendement = 84%
RMN ¹H, CDCl₃, 400MHz, δ (ppm) : 8,5 (s,1H); 8,25 (d,1H); 7,8 (dd,1H); 7,7 (t,1H); 7,5 (d,1H); 7,3 (d,1H); 7,15 (t,1H); 3,2 (s,3H).

### 4.2. 6-hydrazino-N-méthyl-N-pyridin-2-ylpyridine-3-sulfonamide

Une solution de 1,4 g (4,9 mmoles) de 6-chloro-N-méthyl-N-pyridin-2-ylpyridine-3-sulfonamide et de 0,96 mL (19,7 mmoles) d'hydrazine monohydrate dans 8 mL d'EtOH est chauffé 12 h à 80°C. Le précipité obtenu, après retour à TA, est filtré, lavé avec 10 mL d'EtOH puis séché sous vide. On obtient 0,85 g de 6-hydrazino-N-méthyl-N-pyridin-2-ylpyridine-3-sulfonamide sous forme de cristaux jaune pâle.
Rendement = 62%.
RMN ¹H, CDCl₃, 400MHz, δ (ppm) : 8,35 (s,2H); 8,7 (m,2H); 7,5 (d,1H); 7,15 (dd,1H); 6,7 (d,1 H); 6,4 (sl,1H); 3,5-4,0 (sl,2H) ; 3,3 (s,3H).

### 4.3. N-méthyl-6-(3-oxo-4,6-dihydro-1H-thiéno[3,4-c]pyrazol-2(3H)-yl)-N-pyridin-2-ylpyridine-3-sulfonamide

Selon le procédé décrit dans l'exemple 1, on obtient, à partir de 476 mg de 6-hydrazino-N-méthyl-N-pyridin-2-ylpyridine-3-sulfonamide et de 273 mg de 4-oxotétrahydrothiophéne-3-carboxylate de méthyle, 416 mg de N-méthyl-6-(3-oxo-4,6-dihydro-1H-thiéno[3,4-c]pyrazol-2(3H)-yl)-N-pyridin-2-ylpyridine-3-sulfonamide sous forme d'un solide blanc.
Rendement = 45%
PF(°C) = 226
M = C₁₆H₁₅N₅O₃S₂ = 389 ; M+H = 390; Méthode 2: Tr=0,98 min.
RMN ¹H, d6-DMSO, 400MHz, δ (ppm) : 12,0 (sl,1H); 8,6 (s,1H); 8,5 (sl,1H); 8,4 (d,1 H); 8,2 (dd,1H); 7,55 (d,1H); 7,3 (t,1H); 4,0,(s,2H); 3,8 (s,2H); 3,3 (s,3H).

### Exemple 5 : N-éthyl-6-(3-oxo-3,5,6,7-tétrahydrothiopyrano[3,2-c]pyrazol-2(1H)-yl)-N-phénylpyridine-3-sulfonamide (Composé 24 du Tableau I)

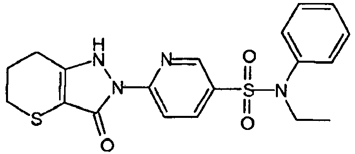

### 5.1. 3-oxotétrahydro-2H-thiopyrane-2-carboxylate d'éthyle

A une solution de 5 g (219 mmoles) de sodium dans 13 mL d'EtOH anh. et 88 mL d'éther, on ajoute goutte à goutte, à 0°C et sous argon, 20,5 g (87,5 mmoles) de 4-[(2-éthoxy-2-oxoéthyl)sulfanyl]butanoate d'éthyle. Après 18 h d'agitation à TA, le mélange réactionnel est versé sur un mélange d'AcOH (12 mL)/glace (70 g). Le milieu est ensuite concentré sous pression réduite, le résidu obtenu est repris par 100 mL d'éther, lavé avec de la saumure (2x50 mL), séché sur Na₂SO₄, filtré et concentré sous pression réduite. Le résidu obtenu est purifié par chromatographie sur gel de silice en éluant avec un gradient cyclohexane / AcOEt de 0 à 10 % d'AcOEt. On obtient 5,64 g de 3-oxotétrahydro-2H-thiopyrane-2-carboxylate d'éthyle sous forme d'une huile jaune.
Rendement = 34%.
RMN ¹H, CDCl₃, 400MHz, δ (ppm) : 12,3 (s,1 H); 4,3 (q,2H); 2,80 (m,2H); 2,4 (t,2H); 2,15 (m,2H); 1,4 (t,3H).

### 5.2. N-éthyl-6-(3-oxo-3,5,6,7-tétrahydrothiopyrano[3,2-c]pyrazol-2(1H)-yl)-N-phénylpyridine-3-sulfonamide

Un mélange de 1 g (5,31 mmoles) de 3-oxotétrahydro-2H-thiopyrane-2-carboxylate d'éthyle et de de 1,55 g de 6-hydrazino-N-méthyl-N-pyridin-2-ylpyridine-3-sulfonamide dans 10 ml de MeOH est chauffé 15 h à 80°C. Après retour a TA, le précipité obtenu est filtré et lavé avec 5 mL de MeOH, puis recristallisé dans l'EtOH. On obtient 395 mg de N-éthyl-6-(3-oxo-3,5,6,7-tétrahydrothiopyrano[3,2-c]pyrazol-2(1H)-yl)-N-phénylpyridine-3-sulfonamide sous forme de poudre blanche.
Rendement = 16%
PF(°C) = 198
M = C₁₉H₂₀N₄O₃S₂ = 416 ; M+H = 417; Méthode 2: Tr=1,12 min
RMN ¹H, d6-DMSO, 400MHz, δ (ppm) : 11,9 (sl,1H); 8,6 (sl,1H); 8,5 (s,1H); 8,1 (d,1H); 7,4 (m, 3H); 7,1 (d,2H); 3,6 (q,2H); 3,0 (t,2H); 2,6 (t,2H); 2,0 (q,2,0); 1,0 (t,3H).

### Exemple 6: 2-[5-[éthyl(phényl)sulfamoyl]pyridin-2-yl]-3-oxo-1,2,3,4,6,7-hexahydro-5H-pyrazolo[4,3,c]pyridine-5-carboxylate de tert-butyle (Composé 28 du Tableau I)

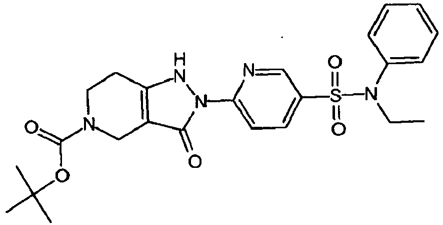

### 6.1. 1-tert-butyl 3 méthyl 4 oxopipéridine 1,3-dicarboxylate

A un mélange de 10 g (51,6 mmoles) de 4-oxopipéridine-3-carboxylate de méthyle et de 7,3 mL (51,6 mmoles) de triéthylamine dans 100mL de DCM, on ajoute 11,3 g (51,6 mmoles) de di-tert-butyldicarbonate. Après 2 h à TA, le milieu est repris par 300 mL de DCM, lavé avec 200mL d'eau, séché sur Na2SO4, puis filtré et concentré sous pression réduite. On obtient 13 g de 1-tert-butyl-3-méthyl-4-oxopipéridine 1,3-dicarboxylate, sous forme d'un solide blanc, qui est engagé tel quel à l'étape suivante.

### 6.2. 2-[5-[éthyl(phényl)sulfamoyl]pyridin-2-yl]-3-oxo-1,2,3,4,6,7-hexahydro-5H-pyrazolo[4,3,c]pyridine-5-carboxylate de tert-butyle

Selon le procédé décrit dans l'exemple 1, on obtient, à partir de 1,7 g de 6-hydrazino-N-méthyl-N-pyridin-2-ylpyridine-3-sulfonamide et de 1,5 g de 1-tert-butyl-3-méthyl-4-oxopipéridine 1,3-dicarboxylate, 190 mg de 2-[5-[éthyl(phényl)sulfamoyl]pyridin-2-yl]-3-oxo-1,2,3,4,6,7-héxahydro-5H-pyrazolo[4,3,c]pyridine-5-carboxylate de tert-butyle sous forme d'un solide blanc.
Rendement = 16%
PF(°C)=192
M = C₂₄H₂₉N₅O₅S =499; M+H = 500; Méthode 2: Tr=1,23 min
RMN ¹H, d6-DMSO, 400MHz, δ (ppm) : 12,1 (sl,1H); 8,6 (sl,1H); 8,5 (s,1H); 8,1 (dd,1H); 7,4 (m,3H); 7,1 (d,2H); 4,1 (s, 2H); 3,6 (m,4H); 2,7 (m,2H); 1,4 (s,9H); 1,0 (t,3H).

### Exemple 7 : N-éthyl-6-(3-oxo-1,4,6,7-tétrahydropyrano[4,3-c]pyrazol-2(3H)-yl)-N-phénylpyrldlne-3-sulfonamide (Composé 25 Tableau I)

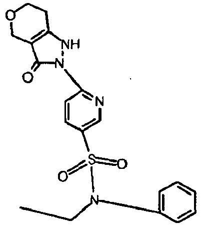

### 7.1. 4-oxotétrahydro-2H-pyran-3-carboxylate de propyle

Le composé est préparé selon le mode opératoire décrit dans JACS Vol 119, n°18 p 4285-4291.

### 7.2. N-éthyl-6-(3-oxo-1,4,6,7-tétrahydropyrano[4,3-c]pyrazol-2(3H)-yl)-N-phénylpyridine-3-sulfonamide

Selon le procédé décrit dans l'exemple 1, on obtient, à partir de 0,47 g de 6-hydrazino-N-méthyl-N-pyridin-2-ylpyridine-3-sulfonamide et de 0,3 g de 4-oxotétrahydro-2H-pyran-3-carboxylate de propyle, 370 mg de N-éthyl-6-(3-oxo-1,4,6,7-tétrahydropyrano[4,3-c]pyrazol-2(3H)-yl)-N-phénylpyridine-3-sulfonamide sous forme de poudre blanche
Rendement = 57%
PF(°C)=164
M = C₁₉H₂₀N₄O₄S = 400; M+H = 401; Méthode 2: Tr=1,0 min.
RMN ¹H, d6-DMSO, 400MHz, δ (ppm) :12,0 (sl,1H); 8,6 (d,1H); 8,4 (s,1H); 8,0 (d,1H); 7,4 (m,3H); 7,1 (m,2H); 4,3 (s,2H); 3,85 (t,2H); 3,6 (q,2H); 2,6 (t,2,H); 1,0 (t,3H).

### Exemple 8: 4-benzyl-2-{5-[éthyl(phényl)sulfamoyllpyridin-2-yl}-5-oxo-4,5,6,7-tétrahydro-2H-pyrazolo[4,3-blpyridin-3-olate de sodium (Composé 35 du Tableau I)

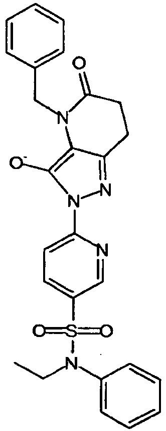

Selon le procédé décrit dans l'exemple 1, on obtient, à partir de de 0,8 g de 6-hydrazino-N-méthyl-N-pyridin-2-ylpyridine-3-sulfonamide et de 0,76 g de 1-benzyl-3,6,dioxopipéridine-2-carboxylate d'éthyle obtenu selonTetrahedron Vol 40, n°13 p 2505, 220 mg du composé attendu sous forme d'un solide blanc qui est repris par 6 mL d' un mélange eau/CH3CN (5 :1) et 1 éq de NaOH 1 N, puis lyophilisé. On obtient ainsi 225 mg de 4-benzyl-2-{5-[éthyl(phenyl)sulfamoyl]pyridin-2-yl}-5-oxo-4,5,6,7-tétrahydro-2H-pyrazolo[4,3-b]pyridin-3-olate de sodium sous forme d'un lyophilisat blanc.
Rendement =12%
PF(°C)>250
M = C₂₆H₂₅N₅O₄S= 503; M+H = 504; Méthode 2: Tr=1.16 min.
RMN ¹H, d6-DMSO, 400MHz, δ (ppm) : 8,7(d,1H); 8,4 (s,1H); 7,8 (d,1H); 7,5-7,1 (m,10H); 4,7 (s,2H); 4,2 (s,2H); 3,6 (q,2H); 3,1 (s,2H); 1,0 (t,3H).

### Exemple 9: 2-(4-éthylpyridin-2-yl)-2,6-dihydro-4H-thiéno[3,4-c]pyrazol-3-olate de sodium (Composé 57 du Tableau I)

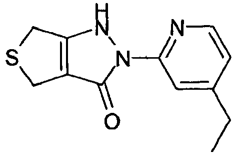

### 9.1. 4-éthyl-2-hydrazinopyridine

A une solution de 5 g (40,9 mmoles) de 4-éthylpyridin-2-amine dans 10 mL d'H₂SO₄ conc. à 0°C, on ajoute 8 mL d'un mélange H₂SO₄/HNO₃ (1:1) à une température comprise entre 0 et 10°C, l'agitation est maintenue 1 h à 0°C. Le milieu réactionnel est ensuite versé sur dans 100 g de glace et le précipité blanc obtenu est filtré, lavé successivement avec 10 mL d'eau, 10 mL d'Et₂O, et 10 mL de pentane. On reprend le solide obtenu par 100 mL de NaOH 10N à 0°C et on ajoute 7,76 g (187 mmoles) de zinc en poudre puis le milieu réactionnel est agité 3 h à 0°C. Le milieu réactionnel est ensuite filtré sur célite et le filtrat est extrait par de l'AcOEt (3X200 mL). Les phases organiques sont rassemblées, séchées sur Na₂SO₄, filtrées puis concentrées sous pression réduite. On obtient 4,3 g de 4-éthyl-2-hydrazinopyridine sous forme d'huile rouge qui est engagée telle quelle à l'étape suivante.
Rendement =77%
RMN ¹H, CDCl₃, 400MHz, δ (ppm) : 7,9 (d,1H); 7,2 (sl,1H); 6,5 (s,1H); 6,4 (d,1H); 4,1 (s,2H); 2,5 (q,2H); 1,1 (t,3H).

### 9.2. 2-(4-éthylpyridin-2-yl)-2,6-dihydro-4H-thiéno[3,4-c]pyrazol-3-olate de sodium

Selon le procédé décrit dans l'exemple 8, on obtient, à partir de 0,88 g de 4-éthyl-2-hydrazinopyridine et de 1,0 g de 4-oxotétrahydrothiophéne-3-carboxylate de méthyle". 225 mg de 2-(4-éthylpyridin-2-yl)-2,6-dihydro-4H-thiéno[3,4-c]pyrazol-3-olate de sodium sous forme d'un lyophilisat blanc.
Rendement =15%
PF(°C) >260°C
M = C₁₂H₁₃N₃OS = 247; M+H = 248; Méthode 2 : Tr=1,05 min.
RMN ¹H, d6-DMSO, 400MHz, δ (ppm) : 8,2 (s,1H); 8,1 (d,1H); 6,7 (d,1H); 3,7 (s,2H); 3,5 (s,2H); 2,5 (q,2H); 1,1 (t, 3H).

### Exemple 10: 2-{5-[tert-butyl(méthyl)sulfamoyl]pyridin-2-yl}-2,6-dihydro-4H-thiéno[3,4-c]pyrazol-3-olate de sodium (Composé 36 du Tableau I)

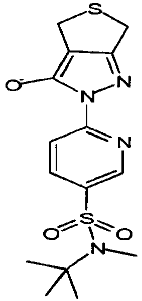

### 10.1. N-tert-butyl-6-chloropyridine-3-sulfonamide

Selon le procédé décrit dans l'exemple 4.1, on obtient, à partir de 2g (9,43 mmoles) de chlorure de 6-chloropyridine-3-suffonyle et de 0,99mL (9,43mmoles) de tertbutylamine, 1,91 g de N-tert-butyl-6-chloropyridine-3-sulfonamide sous forme d'un solide blanc.
Rendement =82%
RMN ¹H, CDCl₃, 400MHz, δ (ppm) : 8,8 (d,1H); 8 (dd,1H); 7,4 (d,1H); 4,5 (sl,1H); 1,2 (s,9H).

### 10.2. N-tert-butyl-6-chloro-N-méthylpyridine-3-sulfonamide

Un mélange de 0,97 g (3,9 mmoles) de N-tert-butyl-6-chloropyridine-3-sulfonamide, de 2,43 mL (39 mmoles) d'iodure de méthyle et de 5,4 g (39 mmoles) de K₂CO₃ dans 40 mL d'acétone est chauffé 12h au reflux. Le milieu réactionnel est filtré à TA, et le filtrat est concentré sous pression réduite. Le résidu obtenu est purifié sur gel de silice en éluant avec un gradient cyclohexane / AcOEt de 0 à 20 % d'AcOEt, on obtient 0,66 g de N-tert-butyl-6-chloro-N-méthylpyridine-3-sulfonamide sous forme d'un solide jaune.
Rendement =65%
RMN ¹H, CDCl₃, 400MHz, δ (ppm) : 8,8 (d,1H); 8 (dd,1H); 7,4 (d,1H);3(s,3H); 1,4 (s,9H).

### 10.3. N-tert-butyl-6-hydrazino-N-méthylpyridine-3-sulfonamide

Selon le procédé décrit dans l'exemple 4.2, on obtient, à partir de 0,66 g (2,53 mmoles) de N-tert-butyl-6-chloro-N-méthylpyridine-3-sulfonamide et de 0,46 mL d'hydrazine monohydrate, 0,55 g de N-tert-butyl-6-hydrazino-N-methylpyridine-3-sulfonamide, sous forme d'un solide blanc, qui est engagé tel quel à l'étape suivante.
Rendement = 84%
RMN ¹H, CDCl₃, 400MHz, δ (ppm) : 8,8 (d,1H); 8 (dd,1H); 7 (d,1H); 6,6 (sl,1H); 4,1 (sl,2H); 3,1 (s,3H); 1,5 (s,9H).

### 10.4. 2-{5-[tert-butyl(méthyl)sulfamoyl]pyridin-2-yl}-2,6-dihydro-4H-thiéno[3,4-c]pyrazol-3-olate de sodium

Selon le procédé décrit dans l'exemple 8, on obtient, à partir de 0,55 g de N-tert-butyl-6-hydrazino-N-méthylpyridine-3-sulfonamide et de 0,34 g de 4-oxotétrahydrothiophéne-3-carboxylate de méthyle, 0,34 g de 2-{5-[tert-butyl(méthyl)sulfamoyl]pyridin-2-yl}-2,6-dihydro-4H-thiéno[3,4-c]pyrazol-3-olate de sodium sous forme d'un lyophilisat blanc.
Rendement =67%
PF(°C) = 130
M = C₁₅H₂₀N₄O₃S2 = 368; M+H = 369; Méthode 2: Tr= 1,09 min.
RMN ¹H, d6-DMSO, 400MHz, δ (ppm) : 8,6 (m,2H); 8,0 (s,1H); 3,75 (s,2H); 3,6 (s,2H); 2,9 (s,3H); 1,3 (s,9H).

### Exemple 11: 2-[5-(tert-butylcarbamoyl)pyridin-2-yl]-2,6-dihydro-4H-thiéno[3,4-c]pyrazol-3-olate de sodium (Composé 33 du Tableau I)

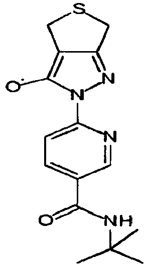

### 11.1. Acide 6-(3-oxo-4,6-dihydro-1H-thiéno[3,4-c]pyrazol-2(3H)-yl)pyridine-3-carboxylique

Selon le procédé décrit dans l'exemple 1, on obtient, à partir de 1,75 g d'acide 6-hydrazinopyridine-3-carboxylique et de 1,8 g de 4-oxotétrahydrothiophéne-3- carboxylate de méthyle, 2,5 g d'acide 6-(3-oxo-4,6-dihydro-1H-thiéno[3,4-c]pyrazol-2(3H)-yl)pyridine-3-carboxylique sous forme d'un solide jaune.
Rendement =94%
RMN ¹H, d6-DMSO, 400MHz, δ (ppm) : 12,5 (sl,1H); 9,0 (s,1H); 8,4 (d,1H); 8,3 (sl,1H); 4,1 (s,2H); 3,8 (s,2H).

### 11.2. 2-[5-(tert-butylcarbamoyl)pyridin-2-yl]-2,6-dihydro-4H-thiéno[3,4-c]pyrazol-3-olate de sodium

A un mélange de 200mg (0,84 mmoles) d'acide 6-(3-oxo-4,6-dihydro-1H-thiéno[3,4-c]pyrazol-2(3H)-yl) pyridine-3-carboxylique, de 90 µl (0,84 mmoles) de tertbutylamine et de 0,46 mL de DIEA, dans 3 mL de CH₃CN refroidi à 0°C, on ajoute 365 mg de TBTU. On laisse ensuite le milieu réactionnel remonter lentement à TA et l'agitation est maintenue 12 h. Le milieu réactionnel est concentré sous pression réduite, le résidu obtenu est repris par 20 mL d'eau et extrait avec du DCM (3 x 20 mL), séché sur Na2SO4, filtré et concentré sous pression réduite, purifié sur gel de silice en éluant avec un gradient DCM / MeOH de 0 à 10 % de MeOH. On obtient 66 mg du composé attendu qui est repris par 2 mL d'un mélange eau /CH₃CN (5:1) et 1 éq de NaOH 1 N, puis lyophilisé. On obtient ainsi 66 mg de 2-[5-(tert-butylcarbamoyl)pyridin-2-yl]-2,6-dihydro-4H-thiéno[3,4-c]pyrazol-3-olate de sodium sous forme d'un lyophilisat blanc.
Rendement =25%
PF(°C) >260°C
M = C₁₅H₁₇N₄O₂S = 317; M+H = 318; Méthode 2: Tr=0.94 min.
RMN ¹H, d6-DMSO, 400MHz, δ (ppm) : 8,7 (s,1 H); 8,5 (d,1 H); 8,1 (d,1 H); 7,6 (s,1H); 3,75 (s,1H); 3,6 (s,2H); 1,4 (s,9H).

### Exemple 12: 6-(3-oxo-4,6-dihydro-1H-thiéno[3,4-c]pyrazol-2(3H)-yl)pyridine-3-carboxylate de tert-butyle (Composé 30 Tableau I)

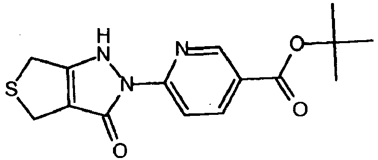

### 12.1. 6-chloropyridine-3-carboxylate de tert-butyle

A une suspension de 1g (6,35 mmoles) d'acide 6-chloropyridine-3-carboxylique dans 10 ml de toluène au reflux, on ajoute au goutte à goutte 7,6 ml (31,75 mmoles) de 1,1-di-tert-butoxy-N,N-diméthylméthanamine, le mélange reéactionnel est ensuite chauffé 1/2h au reflux. Après retour à TA., le milieu réactionnel est repris par 200 ml d'AcOEt, lavé successivement à l'eau (2 X 100 mL), avec une solution saturée en NaHCO₃ (100 mL) et de la saumure (100 mL), séché sur Na₂SO₄, filtré et concentré sous pression réduite. On obtient 1,16 g de 6-chloropyridine-3-carboxylate de tert-butyle, sous forme d'une huile jaune qui est engagée telle quelle à l'étape suivante.
Rendement = 86%
RMN ¹H, d6-DMSO, 400MHz, δ (ppm) : 8,8 (s,1 H); 8,2 (d,1 H); 7,3 (d,1 H); 1,5 (s,9H).

### 12.2. 6-hydrazinopyridine-3-carboxylate de tert-butyle

Selon le procédé décrit dans l'exemple 4.2 on obtient, à partir de 1,16 g de 6-chloropyridine-3-carboxylate de tert-butyle et de 1ml d'hydrazine hydrate, 900 mg de 6-hydrazinopyridine-3-carboxylate de tert-butyle sous forme d'un solide qui est engagé tel quel à l'étape suivante.
Rendement =79%
RMN ¹H, d6-DMSO, 400MHz, δ (ppm) : 8,7 (s,1H); 8,0 (d,1 H); 6,6 (d,1 H); 6,3 (sl,1H); 3,2 (sl,2H); 1,5 (s,9H).

### 12.3. 6-(3-oxo-4,6-dihydro-1H-thiéno[3,4-c]pyrazol-2(3H)-yl)pyridine-3-carboxylate de tert-butyle

Selon le procédé décrit dans l'exemple 1, on obtient, à partir de 0,75 g de 6-hydrazinopyridine-3-carboxylate de tert-butyle et de 0,58 g de 4-oxotétrahydrothiophéne-3- carboxylate de méthyle, 0,7 g de 6-(3-oxo-4,6-dihydro-1H-thiéno[3,4-c]pyrazol-2(3H)-yl)pyridine-3-carboxylate de tert-butyle sous forme d'un solide blanc. Rendement =61%
PF(°C) > 250°C
M = C₁₅H₁₇N₃O₃S = 319; M+H = 320; Méthode 2: Tr=1,27 min
RMN ¹H, d6-DMSO, 400MHz, δ (ppm) : 8,9 (s,1H); 8,4 (d,1 H); 8,2 (d,1 H); 4,0 (s,2H); 3,7 (s,2H), 3,4 (sl, 1 H); 1,6 (s,9H).

### Exemple 13: 2-[5-(propan-2-yl)pyridin-2-yl]-2,6-dihydro-4H-thieno[3,4-c]pyrazol-3-olate de sodium (Composé 44 Tableau I)

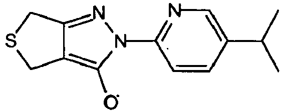

### 13.1. 5-(prop-1-èn-2-yl)pyridin-2-amine

Un mélange de 2 g (11.56 mmoles) de 5-bromopyridin-2-amine, de 2.39 mL(13.87 mmoles) de 4,4,5,5-tétraméthyl-2-(prop-1-èn-2-yl)-1,3,2-dioxaborolane, de 3.83 g (27.74 mmoles) de K2CO3 dans 30 mL de DME et 10 mL d'eau est agité 5min sous argon puis on ajoute 1 g (2.1 mmoles) de palladium bis (tri-t-butylphosphine). Le milieu réactionnel est chauffé 3h à 80°C. Après retour à TA., le milieu réactionnel est repris par 40 ml d'eau et extrait à l'AcOEt (3x30 mL). Les phases organiques sont lavées avec une solution HCl 0.5N (3x30mL). Les phases aqueuses sont neutralisées pars une solution de soude 1 N puis extraites avec de l'AcOEt (2x50mL). Les phases organiques rassemblées sont séchées sur Na₂SO₄, filtrées et concentrées sous pression réduite. On obtient 1,10 g de 5-(prop-1-èn-2-yl)pyridin-2-amine, sous forme d'un solide jaune qui est engagé tel quel à l'étape suivante.
Rendement = 71%
RMN 1H, CDCl3, 400MHz, δ (ppm) : 8,0 (s,1H); 7,6 (d,1H); 6,5 (d,1H); 5,3 (s,1H); 5,0 (s, 1 H); 4,5 (sl,2H); 2,1 (s,3H);

### 13.2. 5-(propan-2-yl)pyridin-2-amine

Dans une fiole de Parr, un mélange de 1.1 g (8.2mmoles) de 5-(prop-1-èn-2-yl)pyridin-2-amine dans 40 mL de MeOH et 0.11 g de Pd/C 10% est hydrogéné sous 7 bars pendant 24h. Le mélange réactionnel est ensuite filtré sur papier whatman GF/F et concentré sous pression réduite. On obtient ainsi 1.07g de 5-(propan-2-yl)pyridin-2-amine sous forme d'une huile jaune utilisée tel quell à l'étape suivante.
Rendement = 96%.
RMN 1 H, CDCl3, 400MHz, δ (ppm) : 8,0 (s, 1 H); 7,4 (1 H,dd); 6,5 (d, 1 H); 4,4 (sl, 2H); 2,8 (m, 1 H); 1,2 (s, 6H)

### 13.3. 2-hydrazinyl-5-(propan-2-yl)pyridine

Selon le procédé décrit dans l'exemple 9.1, on obtient à partir de 1.07 g de 5-(propan-2-yl)pyridin-2-amine , 0.52 g d e 2-hydrazinyl-5-(propan-2-yl)pyridine sous forme d'une huile marron.
Rendement = 44%

### 13.4. 2-[5-(propan-2-yl)pyridin-2-yl]-2,6-dihydro-4H-thiéno[3,4-c]pyrazol-3-olate de sodium

Selon le procédé décrit dans l'exemple 8, on obtient, à partir de 520 mg de 2-hydrazinyl-5-(propan-2-yl)pyridine et de 251 mg de 4-oxotétrahydrothiophéne-3- carboxylate de méthyle, 102 mg de 2-[5-(propan-2-yl)pyridin-2-yl]-2,6-dihydro-4H-thiéno[3,4-c]pyrazol-3-olate de sodium sous forme d'un lyophilisat.
Rendement = 11%
PF(°C) > 260°C
M = C₁₃H₁₅N₃OS = 261; M+H = 262; Méthode 2: Tr=1,26 min
RMN ¹H, d6-DMSO, 400MHz, δ (ppm) : 8.4 (d,1H); 8,1 (s,1H); 7.6 (1H,dd); 3.7 (s, 2H); 3.6 (s, 2H); 2.9 (1 H,qt); 1,2 (s, 6H).

### Exemple 14: 2-[4-(pyridin-3-ylméthoxy)pyridin-2-yl]-2,6-dihydro-4H-thiéno[3,4-c]pyrazol-3-olate de sodium ( Composé 60 du Tableau II )

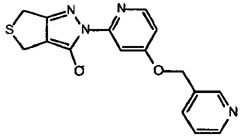

### 14.1. 2-chloro-4-(pyridin-3-ylméthoxy)pyridine

Un mélange de 2 g (15.4 mmoles) de 2-chloropyridin-4-ol, de 3.82 g (15.1 mmoles) de bromhydrate de 3 (bromométhyl)pyridine, de 3g (75.6 mmoles) de soude et de 1.39 g (4.3 mmoles) de bromure de tétrabutylammonium dans 90 mL de toluène est chauffé 12h à reflux.. Après retour à TA., le milieu réactionnel est repris par 300mL d'AcOEt, lavé successivement à l'eau (2 X 100 mL), avec une solution saturée en NaHCO₃ (100 mL) et de la saumure (100 mL), séché sur Na₂SO₄, filtré, concentré sous pression réduite puis purifié sur chromatographie sur gel de silice en éluant avec un mélange DCM / MeOH de 98 :2. On obtient 2.5 g de 2-chloro-4-(pyridin-3-ylméthoxy)pyridine, sous forme d'une huile jaune.
Rendement = 73%
RMN 1 H, CDCl3, 400MHz, δ (ppm) : 8,7 (s, 1 H); 8,6 (d, 1 H); 8,25 (d, 1 H); 7,8 (d, 1 H); 7,4 (1 H,dd); 7,0 (s, 1 H); 6,8 (d, 1 H) ; 5,1 (s, 2H)

### 14.2. 2-hydrazinyl-4-(pyridin-3-ylméthoxy)pyridine

Un mélange de 0.5 g (2.27 mmoles) de 2-chloro-4-(pyridin-3-ylméthoxy)pyridine et de 2 mL (40.5 mmoles) d'hydrazine hydrate est chauffé à 80°C pendant 12h. Le milieu réactionnel est ensuite repris par 30 ml d'eau et extrait à l'AcOEt (3*30 mL). Les phases organiques sont rassemblées, avées avec de la saumure (20 mL) puis séchées sur Na₂SO₄, filtrées et concentrées sous pression réduite et purifiées sur gel de silice en éluant avec un mélange DCM / MeOH de 95 :5 . On 197 mg de 2-hydrazinyl-4-(pyridin-3-ylméthoxy)pyridine sous forme d'une huile jaune.
Rendement = 37%
RMN ¹H, d6-DMSO, 400MHz, δ (ppm) : 8.7 (s, 1H); 8,5 (d,1H); 7,9 (d, 1H); 7,8 (d, 1H) ; 7,4 (dd, 1 H); 7,3 (s, 1 H); 5,2 ( s, 2H); 4,1 (s,2H);

### 14.3. 2-[4-(pyridin-3-ylméthoxy)pyridin-2-yl]-2,6-dihydro-4H-thiéno[3.4-c]pyrazol-3-olate de sodium

Selon le procédé décrit dans l'exemple 8, on obtient, à partir de 197 mg de 2-hydrazinyl-4-(pyridin-3-ylméthoxy)pyridine et de 146 mg de 4-oxotétrahydrothiophéne-3-carboxylate de méthyle, 225 mg de 2-(4-éthylpyridin-2-yl)-2,6-dihydro-4H-thiéno[3,4-c]pyrazol-3-olate de sodium sous forme d'un lyophilisat blanc.
Rendement =48%
PF(°C) >260°C
M = C₁₆H₄N4O₂S = 326; M+H = 327; Méthode 2 : Tr=0.71 min.
RMN ¹H, d6-DMSO, 400MHz, δ (ppm) : 8.8 (s,1H); 8,6 (d,1 H); 8,2 (s,1H); 8,1 (d, 1H); 8,0 (d, 1 H); 7,5 (t, 1 H); 6,5 (d,1 H); 5,2 ( s, 2H); 3,75 (s,2H); 3,5 (s,2H)

### Exemple 15A: 2-[5-(3-tert-butyl-1,2,4-oxadiazol-5-yl)pyridin-2-yl]-1,2,4,6-tetrahydro-3H-thieno[3,4-c]pyrazol-3-one (Composé 50A du Tableau I)

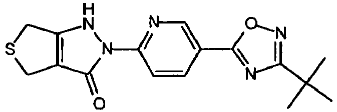

### 15A.1. 5-(3-tert-butyl-1,2,4-oxadiazol-5-yl)-2-chloropyridine

Dans un montage à Dean-Stark, à une solution de 3.8 g (21.5 mmoles) de chlorure de 6-chloropyridine-3-carbonyle dans 100 mL de toluène, on ajoute par petites portions 2.5 g (21.5 mmoles) N-hydroxy-2,2-diméthylpropanimidamide, puis le milieu réactionnel est agité 2 h à T.A puis chauffé 2h. Après retour à T.A, le milieu est concentré sous pression réduite et purifié par chromatographie sur gel de silice en éluant avec un gradient cyclohexane / AcOEt de 0 à 10 % d'AcOEt .On obtient 4.1 g de 5-(3-tert-butyl-1,2,4-oxadiazol-5-yl)-2-chloropyridine sous forme d'un solide blanc.
Rendement = 81%
RMN 1 H, CDCl3, 400MHz, δ (ppm) : 9,2 (s, 1 H); 8,4 (dd, 1 H); 7,5 (d, 1H) ; 7,8 (d, 1 H); 1,4 (s, 9H)

### 15A.2. 5-(3-tert-butyl-1,2,4-oxadiazol-5-yl)-2-hydrazinylpyridine

Selon le procédé décrit dans l'exemple 4.2 on obtient, à partir de 2 g de 5-(3-tert-butyl-1,2,4-oxadiazol-5-yl)-2-chloropyridine et de 3.9 mL d'hydrazine hydrate, 1.9 g de 5-(3-tert-butyl-1,2,4-oxadiazol-5-yl)-2-hydrazinylpyridine sous forme d'un solide qui est engagé tel quel à l'étape suivante.
Rendement =97%
RMN ¹H, d6-DMSO, 400MHz, δ (ppm) : 8,7 (s,1H); 8,1 (d,1H); 6,9 (d,1H); 6,5 (sl,1H); 3,5 (sl,2H); 1,3 (s,9H).

### 15A.3. 2-[5-(3-tert-butyl-1,2,4-oxadiazol-5-yl)pyridin-2-yl]-1,2,4,6-tétrahydro-3H-thiéno[3,4-c]pyrazol-3-one

Selon le procédé décrit dans l'exemple 1, on obtient, à partir de 0.6 g de 5-(3-tert-butyl-1,2,4-oxadiazol-5-yl)-2-hydrazinylpyridine et de 0.41 g de 4-oxotétrahydrothiophéne-3-carboxylate de méthyle, 0.32 g de 2-[5-(3-tert-butyl-1,2,4-oxadiazol-5-yl)pyridin-2-yl]-1,2,4,6-tétrahydro-3H-thiéno[3,4-c]pyrazol-3-one sous forme d'un solide blanc.
Rendement =38%
PF(°C) =240°C
M = C₁₆H₁₇N₅O₂S = 343; M+H = 344; Méthode 2 : Tr=1.34 min.
RMN ¹H, d6-DMSO, 400MHz, δ (ppm) : 12,3 (sl, 1H); 9,1 (s, 1H); 8.6 (d,1H); 8,4 (d,1H); 4,0 (s, 2H); 3,8 (s,2H); 1;4 (s,9H).

### Exemple 15B: 2-[5-(5-tert-butyl-1,2,4-oxadiazol-3-yl)pyridin-2-yl]-1,2,4,6-tetrahydro-3H-thieno[3,4-c]pyrazol-3-one (Composé 50B du Tableau I)

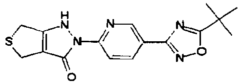

### 15B.1 5-(5-tert-butyl-1,2,4-oxadiazol-3-yl)-2-chloropyridine

Un mélange de 1.5 g (8.74 mmoles) de 6-chloro-N'-hydroxypyridine-3-carboximidamide et de 1.13 mL(9.18 mmoles) de chlorure de triméthylacétyle, dans 15 mL de toluène, est agité 1h à T.A puis chauffé 4h au reflux.Le milieu est filtré .L'insoluble formé est dissous dans 15mL d'AcOH puis chauffé à 60°C pendant 5h. Après retour à T.A, le milieu est concentré sous pression réduite puis purifié par chromatographie sur gel de silice en éluant avec un mélange cyclohexane / AcOEt (9 :1).On obtient 0.39 g de 5-(5-tert-butyl-1,2,4-oxadiazol-3-yl)-2-chloropyridine sous forme d'un solide blanc.
Rendement = 21 %

### 15.B2 2-[5-(5-tert-butyl-1,2,4-oxadiazol-3-yl)pyridin-2-yl]-1,2,4,6-tetrahydro-3H-thieno[3,4-c]pyrazol-3-one (Composé 50 B du Tableau I)

Le composé est obtenu, selon les procédés 15.A2-15.A3, à partir du 5-(5-tert-butyl-1,2,4-oxadiazol-3-yl)-2-chloropyridine et du 4-oxotétrahydrothiophéne-3-carboxylate de méthyle sous forme de solide blanc.
PF(°C) =240°C
M = C₁₆H₁₇N₅O₂S = 343; M+H = 344; Méthode 2 : Tr=1.39 min.
RMN ¹H, d6-DMSO, 400MHz, δ (ppm) : 12,3 (sl, 1H); 9,1 (s, 1H); 8.5 (d,1H); 8,4 (d,1H); 4,1 (s, 2H); 3,8 (s,2H); 1;5 (s,9H)

### Exemple 16: N-cyclopentyl-N-(2,3-dihydroxypropyl)-6-(3-oxo-4,6-dihydro-1H-thieno[3,4-c]pyrazol-2(3H)-yl)pyridine-3-sulfonamide (Composé 53 du Tableau I)

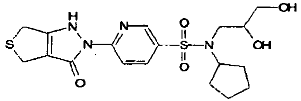

### 16.1. 6-chloro-N-cyclopentylpyridine-3-sulfonamide

Selon le procédé 4.1 , on obtient à partir de 5g de chlorure de 6-chloropyridine-3-sulfonyle et de 2g de cyclopentylamine , 5.1 g 6-chloro-N-cyclopentylpyridine-3-sulfonamide de sous forme d'un solide jaune.
Rendement= 83%
RMN ¹H, CDCl₃, 400MHz, δ (ppm): 8,9 (s,1H); 8,0 (d,1H); 7,4 (d,1H); 4,5 (d,1H); 3,6(q, 1H); 1,8-1,2 (m, 8H)

### 16.2 6-chloro-N-cyclopentyl-N-(prop-2-èn-1-yl)pyridine-3-sulfonamide

Selon le procédé 10.2, on obtient à partir de 1 g de 6-chloro-N-cyclopentylpyridine-3-sulfonamide et de 0.42 mL de bromure d'allyle, 1.2 g de 6-chloro-N-phényl-N-(prop-2-èn-1-yl)pyridine-3-sulfonamide sous forme d'une huile orange.
Rendement= 94%
RMN ¹H, CDCl₃, 400MHz, δ (ppm): 8,9 (s,1H); 8,0 (d,1H); 7,4 (d,1H); 5,7 ( dd, 1 H); 5,2 (dd, 1H); 5,1 (d,1H); 4,2 (m,1H); 3,7 (d, 2H); 1,8-1,2 (m, 8H)

### 16.3. 6-chloro-N-cyclopentyl-N-(2,3-dihydroxypropyl)pyridine-3-sulfonamide

A une solution de 1,.1 g (3.7 mmoles) de 6-chloro-N-cyclopentyl-N-(prop-2-èn-1-yl)pyridine-3-sulfonamide dans 15mL d'un mélange (1/1) de tBuOH et d'eau , on ajoute, à T.A, 1.2 g (10.3 mmoles) de NMO et 0,52 mL(0.04 mmoles) d'OsO4 2.5% dans le tBuOH. L'agitation est maintenue 12h . Le milieu est alors dilué avec 200mL d'eau et extrait avec de l'Et₂O (2X 100mL), séché sur Na₂SO₄, filtré et concentré sous pression réduite. On obtient 0.92 g de 6-chloro-N-cyclopentyl-N-(2,3-dihydroxypropyl)pyridine-3-sulfonamide sous forme d'un solide marron utilisé tel quel à l'étape suivante.
Rendement= 75%.
RMN ¹H, CDCl₃, 400MHz, δ (ppm) : 8,8 (s, 1H); 8,1 (d,1H); 7,5 (d, 1H); 4,3 (m, 1H); 4,0 (m, 1 H); 3,6 (dd,2H); 3,2 (dd, 2H); 1,8-1,4 (m, 10H)

### 16.4. 6-chloro-N-cyclopentyl-N-[(2,2-diméthyl-1,3-dioxolan-4-yl) méthy]pyridine-3-sulfonamide

Un mélange de 0.83 g (2.48 mmoles) de 6-chloro-N-cyclopentyl-N-(2,3-dihydroxypropyl)pyridine-3-sulfonamide, de 0.67 mL (5.45 mmoles), de 2,2 diméthoxypropane et de 47 mg de pTsOH dans 5 mL de DMF est agité 3h à T.A. Le milieu est repris par 100 mL d'AcOEt, lavé avec 50 mL d'une solution saturée en NaHCO₃, 50mL d'eau puis séché sur Na₂SO₄, filtré et concentré sous pression réduite. On obtient 0.92 g 6-chloro-N-cyclopentyl-N-[(2,2-diméthyl-1,3-dioxolan-4-yl) méthyl]pyridine-3-sulfonamide sous forme d'une huile marron utilisée tel quel à l'étape suivante.
Rendement= 98%.
RMN ¹H, CDCl₃, 400MHz, δ (ppm) : 8,8 (s, 1H); 8,0 (d,1H); 7,3 ( d, 1H); 4,3 (m, 1H); 4,0 m,2H); 3,7 (m, 1H), 3,3 (dd,1H); 3,1 (dd,1H);1,8-1,3 (m, 8H); 1,3 (s, 3H); 1,2 (s,3H)

### 16.5. N-cyclorpentyl-N-[(2,2-diméthyl-1,3-dioxolan-4-yl)méthyl]-6-hydrazinylpyridine-3-sulfonamide

Selon le procédé 4.2, on obtient à partir de 0.92 g de 6-chloro-N-cyclopentyl-N-[(2,2-diméthyl-1,3-dioxolan-4-yl) méthyl]pyridine-3-sulfonamide et de 0.24 mL d'hydrazine hydrate, 0.85 g de N-cyclopentyl-N-[(2,2-diméthyl-1,3-dioxolan-4-yl)méthyl)-6-hydrazinylpyridine-3-sulfonamide sous forme d'une huile jaune.
Rendement= 99%

### 16.6. N-cyclopentyl-N-[(2,2-diméthyl-1,3-dioxolan-4-yl) méthyl]-6-(3-oxo-4,6-dihydro-1H-thieno[3,4-c]pyrazol-2(3H)-yl)pyridine-3-sulfonamide

Selon le procédé 1, on obtient, à partir de 0.4 g de N-cyclopentyl-N-[(2,2-diméthyl-1,3-dioxolan-4-yl)méthyl]-6-hydrazinylpyridine-3-sulfonamide et 0.173 g de 4-oxotétrahydrothiophéne-3-carboxylate de méthyle, 470 mg N-cyclopentyl-N-[(2,2-diméthyl-1,3-dioxolan-4-yl)méthyl]-6-(3-oxo-4,6-dihydro-1H-thiéno[3,4-c]pyrazol-2(3H)-yl) pyridine-3-sulfonamide sous forme d'un solide marron.
Rendement= 97%

### 16.7.N-cyclopentyl-N-(2,3-dihydroxypropyl)-6-(3-oxo-4,6-dihydro-1H-thieno[3,4-c]pyrazol-2(3H)-yl)pyridine-3-sulfonamide

Une solution de 470mg (0;54 mmoles) de 6 N-cyclopentyl-N-[(2,2-diméthyl-1,3-dioxolan-4-yl) méthyl]-6- (3-oxo-4,6-dihydro-1H-thièno[3,4-c] pyrazol-2(3H)-yl) pyridine-3-sulfonamide dans 1mL d'AcOH est chauffé à 80°C pendant 4h.Le milieu est concentré sous pression réduite puis purifié en phase inverse sur colonne de C18 en éluant avec un gradient HCl 10⁻³N / CH3CN de 0 à 100 % de CH3CN .On obtient 48 mg de N-cyclopentyl-N-(2,3-dihydroxypropyl)-6-(3-oxo-4,6-dihydro-1H-thieno[3,4-c]pyrazol-2(3H)-yl)pyridine-3-sulfonamide sous forme d'un solide blanc.
Rendement= 30%
PF(°C)=190
M = C₁₈H₂₄N₄O₅S₂ = 440 ; M+H = 441; Méthode 2: Tr= 0.89 min.
RMN ¹H, d6-DMSO, 400MHz, δ (ppm): 12,1 (sl,1H); 8,7 (s,1H); 8,3 (sl, 1 H) ;8,2 (d,1H); 4,7 (s, 1H); 4,5 (t ,1H); 4,1 (m, 1H); 3,9 (s, 2H); 3,6 (m,3H);3,4-3,2 (m, ,3H); 2,8 (dd,1 H); 1,7-1,2 (m, 8H)

### Exemple 17: 6-[5-(methylsulfonyl)-3-oxo-1,3,4,5,6,7-hexahydro-2H-pyrazolo[4,3-c]pyridin-2-yl]pyridine-3-carboxylate de 2,2-diméthylpropyle (Composé 54 du Tableau I)

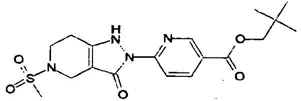

### 17.1. méthyl 1-(méthylsulfonyl)-oxopipéridine-3-carboxylate de méthyle

A un mélange de 1g (6.36 mmoles) de chlorhydrate de 4-oxopipéridine-3-carboxylate de méthyle,de 2.2 mL de NEt3 dans 12 mL de DCM, on ajoute à T.A au goutte à goutte , 0.5 mL de chlorure de mésyle .Le milieu est agité 1h puis repris dans 50 mL d'AcOEt , lavé avec 20 mL d'eau et 20 mL de saumure puis séché sur Na₂SO₄, filtré et concentré sous pression réduite. On obtient 0,35 g de méthyl 1-(méthylsulfonyl)-4-oxopipéridine-3-carboxylate de méthyle.
Rendement= 23%

### 17.2. 6-chloropyridine-3-carboxylate de 2,2-diméthylpropyle

A une solution de 10 g (56.8 mmoles) de chlorure de 6-chloropyridine-3-carbonyle dans 100 mL de toluène anh. sont ajoutés sous argon à T.A, 15 g (170.4 mmoles) de 2,2-diméthylpropanol. Le milieu réactionnel est chauffé ensuite 6h à 80°C. Après retour à T.A, le milieu est concentré et le résidu obtenu est repris par 800 mL d'AcOEt, lavé successivement avec de l'eau (2x200mL), une solution saturée en NaHCO₃ (2x200mL) et de la saumure (100mL), séché sur Na₂SO₄, puis concentré sous pression réduite et purifié par chromatographie sur colonne de gel de silice en éluant avec un gradient cyclohexane / AcOEt de 0 à 5% d'AcOEt. On obtient 11.9 g de 6-chloropyridine-3-carboxylate de 2,2-diméthylpropyle sous forme d'une poudre blanche.
Rendement= 92%.
RMN ¹H, CDCl₃, 400MHz, δ (ppm) : 7,5 (m, 5H); 4,2 (m, 5H); 3,0 (dd,2H; 1,0 (t,6H)

### 17.3. 6-hydrazinylpyridine-3-carboxylate de 2,2-diméthylpropyle

Selon le procédé décrit dans l'exemple 4.2 on obtient, à partir de 11,9 g (52.26 mmoles) de 6-chloropyridine-3-carboxylate de 2,2-diméthylpropyle, 4.3 g de 6-hydrazinylpyridine-3-carboxylate de 2,2-diméthylpropyle sous forme d'une poudre blanche.
Rendement= 37%
RMN ¹H, d6-DMSO, 400MHz, δ (ppm) : 8,7 (s, 1H); 8,15 (d,1H) ; 6,9 (d, 1H); 4,0 ( s,2H); 3.5 (sl, 1 H); 1,0 (s, 9H) .

### 17.4. 6-[5-(méthylsulfonyl)-3-oxo-1,3,4,5,6,7-hexahydro-2H-pyrazolo[4,3-c]pyridin-2-yl]pyridine-3-carboxylate de 2,2-diméthylpropyle

Selon le procédé 1 ,on obtient à partir de 0.21 g de méthyl 1-(méthylsulfonyl)-4-oxopipéridine-3-carboxylate de méthyle et de 0.2 g 6-hydrazinylpyridine-3-carboxylate de 2,2-diméthylpropyle,10 mg de 2,2-diméthylpropyl 6-[5-(méthylsulfonyl)-3-oxo-1,3,4,5,6,7-hexahydro-2H-pyrazolo[4,3-c]pyridin-2-yl]pyridine-3-carboxylate.
Rendement= 3%
PF(°C)=230
M = C₁₈H₂₄N₄O₅S = 408 ; M+H = 409; Méthode 2: Tr= 1.11 min.
RMN ¹H, d6-DMSO, 400MHz, δ (ppm): 12,2 (sl,1H); 9,0 (s,1H); 8,6 (sl, 1H);8,5 (d,1H); 4,1 (s, 2H); 4,0 (s ,2H); 3,5 (t,2H);3,0 (s ,3H); 2,8 (t,2H); 1,0 (s, 9H)

Les tableaux qui suivent illustrent les structures chimiques et les propriétés physiques de quelques exemples de composés selon l'invention.

Le **tableau I** illustre des composés de formule (I) selon l'invention dans laquelle R2 est en position beta. Ces composés sont appelés ci-après composés de formule (l').

Le **tableau II** illustre des composés de formule (I) selon l'invention dans laquelle R2 est en position gamma. Ces composés sont appelés ci-après composés de formule (I").

Les **tableaux I & II** qui suivent illustrent les structures chimiques et les propriétés physiques de quelques exemples de composés selon l'invention.
Dans ces tableaux :
- dans la colonne « sel », « - » représente un composé sous forme de base libre, alors que « CF₃COOH », « HCI » et « Na » représentent respectivement un composé sous forme de sel d'acide trifluoroacétique, sous forme de sels de chlorhydrate et sous forme de sels de sodium;
- dans les autres colonnes, « - » signifie que le substituant en question n'est pas présent sur la molécule ;
- Me, Et, n-Pr, i-Pr, n-Bu et i-Bu représentent respectivement les groupes méthyle, éthyle, n-propyle, isopropyle, n-butyle et isobutyle ;
- Ph et Bn représentent respectivement les groupes phényle et benzyle ;
- la colonne « PF » indique le point de fusion, en °C, du composé concerné ;
- dans la colonne « LC/MS » et la colonne « Méthode » sont indiqués respectivement, le pic MH⁺ identifié par spectrométrie de masse et la méthode analytique de chromatographie liquide haute performance utilisée et détaillée précédemment.

**TABLEAU I**

| | | | | | | | | | | | |
|---|---|---|---|---|---|---|---|---|---|---|---|
| 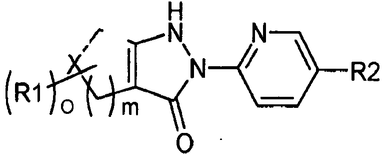 | | | | | | | | | | | |
| | | | | | | 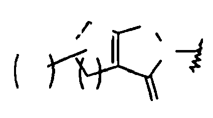 | | | | | |
| 1 | S | 1 | 1 | 0 | - | 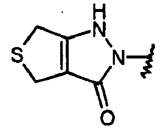 | H | 152 | 220 | - | 2 |
| 2 | CH₂ | 2 | 1 | 0 | - | 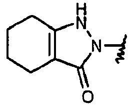 | 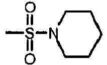 | 206 | 363 | - | 1 |
| 3 | -CH(Me)- | 1 | 2 | 0 | - | 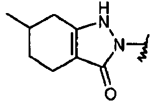 | 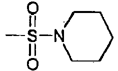 | 240 | 377 | - | 1 |
| 4 | CH₂ | 3 | 1 | 0 | - | 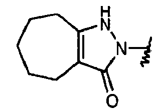 | 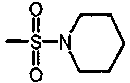 | 240 | 391 | - | 1 |

| N° | X | n | m | o | R1 | 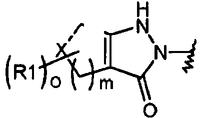 | | | | | |
|---|---|---|---|---|---|---|---|---|---|---|---|
| 5 | 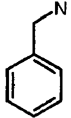 | 2 | 1 | 0 | - | 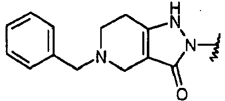 | 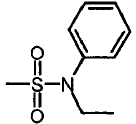 | 210 | 490 | CF₃CO₂H | 1 |
| 6 | CH₂ | 2 | 1 | 0 | - | 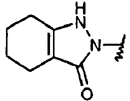 | 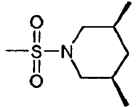 | 230 | 391 | - | 1 |
| 7 | CH₂ | 1 | 1 | 0 | - | 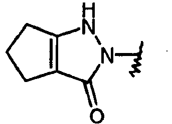 | H | 156 | 202 | - | 2 |
| 8 | 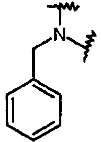 | 2 | 1 | 0 | - | 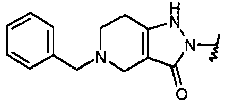 | 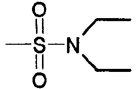 | 248 | 442 | - | 2 |
| 9 | S | 1 | 1 | 0 | - | 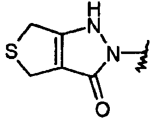 | 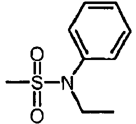 | 208 | 403 | - | 2 |
| 10 | 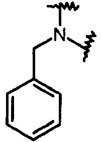 | 2 | 1 | 0 | - | 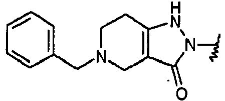 | 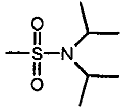 | 160 | 470 | - | 2 |
| 11 | -CH(OMe)- | 0 | 2 | 0 | - | 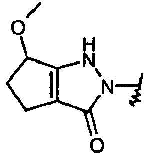 | H | 76 | 232 | - | 2 |
| 12 | S | 2 | 1 | 0 | - | 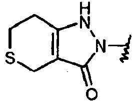 | H | 175 | 234 | - | 2 |
| 13 | S | 2 | 1 | 0 | - | 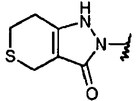 | 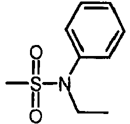 | 176 | 417 | - | 2 |
| 14 | S | 1 | 1 | 0 | - | 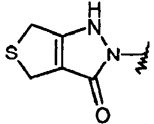 | 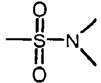 | 327 | >260 | - | 2 |
| 15 | S | 1 | 1 | 0 | - | 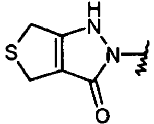 | 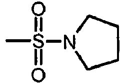 | 152 | 353 | - | 2 |
| 16 | S | 1 | 1 | 0 | - | 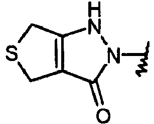 | 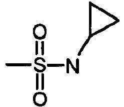 | 262 | 339 | - | 2 |
| 17 | S | 1 | 1 | 0 | - | 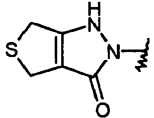 | 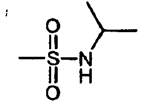 | 258 | 341 | - | 2 |
| 18 | S | 1 | 1 | 0 | - | 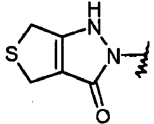 | 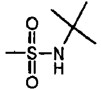 | 257 | 355 | - | 2 |
| 19 | S | 1 | 1 | 0 | - | 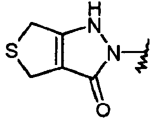 | 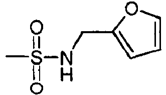 | 238 | 379 | - | 2 |
| 20 | S | 1 | 1 | 0 | - | 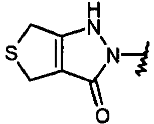 | 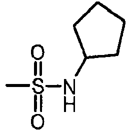 | >260 | 367 | - | 2 |
| 21 | S | 1 | 1 | 0 | - | 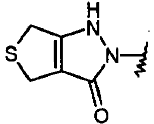 | 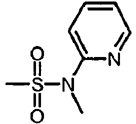 | 226 | 390 | - | 2 |
| 22 | S | 1 | 1 | 0 | - | 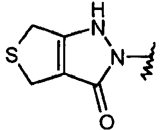 | 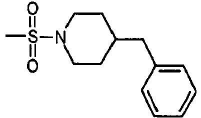 | 254 | 457 | - | 2 |
| 23 | S | 1 | 1 | 0 | - | 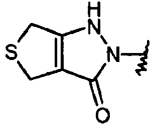 | 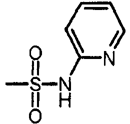 | >260 | 376 | - | 2 |
| 24 | S | 3 | 0 | 0 | - | 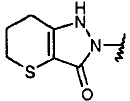 | 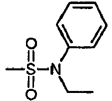 | 198 | 417 | - | 2 |
| 25 | O | 2 | 1 | 0 | - | 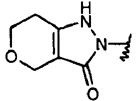 | 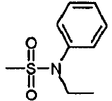 | 164 | 401 | - | 2 |
| 26 | S | 1 | 1 | 0 | - | 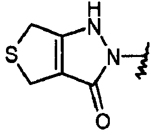 | 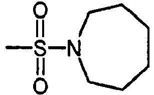 | 236 | 381 | - | 2 |
| 27 | S | 1 | 1 | 0 | - | 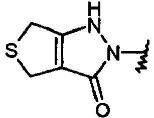 | 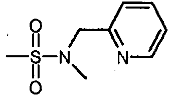 | 198 | 404 | HCl | 2 |
| 28 | 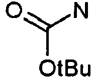 | 2 | 1 | 0 | - | 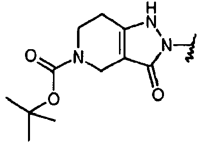 | 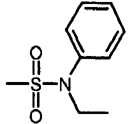 | 192 | 500 | - | 2 |
| 29 | 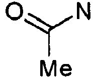 | 2 | 1 | 0 | - | 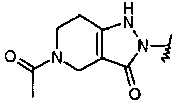 | 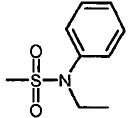 | 118 | 442 | - | 2 |
| 30 | S | 1 | 1 | 0 | - | 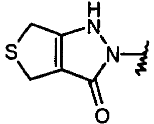 | -CO₂-tBu | >250 | 320 | - | 2 |
| 31 | S | 1 | 1 | 0 | - | 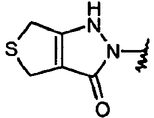 | Me | >220 | 234 | Na | 2 |
| 32 | S | 1 | 1 | 0 | - | 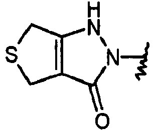 | 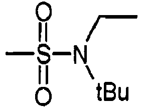 | >260 | 383 | Na | 2 |
| 33 | S | 1 | 1 | 0 | - | 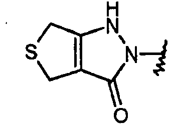 | 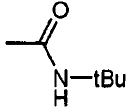 | >260 | 318 | Na | 2 |
| 34 | S | 1 | 1 | 0 | - | 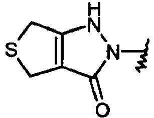 | -OMe | >220 | 250 | Na | 2 |
| 35 | 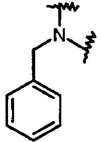 | 3 | 0 | 1 | Oxo | 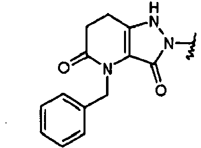 | 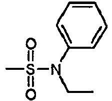 | >250 | 504 | Na | 2 |
| 36 | S | 1 | 1 | 0 | - | 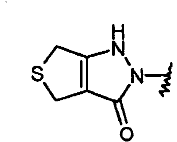 | 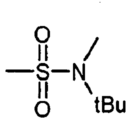 | 130 | 369 | Na | 2 |
| 37 | -CH₂- | 4 | 1 | 0 | - | 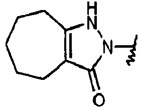 | 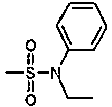 | 200 | 427 | - | 1 |
| 38 | S | 1 | 1 | 0 | - | 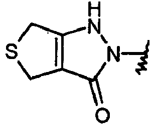 | 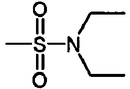 | 192 | 355 | - | 2 |
| 40 | S | 1 | 1 | 0 | - | 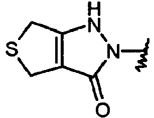 | 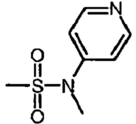 | 182 | 390 | HCl | 2 |
| 41 | S | 1 | 1 | 0 | - | 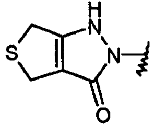 | 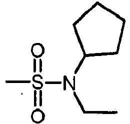 | >260 | 395 | Na | 2 |
| 42 | S | 1 | 1 | 0 | - | 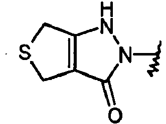 | 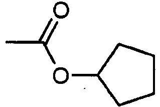 | 244 | 332 | - | 2 |
| 43 | S | 1 | 1 | 0 | - | 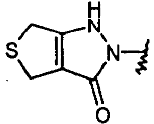 | 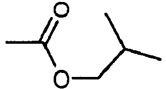 | 200 | 320 | - | 2 |
| 44 | S | 1 | 1 | 0 | - | 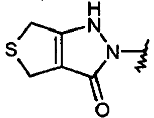 | 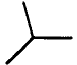 | >260 | 262 | Na | 2 |
| 45 | S | 1 | 1 | 1 | -COOMe | 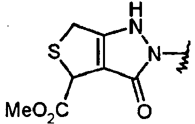 | 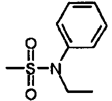 | 158 | 461 | - | 2 |
| 46 | S | 1 | 1 | 0 | - | 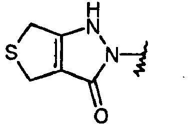 | 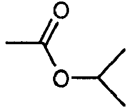 | 220 | 306 | - | 2 |
| 47 | -CH(OMe)- | 0 | 2 | 0 | - | 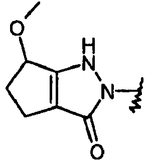 | 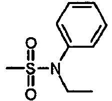 | 220 | 414 | Na | 2 |
| 48 | CH₂ | 1 | 1 | 0 | - | 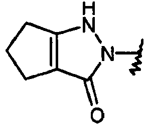 | 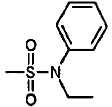 | 204 | 385 | - | 2 |
| 49 | S | 1 | 1 | 0 | - | 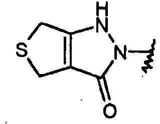 | 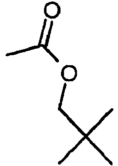 | 204 | 334 | - | 2 |
| 50A | S | 1 | 1 | 0 | | 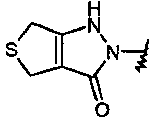 | 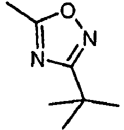 | 240 | 344 | | 2 |
| 50B | S | 1 | 1 | 0 | - | 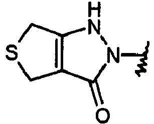 | 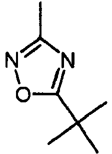 | 240 | 344 | - | 2 |
| 51 | S | 1 | 1 | 0 | - | 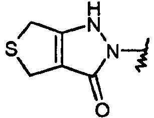 | 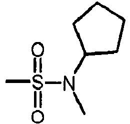 | 208 | 381 | - | 2 |
| 52 | -CH₂. | 1 | 1 | 0 | - | 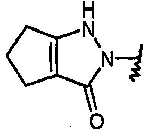 | 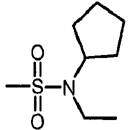 | 220 | 377 | - | 2 |
| 53 | S | 1 | 1 | 0 | - | 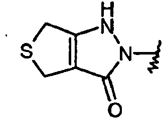 | 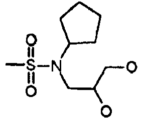 | 190 | 441 | - | 2 |
| 54 | 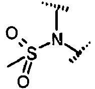 | 2 | 1 | 0 | - | 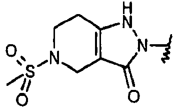 | 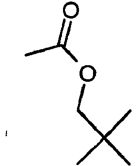 | 230 | 409 | - | 2 |
| 55 | -CH₂- | 2 | 1 | 0 | - | 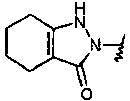 | 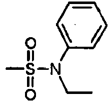 | 200 | 399 | - | 1 |
| 56 | 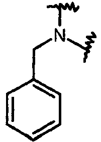 | 2 | 1 | 0 | - | 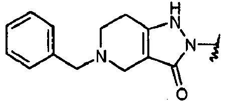 | 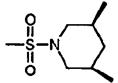 | 220 | 482 | - | 1 |
| 57 | -CH(Me)- | 1 | 2 | 0 | - | 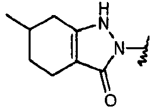 | 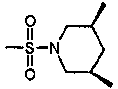 | 215 | 405 | - | 1 |
| 58 | 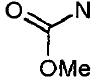 | 2 | 1 | 0 | - | 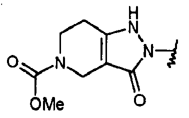 | 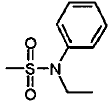 | 86 | 458 | - | 2 |

**TABLEAU II**

| | | | | | | | | | | | | |
|---|---|---|---|---|---|---|---|---|---|---|---|---|
| 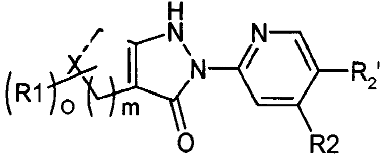 | | | | | | | | | | | | |

| N° | X | n | m | o | R1 | 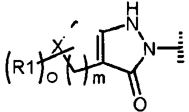 | | | | | | |
|---|---|---|---|---|---|---|---|---|---|---|---|---|
| 59 | S | 1 | 1 | 0 | - | 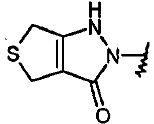 | -OMe | - | 254 | 250 | - | 2 |
| 60 | S | 1 | 1 | 0 | - | 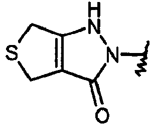 | -NMe₂ | - | 182 | 263 | HCl | 2 |
| 61 | S | 1 | 1 | 0 | - | 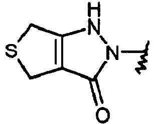 | Et- | - | >260 | 247 | Na | 2 |
| 62 | S | 1 | 1 | 0 | - | 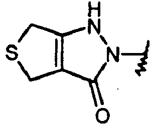 | Me | - | >250 | 234 | Na | 2 |
| 63 | S | 1 | 1 | 0 | - | 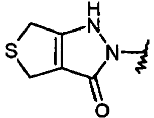 | -OCH₂Ph | - | >250 | 326 | Na | 2 |
| 64 | S | 1 | 1 | 0 | - | 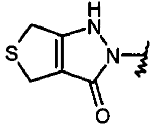 | 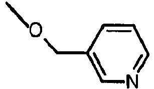 | - | >260 | 327 | Na | 2 |
| 65 | S | 1 | 1 | 0 | - | 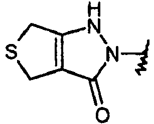 | 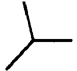 | - | 92 | 262 | - | 2 |

Les composés selon l'invention ont fait l'objet d'essais pharmacologiques en vue de déterminer leurs propriétés, avec en particulier :
- Un test *in vitro* de mesure directe de stabilisation de la protéine HIF1-alpha, facteur de transcription exprimé de façon constitutive dans les cellules mais dégradé dans les conditions normales en oxygène par le système ubiquitin/protéasome.
- Un test fonctionnel permettant la mesure dans des cellules He3pB de la sécrétion de VEGF et d'EPO qui sont deux marqueurs de l'activation de HIF1-alpha dans les hépatocytes.

Ces deux tests sont décrits ci-dessous.

### 1. Mesure de la stabilisation de HIF1alpha dans les cellules HEKEA

### 1.1 Objectif

HIF est un facteur de transcription impliqué dans l'adaptation des cellules à l'hypoxie. Ce facteur de transcription est a minima un hétéro-dimère constitué de 2 protéines, ARNT et HIF1alpha. ARNT est exprimé de façon constitutive par les cellules et l'essentiel de la régulation du complexe s'effectue via la stabilisation de la protéine HIF1-alpha. En effet cette protéine, dans les conditions normales en oxygène (20% équivalents approximativement à la valeur de l'oxygène ambiant), est hydroxylée spécifiquement sur 2 prolines (proline 402 et 564 pour la protéine humaine) par des HIF prolyl-hydroxylases entrainant la liaison de la protéine de von Hippel Lindau (VHL). Cette liaison de VHL sur HIF1-alpha provoque alors la dégradation de HIF1alpha par le système ubiquitine/protéasome. En hypoxie (O₂ < 5% dans les tests cellulaires) les HIF prolyl-hydroxylases sont inhibées ce qui se traduit par une augmentation de la quantité de la protéine HIF1alpha dans les cellules. Ce dernier peut alors s'associer à ARNT pour basculer dans le noyau et activer ses gènes cibles.

Les gènes activés par HIF étant impliqués dans la réponse adaptative des cellules à l'hypoxie et des tissus à l'ischémie, l'objectif est d'identifier et de caractériser des composés qui stabilisent HIF1alpha dans les cellules afin d'amplifier ou de mimer son activité bénéfique.

Il existe de nombreux tests décrivant la mesure indirecte de l'activité de HIF via des systèmes de gènes rapporteur (HRE_luciférase) ou via la mesure de protéines induites par HIF (exemple VEGF ou EPO). De plus les seuls tests permettant de mesurer directement la quantité de la protéine HIF1alpha dans les cellules sont des tests utilisant des anticorps comme le Western Blot comprenant des phases d'extraction des cellules (lysats totaux ou extraits nucléaires) qui sont consommatrices en termes de cellules, de temps en limitant ainsi la capacité de screening de composés. L'objectif était donc de développer un test de screening sensible adaptable en plaques 384 puits permettant de mesurer directement la quantité de la protéine HIF1alpha dans le noyau des cellules. Ce test a été établi dans des cellules HEK (Cellules humaines épithéliales issues d'adénocarcinome rénal).

### 1.2 Principe du Test

Le test est un test cellulaire basé sur le principe de complémentation d'enzyme, l'enzyme utilisée ici étant la beta galactosidase. Les cellules HEKEA sont des cellules HEK exprimant de façon stable et restreinte dans leur noyau, la beta galactosidase mutante (fragment omega aussi appelé EA) (lignée vendue par DiscoverX). Cette construction permet d'avoir une activité beta galactosidase uniquement quand la protéine comportant le fragment de complémentation Prolabel a migré dans le noyau.

La protéine d'intérêt comportant le fragment Prolabel est ici un HIF1alpha ou HIF1alpha muté sur les 2 Prolines 402 et 564 remplacées par des Alanines, est fusionné coté C_terminal par biologie moléculaire (vecteur DiscoverX vendu par Clontech) avec le petit fragment peptidique de complémentation (Prolabel ou ED, 4 kDa environ). Le vecteur codant ensuite pour la protéine chimère HIF1alpha_Prolabel est ensuite transfecté dans des cellules HEKEA pour l'obtention de clones stables (HEKEA_HIF1alphaPLBL)

La quantité de protéine HIF1alpha « taggué »Prolabel en position C-terminale obtenue après traitement des cellules à l'hypoxie ou des composés potentiellement activateurs de HIF est mesurée par ajout aux cellules d'un tampon de lyse contenant un substrat chemiluminescent de la beta galactosidase.

La mesure de l'activité beta galactosidase sera proportionnelle à la quantité de Prolabel donc de HIF1alpha ayant migré dans le noyau des cellules.

Des expériences ont été réalisées en interne en parallèle pour valider que le fragment Prolabel seul n'était pas stable dans les cellules et ne permettait donc pas la mesure d'une activité.

### 1.3 Protocole

### 1.3.1 Plan de l'expérience

1) Ensemencement des cellules à J 0
2) Adhérence 24 heures en normoxie
3) Préparation et ajout des produits (Biomek 2000 et FX) J+1
4) Incubation en Normoxie pendant 6h
5) Lecture des plaques (en luminescence)

### 1.3.2 Ensemencement des cellules

Les cellules sont ensemencées avec le Multidrop en plaques 384 puits blanches à fond opaque (Greiner ref 3704), dans 30µl de milieu de culture (1% SVF) à 10.000 cellules/puits (plaque cellules).

### 1.3.3 Traitement

### • Préparation de la plaque de dilution (plaque DL)

Les produits à tester sont préparés à 3 x 10⁻² M dans 100% DMSO puis dilués à 3x10⁻⁴M dans le milieu à 0.1% SVF (10µl dans 990µl MEM). Ils sont ensuite déposés à la main dans la colonne 12 d'une plaque 96 puits à fond rond (200 µl de chaque composé) appelée plaque de dilution (dl). La plaque dl complète de 3x 10⁻⁴M à 10⁻⁹M est alors réalisée par le Biomek 2000 (programme : Gamme 10 points en série). Pour les références et contrôles, 100µl de DMEM 0.1% SVF sont ajoutés en colonne 1, 100µl de Deferoxamine 10⁻³M en colonne 2, puits A B C D et 100 µl de Deferoxamine 5x10⁻³ M colonne 2, puits E F G H.

### • Distribution plaque DL dans plaques cellules

3.3 µL sont prélevés de la plaque DL par pipetage avec le Biomek FX 96 pour être déposés en duplicate horizontaux (colonne 1 à 24).dans chaque plaque cellules 384 puits (plaque cellules HEKEA_HIF1alphaPLBL)

Les cellules sont alors déposées pendant 6 h dans un incubateur à 37°C (O₂ ambiant, 6% CO₂).

### 1.3.4 Mesure de l'activité beta galactosidase.

### Le Kit utilisé est le Kit chemiluminescent PROLABEL(Ref 93-0001 DiscoverX)

Après les 6 h d'incubation à 37°C les cellules sont lysées avec ajout de15 µl de tampon de lyse contenant le substrat de la beta galactosidase (19 volumes de Path hunter cell assay buffer + 5 volumes de Emarald II solution +1 volume de Galacton star) directement rajouté au 30 µl de milieu dans la plaque.Les plaques sont incubées 60 minutes à l'abri de la lumière avant lecture de la luminescence au Top Count. Les EC50 des composés sont ensuite calculés avec un logiciel de fitting approprié et reportés dans le **tableau III** ci-dessous.

L'activité activatrice d'un composé vis à vis de HIF est donnée par la concentration molaire qui produit 50% de la réponse maximale de ce même composé.

**TABLEAU III**

| **N° composé** | **EC50 (M)** |
|---|---|
| **1** | 8.8E-06 |
| **7** | 6,0E-06 |
| **9** | 1,4E-06 |
| **13** | 5,2E-06 |
| **15** | 1,6E-05 |
| **16** | 8,6E-06 |
| **17** | 1,0E-05 |
| **18** | 4,4E-06 |
| **19** | 5,5E-06 |
| **20** | 3,4E-06 |
| **21** | 8,7E-06 |
| **23** | 1,2E-05 |
| **24** | 3.E-06 |
| **25** | 3,3E-06 |
| **26** | 1,5E-06 |
| **27** | 9,5E-06 |
| **28** | 1,6E-06 |
| **29** | 2,5E-06 |
| **30** | 6,5E-07 |
| **32** | 1,3E-06 |
| **33** | 9,4E-06 |
| **34** | 1,0E-05 |
| **35** | 2,5E-06 |
| **36** | 1,2E-06 |
| **37** | 2,2E-06 |
| **38** | 3,8E-06 |
| **60** | 2,1E-05 |
| **61** | 1,9E-06 |
| **62** | 3,9E-06 |

### 1.4 Divers

### 1.4.1. Entretien des cellules HEKEA HIF1alpha PLBL.

Les cellules sont cultivées en milieu complet (cf ci dessous) en Flask T225. à 37°C dans un incubateur à CO₂

### 1.4.2. Milieu de culture des cellules HEKEA HIF1alpha PLBL

| | |
|---|---|
| DMEM | 500 mL |
| +SVF10%(GIBCO 10500-056) | 50 mL |
| +Glutamine (2mM final) | 5 mL |
| +Penicillline + streptomycine (200mg/mL) | 5 mL |
| +Hygromycine B (100µg/mL) | 1.1 mL |
| +Geneticine (400µg/mL final) | 4.4 mL |

### 2. Mesure de la sécrétion de VEGF et d'EPO par les hépatocytes Hep3B

### 2.1. Obiectif

HIF est un facteur de transcription impliqué dans l'adaptation des cellules à l'hypoxie Les gènes activés par HIF étant impliqués dans la réponse adaptative des cellules à l'hypoxie et des tissus à l'ischémie, l'objectif est d'identifier et de caractériser des composés qui stabilisent HIF1alpha dans les cellules afin d'amplifier ou de mimer son activité bénéfique. HIF1alpha a été identifié suite à l'analyse du promoteur du gène de l'EPO, ce qui fait de cette protéine un des marqueurs princeps de l'activation de HIF1alpha. D'autre part, le VEGF est également identifié dans la littérature comme un des principaux marqueurs de l'activation de HIF. C'est la raison pour laquelle la mesure de ces deux protéines a été retenue pour caractériser les composés activateurs de HIF dans les Hep3B.

L'objectif était donc de développer un test de screening sensible adaptable en plaques 96 puits permettant de mesurer directement la quantité de VEGF et d'EPO dans le surnageant des Hep3B. (Cellules issues d'hepato-carcinome humain) en réponse aux potentiels activateurs de HIF.

### 2.2. Principe du Test

Le test est un test ELISA permettant la mesure du VEGF et de l'EPO dans le surnageant des cellules Hep3B traitées par l'hypoxie ou la deferoxamine en contrôles ou les potentiels activateurs de HIF. Le test a été adapté en 96 puits permettant une plus grande capacité de screening des composés.

### 2.3. Protocole

### 2.3.1 Plan de l'expérience

1) Ensemencement des cellules à J 0
2) Adhérence 6heures en normoxie
3) Préparation et ajout des produits (Biomek 2000 et FX)
4) Incubation en normoxie pendant 18h
5) Dosage EPO et VEGF dans le surnageant à J+1

### 2.3.2 Ensemencement des cellules

Les cellules sont repiquées dans 100 µl de milieu de culture (10% SVF) en plaques 96 puits noires à fond opaque (référence Costar 3916) à 30.000 cellules/puits, avec le multidrop.

### 2.3.3 Traitement des cellules

### • Préparation de la plaque de dilution (plaque DL)

Les produits à tester sont préparés à 10⁻² M dans 100% DMSO puis dilués à 310 ⁴M dans le milieu à 0.1% SVF (6 µl dans 194 µl MEM) Déposer 200 µl de chaque composé dans la colonne 12 d'une plaque dilution 96 puits Les gammes de dilution de 3x10⁻⁴M à 3x10⁻⁸M sont réalisées par le Biomek 2000 (programme : Gamme 9 points en série).100µl de MEM 0.1% SVF et de Deferoxamine 5x10⁻³M sont ajoutés comme contrôles en colonne 3 respectivement puits A,B,C,D et puits E,F,G,H

### • Distribution plaque DL dans plaques cellules

Le milieu des cellules ensemencées la veille en plaques 96 puits est changé pour 90 µl de milieu 0.1% SVF et 10 µl sont distribués avec le FX 96 à partir des plaques DL 96 vers les plaques cellules

Les plaques cellules ainsi traitées sont déposées pendant 18 h dans un incubateur à 37°C (O₂ ambiant, 6% CO₂).

### 2.3.4 Dosage EPO et VEGF

Les sumageants (80 µl) des Hep3B en plaques 96 puits traités avec les potentiels activateurs de HIF sont récupérés à la pipette multicanaux pour dosage simultané du VEGF et de l'EPO en ELISA selon les instructions du fournisseur (Kit EPO Mesoscale (ref K15122B-2)). Les EC50 pour EPO et VEGF des composés sont ensuite calculés avec un logiciel de fitting approprié et reportés dans le **tableau IV** ci-dessous.

### 2.4. Divers

### Milieu de culture des cellules Hep3B:

| | |
|---|---|
| MEM + Earles (GIBCO 310095) | 500mL |
| + 10% SVF (GIBCO 10500-056) | 50mL |
| + Glutamine 2mM final | 5mL |
| + Acides aminés non essentiels | 1% 5mL |

### 3. Résultats

L'activité activatrice d'un composé vis à vis de HIF est donnée par la concentration qui produit 50% de la réponse maximale de ce même compose dans **le tableau IV** ci-dessous.

**TABLEAU IV**

| **N° composé** | **EC50 EPO (M)** | **EC50 VEGF (M)** |
|---|---|---|
| **1** | 2.9 E-06 | 3E-06 |
| **9** | 7,0E-07 | 6,6E-07 |
| **13** | 9,0E-07 | 1,0E-06 |
| **15** | 2,8E-06 | 3,0E-06 |
| **16** | 3,5E-06 | 3,0E-06 |
| **17** | 2,9E-06 | 2,9E-06 |
| **18** | 5,0E-07 | 4,0E-07 |
| **19** | 2,4E-06 | 2,4E-06 |
| **20** | 1,0E-06 | 9,0E-07 |
| **21** | 1,4E-06 | 2,4E-06 |
| **24** | 5E-07 | 5E-07 |
| **25** | 2.4 E-06 | 2.5 E-06 |
| **26** | 1 E-06 | 1.3 E-06 |
| **28** | 1 E-06 | 1 E-06 |
| **29** | 2.5 E-06 | 2.6 E-06 |
| **30** | 1.5 E-06 | 1.8 E-06 |
| **32** | 2.6 E-06 | 3.2 E-06 |
| **35** | 1.7 E-06 | 1.1 E-06 |
| **36** | 1.6 E-06 | 2 E-06 |
| **59** | 2.6 E-06 | 3.1 E-06 |
| **61** | 2.2 E-06 | 2.8 E-06 |

Les composés selon l'invention peuvent donc être utilisés pour la préparation de médicaments, en particulier de médicaments activateurs du facteur de transcription HIF. Ainsi, selon un autre de ses aspects, l'invention a pour objet des médicaments qui comprennent un composé de formule (I), ou un sel d'addition de ce dernier à un acide pharmaceutiquement acceptable du composé de formule (I).

L'invention concerne également une composition pharmaceutique comprenant un composé de formule (I) selon la présente invention, ou un sel pharmaceutiquement acceptable de ce composé, ainsi qu'au moins un excipient pharmaceutiquement acceptable.

Ces médicaments trouvent leur emploi en thérapeutique, notamment dans le traitement/la prophylaxie en particulier de maladies cardiovasculaires, l'ischémie des membres inférieurs, l'insuffisance cardiaque, les maladies coronariennes d'origine ischémique comme l'angine de poitrine ou l'infarctus du myocarde, l'arterosclérose, les accidents vasculaires cérébraux d'origine ischémique, l'hypertension pulmonaire et toutes les pathologies provoquée par une occlusion vasculaire partielle ou totale chez l'homme et l'animal.

Ces médicaments trouvent également leur emploi en thérapeutique dans le traitement/la prophylaxie des glaucomes, des maladies rénales ou dans les maladies cérébrales d'origine neurodégénératives ou pas, des anémies, ou d'un médicament destiné à favoriser la cicatrisation ou agents ou à raccourcir la période de reconvalescence post-opératoire ou d'un médicament destiné au traitement des états de fatigue générale ou encore d'un médicament utilisé dans le but d'obtenir du sang dans le cadre d'autotransfusions nécessaires à la suite d'interventions chirurgicales lourdes telles que la chirurgie cranienne ou thoracique ou comme les opérations cardiaques ou au niveau carotidien ou aortique.

Ces composés trouvent leur emploi en thérapeutique, notamment dans le traitement/la prophylaxie des anémies.

Ces composés sont également utilisables chez l'homme et l'animal dans le but d'obtenir du sang dans le cadre d'autotransfusions nécessaires à la suite d'interventions chirurgicales lourdes telles que la chirurgie cranienne ou thoracique ou comme les opérations cardiaques ou au niveau carotidien ou aortique

Ces composés sont potentiellement utilisables chez l'homme et l'animal en tant qu'agents favorisant la cicatrisation ou agents permettant de racourcir la période de reconvalescence post opératoire.

Ces composés sont potentiellement utilisables chez l'homme et l'animal dans le traitement des états de fatigue générale allant jusqu'à la cachexie apparaissant en particulier chez les sujets agés.

Ces composés sont potentiellement utilisables chez l'homme et l'animal dans le traitement des glaucones, des maladies rénales ou dans les maladies cérébrales d'origine neurodégénératives ou pas.

Enfin les composés décrits dans l'invention sont potentiellement utilisables chez l'homme et l'animal pour le traitement de maladies cardiaques ou périphériques d'origine ischémique via une médecine régénérative dans des approches autologues et hétérologues utilisant des cellules souches non embryonnaires ou de cellules myoblastiques à des fins thérapeutiques que ce soit en traitement de ces cellules avant administration ou en traitement simultané à l'administration locale de ces cellules.

D'autre part, les composés décrits dans l'invention peuvent être utilisés seuls ou si nécessaire en combinaison avec un ou des autres composé(s) actif(s) utiles dans le traitement de l'hypertension, de l'insuffisance cardiaque, du diabète et de l'anémie.
Par exemple, on peut citer l'association d'un composé selon l'invention avec un ou plusieurs composés choisis parmi des inhibiteurs de l'enzyme de conversion, des antagonistes du récepteur de l'angiotensine II, des beta blockants, des antagonistes du récepteur aux mineralocorticoides, des diurétiques, des antagonistes calciques, des statines et des dérivés de la digitaline.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable dudit composé, ainsi qu'au moins un excipient pharmaceutiquement acceptable.
Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables.

## Revendications

1. Composé de formule (I): 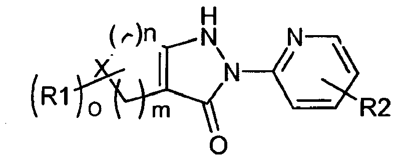 dans laquelle
**n** est égal à 0, 1, 2, 3 ou 4 ;
**m** est égal à 0, 1 ou 2 ;
**o** est égal à 0 ou 1 ;
**X** représente un groupe -CH₂, -CH(R')-, -N(R')- ou un hétéroatome choisi parmi un atome d'oxygène et un atome de soufre, étant entendu que R' représente un groupe - (C1-C5)alkyle, -(C1-C5)alcoxy, -CH₂-aryle, -C(O)R5 ou -COOR5 avec R5 tel que défini ci-dessous ;
**R1** représente un groupe oxo, un groupe -COOR5, un groupe -W-OH ou un groupe - W-NR5R6, avec W, R5 et R6 tels que définis ci-dessous ; et
**R2** représente un atome d'hydrogène ou un groupe choisi parmi (i) les groupes -(C1-C5)alkyle, (ii) les groupes -(C1-C5)alcoxy, (iii) les groupes -COOR5, (iv) les groupes - NR5R6, (v) les groupes -C(O)-NR5R6, (vi) les groupes -SO₂-NR3R4, (vii) les groupes hétéroaryle eventuellement substitués par un groupe -(C1-C5)alkyle, (viii) les groupes - W-aryle, (ix) les groupes -W-hétéroaryle, (x) les groupes -O-W-aryle, (xi) les groupes -O-W-hétéroaryle et (xii) les groupes -O-W-NR5R6, avec W, R3, R4, R5 et R6 tels que définis ci-dessous ;
Etant entendu que :
**R3** et **R4**,
(i) qui peuvent être identiques ou différents, représentent, indépendamment l'un de l'autre, un atome d'hydrogène, un groupe -(C1-C5)alkyle, un groupe -(C3-C6)cycloalkyle, un groupe aryle, un groupe hétéroaryle, un groupe -CH₂-hétéroaryle, un groupe -(C1-C5)alkyl-NR5R6, un groupe -W-OH ou un groupe-W-NR5R6 ; ou
(ii) forment ensemble avec l'atome d'azote qui les porte un groupe hétérocycloalkyle éventuellement substitué par un ou plusieurs groupes choisis parmi les groupes -(C1-C5)alkyle et les groupes -CH₂-aryle ;
**W** est un groupe -(C1-C5)alkylène, éventuellement substitué par un ou plusieurs groupes hydroxy; et
**R5** et **R6**, qui peuvent être identiques ou différents, représentent, indépendamment l'un de l'autre, un atome d'hydrogène ou un groupe choisi parmi les groupes -(C1-C5)alkyle et les groupes -(C3-C6)cycloalkyle ;
à l'état de base ou de sel d'addition à un acide.

2. Composé de formule (I) selon la revendication 1, **caractérisé en ce que**
• **n** est égal à 0, 1, 2, 3 ou 4 ;
• **m** est égal à 0, 1 ou 2 ;
• **o** est égal à 0 ou 1 ;
• **X** représente un groupe -CH₂-, -CH(R')-, -N(R')- ou un hétéroatome choisi parmi l'atome d'oxygène et l'atome de soufre ;
• **R'** représente un groupe -(C1-C5)alkyle, un groupe -(C1-C5)alcoxy, un groupe -CH₂-aryle, un groupe -C(O)R5 ou un groupe -COOR5 ;
• **R1** représente un groupe oxo, un groupe -COOR5, un groupe -W-OH ou un groupe - W-NR5R6 ;
• **R2** représente un atome d'hydrogène, un groupe -(C1-C5)alkyle, un groupe -(C1-C5)alcoxy, un groupe -COOR5, un groupe -NR5R6, un groupe -C(O)-NR5R6 ou un groupe -SO₂-NR3R4;
• **R3** et **R4**
(i) représentent, indépendamment l'un de l'autre, un atome d'hydrogène, un groupe -(C1-C5)alkyle, un groupe -(C3-C6)cycloalkyle, un groupe aryle, un groupe hétéroaryle, un groupe -CH₂-hétéroaryle ou un groupe -(C1-C5)alkyl-NR5R6 ; ou
(ii) forment ensemble avec l'atome d'azote qui les porte un groupe hétérocycloalkyle éventuellement substitué par un groupe -(C1-C5)alkyle ou par un groupe aryle ;
• **W** représente un groupe -(C1-C5)alkylène, éventuellement substitué par un ou plusieurs groupes hydroxy; et/ou
• **R5** et **R6** représentent indépendamment l'un de l'autre un atome d'hydrogène ou un groupe -(C1-C5)alkyle.

3. Composé de formule (I) selon la revendication 1, **caractérisé en ce que**
• **n** est égal à 0, 1, 2, 3 ou 4 ;
• **m** est égal à 0, 1 ou 2 ;
• **o** est égal à 0 ;
• **R1** représente un groupe oxo, un groupe -CH₂-aryle, un groupe -C(O)R5- ou un groupe -COOR5, ledit groupe R1 pouvant être lié à un atome de carbone ou un hétéroatome, avantageusement ledit groupe aryle représentant un groupe phényle ;
• **R3** et **R4**
(i) représentent indépendamment l'un de l'autre, un atome d'hydrogène, un groupe -(C1-C5)alkyle, un groupe -(C3-C6)cycloalkyle, un groupe aryle, un groupe hétéroaryle, un groupe -CH₂-hétéroaryle ou un groupe -(C1-C5)alkyl-NR5R6, avantageusement ledit groupe aryle représentant un groupe phényle et ledit groupe hétéroaryle représentant un groupe pyridinyle ou un groupe furanyle ; ou
(ii) forment ensemble avec l'atome d'azote qui les porte un groupe hétérocycloalkyle éventuellement substitué par un ou plusieurs groupe(s) -(C1-C5)alkyle et/ou aryle, avantageusement ledit groupe hétérocycloalkyle représentant un groupe pipéridinyle, un groupe pyrrolidinyle ou un groupe hexaméthylèneimino et ledit groupe aryle représentant un groupe phényle ;
• **R5** représente un groupe -(C1-C5)alkyle ou un groupe -(C1-C5)cycloalkyle ; et/ou
• **R6** représente un atome d'hydrogène ou un groupe (C1-C5)alkyle.

4. Composé de formule (I) selon la revendication 1, **caractérisé en ce que**
• **n** est égal à 1, 2, 3 ou 4 ;
• **m** est égal à 0, 1 ou 2 ;
• **o** est égal à 0 ou 1 ;
• **X** représente un groupe -CH₂-, un groupe -CH(R')-, un groupe -N(R')- ou un hétéroatome choisi parmi l'atome d'oxygène et l'atome de soufre ;
• **R'** représente un groupe -(C1-C5)alkyle, un groupe -(C1-C5)alcoxy, un groupe - CH₂-aryle, un groupe -C(O)R5 ou un groupe -COOR5 ;
• **R1** représente un groupe oxo, un groupe COOR5, un groupe -W-OH ou un groupe -W-NR5R6 ;
• **R2** représente un groupe -SO₂-NR3R4;
• **R3** et **R4**
(i) représentent, indépendamment l'un de l'autre, un atome d'hydrogène, un groupe -(C1-C5)alkyle, un groupe -(C3-C6)cycloalkyle, un groupe aryle, un groupe hétéroaryle ou un groupe -CH₂-hétéroaryle, ou
(ii) forment ensemble avec l'atome d'azote qui les porte un groupe hétérocycloalkyle ; et
• **R5** et **R6** représente un groupe -(C1-C5)alkyle.

5. Composé de formule (I) selon la revendication 1, **caractérisé en ce que**
• **n** est égal à 1, 2, 3 ou 4 ;
• **m** est égal à 0, 1 ou 2 ;
• **o** est égal à 0 ou 1 ;
• **X** représente un groupe -CH₂-, un groupe -CH(R')-, un groupe -N(R')- ou un hétéroatome choisi parmi l'atome d'oxygène et l'atome de soufre ;
• **R'** représente un groupe -(C1-C5)alkyle, un groupe -(C1-C5)alcoxy, un groupe-CH₂-aryle, un groupe -C(O)R5 ou un groupe -COOR5 ;
• **R1** représente un groupe oxo ;
• **R2** représente un atome d'hydrogène, un groupe -(C1-C5)alkyle, un groupe -(C1-C5)alcoxy, un groupe -COOR5, un groupe -NR5R6 ou -C(O)-NR5R6 ; et
• **R5** et **R6** représentent, indépendamment l'un de l'autre, un atome d'hydrogène, un groupe -(C1-C5)alkyle ou un groupe -(C1-C5)cycloalkyle.

6. Composé de formule (I) selon la revendication 1, **caractérisé en ce que R2** représente (i) un atome d'hydrogène, (ii) un groupe- (C1-C5)alkyle, (iii) un groupe -(C1-C5)alcoxy, (iv) un groupe -COOR5, (v) un groupe -NR5R6, (vi) un groupe -C(O)-NR5R6, (vii) un hétéroaryle substitué par un groupe -(C1-C5) alkyle, (viii) un groupe -O-W-aryle ou (ix) un groupe -O-W-heteroaryle.

7. Composé de formule (I) selon la revendication 1, **caractérisé en ce que** R2 représente un groupe -SO₂-NR3R4.

8. Composé de formule (I) selon l'une quelconque des revendications 1 à 7, **caractérisé en ce que R2** est un substituant de l'atome situé en position beta de la pyridine.

9. Composé de formule (I) selon l'une quelconque des revendications 1 à 7, **caractérisé en ce que R2** est un substituant de l'atome situé en position gamma de la pyridine.

10. Composé de formule (I) selon l'une quelconque des revendications précédentes, **caractérisé en ce qu'**il s'agit du :
• 2-[5-(pipéridin-1-ylsulfonyl)pyridin-2-yl]-1,2,4,5,6,7-hexahydro-3H-indazol-3-one ;
• 6-méthyl-2-[5-(piperidin-1-ylsulfonyl)pyridin-2-yl]-1,2,4,5,6,7-hexahydro-3H-indazol-3-one ;
• 2-[5-(pipéridin-1-ylsulfonyl)pyridin-2-yl]-1,4,5,6,7,8-hexahydrocyclohepta[c]pyrazol-3(2H)-one ;
• N-éthyl-6-(3-oxo-1,3,4,5,6,7-hexahydro-2H-indazol-2-yl)-N-phénylpyridine-3-sulfonamide;
• 6-(5-benzyl-3-oxo-1,3,4,5,6,7-hexahydro-2H-pyrazolo[4,3-c]pyridin-2-yl)-N-éthyl-N-phénylpyridine-3-sulfonamide;
• (+/-)2-(5-{[(3R,5S)-3,5-diméthylpipéridin-1-yl]sulfonyl}pyridin-2-yl)-1,2,4,5,6,7-hexahydro-3H-indazol-3-one;
• 2-(4-méthoxypyridin-2-yl)-1,2,4,6-tetrahydro-3H-thieno[3,4-c]pyrazol-3-one;
• 2-(pyridin-2-yl)-1,4,5,6-tétrahydrocyclopenta[c]pyrazol-3(2H)-one;
• (+/-)5-benzyl-2-(5-{[(3R,5S)-3,5-diméthylpiperidin-1-yl]sulfonyl}pyridin-2-yl)-1,2,4,5,6,7-hexahydro-3H-pyrazolo[4,3-c]pyridin-3-one;
• (+/-)2-(5-{[(3R,5S)-3,5-dimethylpiperidin-1-yl]sulfonyl}pyridin-2-yl)-6-méthyl-1,2,4,5,6,7-hexahydro-3H-indazol-3-one;
• 6-(5-benzyl-3-oxo-1,3,4,5,6,7-hexahydro-2H-pyrazolo[4,3-c]pyridin-2-yl)-N,N-diéthylpyridine-3-sulfonamide;
• N-éthyl-6-(3-oxo-1,3,4,5,6,7,8,9-octahydro-2H-cycloocta[c]pyrazol-2-yl)-N-phenylpyridine-3-sulfonamide;
• N-éthyl-6-(3-oxo-4,6-dihydro-1H-thieno[3,4-c]pyrazol-2(3H)-yl)-N-phénylpyridine-3-sulfonamide;
• 6-(5-benzyl-3-oxo-1,3,4,5,6,7-hexahydro-2H-pyrazolo[4,3-c]pyridin-2-yl)-N,N-di(propan-2-yl)pyridine-3-sulfonamide;
• 6-méthoxy-2-(pyridin-2-yl)-1,4,5,6-tétrahydrocydopenta[c]pyrazol-3(2H)-one;
• 2-(pyridin-2-yl)-1,4,6,7-tétrahydrothiopyrano[4,3-c]pyrazol-3(2H)-one;
• N-éthyl-6-(3-oxo-1,4,6,7-tétrahydrothiopyrano[4,3-c]pyrazol-2(3H)-yl)-N-phenylpyridine-3-sulfonamide;
• N-éthyl-6-(3-oxo-3,5,6,7-tétrahydrothiopyrano[3,2-c]pyrazol-2(1H)-yl)-N-phénylpyridine-3-sulfonamide;
• N,N-diéthyl-6-(3-oxo-4,6-dihydro-1H-thieno[3,4-c]pyrazol-2(3H)-yl)pyridine-3-sulfonamide;
• N,N-diméthyl-6-(3-oxo-4,6-dihydro-1H-thièno[3,4-c]pyrazol-2(3H)-yl)pyridine-3-sulfonamide;
• 2-[5-(pyrrolidin-1-ylsulfonyl)pyridin-2-yl]-1,2,4,6-tétrahydro-3H-thièno[3,4-c]pyrazol-3-one;
• N-cyclopropyl-6-(3-oxo-4,6-dihydro-1H-thieno[3,4-c]pyrazol-2(3H)-yl)pyridine-3-sulfonamide;
• 6-(3-oxo-4,6-dihydro-1H-thieno[3,4-c]pyrazol-2(3H)-yl)-N-(propan-2-yl)pyridine-3-sulfonamide;
• N-tert-butyl-6-(3-oxo-4,6-dihydro-1H-thieno[3,4-c]pyrazol-2(3H)-yl)pyridine-3-sulfonamide;
• N-(furan-2-ylmethyl)-6-(3-oxo-4,6-dihydro-1H-thieno[3,4-c]pyrazol-2(3H)-yl)pyridine-3-sulfonamide;
• N-cyclopentyl-6-(3-oxo-4,6-dihydro-1H-thieno[3,4-c]pyrazol-2(3H)-yl)pyridine-3-sulfonamide;
• N-methyl-6-(3-oxo-4,6-dihydro-1H-thieno[3,4-c]pyrazol-2(3H)-yl)-N-(pyridin-2-yl)pyridine-3-sulfonamide;
• 2-(pyridin-2-yl)-1,2,4,6-tetrahydro-3H-thieno[3,4-c]pyrazol-3-one ;
• 2-[4-(dimethylamino)pyridin-2-yl]-1,2,4,6-tetrahydro-3H-thieno[3,4-c]pyrazol-3-one ;
• 2-{5-[(4-benzylpiperidin-1-yl)sulfonyl]pyridin-2-yl}-1,2,4,6-tetrahydro-3H-thieno[3,4-c]pyrazol-3-one ;
• 6-(3-oxo-4,6-dihydro-1H-thieno[3,4-c]pyrazol-2(3H)-yl)-N-(pyridin-2-yl)pyridine-3-sulfonamide;
• N-ethyl-6-(3-oxo-1,4,6,7-tetrahydropyrano[4,3-c]pyrazol-2(3H)-yl)-N-phenylpyridine-3-sulfonamide;
• 2-(4-ethylpyridin-2-yl)-2,6-dihydro-4H-thieno[3,4-c]pyrazol-3-olate de sodium;
• 2-[5-(azepan-1-ylsulfonyl)pyridin-2-yl]-1,2,4,6-tetrahydro-3H-thieno[3,4-c]pyrazol-3-one;
• 4-benzyl-2-{5-[ethyl(phenyl)sulfamoyl]pyridin-2-yl}-5-oxo-4,5,6,7-tetrahydro-2H-pyrazolo[4,3-b]pyridin-3-olate de sodium;
• N-methyl-6-(3-oxo-4,6-dihydro-1H-thieno[3,4-c]pyrazol-2(3H)-yl)-N-(pyridin-2-ylmethyl)pyridine-3-sulfonamide;
• 2-{5-[ethyl(phenyl)sulfamoyl]pyridin-2-yl}-3-oxo-1,2,3,4,6,7-hexahydro-5H-pyrazolo[4,3-c]pyridine-5-carboxylate de tert-butyle;
• 6-(5-acetyl-3-oxo-1,3,4,5,6,7-hexahydro-2H-pyrazolo[4,3-c]pyridin-2-yl)-N-ethyl-N-phenylpyridine-3-sulfonamide;
• 6-(3-oxo-4,6-dihydro-1H-thieno[3,4-c]pyrazol-2(3H)-yl)pyridine-3-carboxylate tert-butyle;
• 2-(4-methylpyridin-2-yl)-2,6-dihydro-4H-thieno[3,4-c]pyrazol-3-olate de sodium;
• 2-{5-[tert-butyl(methyl)sulfamoyl]pyridin-2-yl}-2,6-dihydro-4H-thieno[3,4-c]pyrazol-3-olate de sodium;
• 2-{5-[tert-butyl(ethyl)sulfamoyl]pyridin-2-yl}-2,6-dihydro-4H-thieno[3,4-c]pyrazol-3-olate de sodium;
• 2-(5-methylpyridin-2-yl)-2,6-dihydro-4H-thieno[3,4-c]pyrazol-3-olate de sodium;
• 2-[5-(tert-butylcarbamoyl)pyridin-2-yl]-2,6-dihydro-4H-thieno[3,4-c]pyrazol-3-olate de sodium;
• 2-(5-methoxypyridin-2-yl)-2,6-dihydro-4H-thieno[3,4-c]pyrazol-3-olate de sodium;
• N-methyl-6-(3-oxo-4,6-dihydro-1H-thieno[3,4-c]pyrazol-2(3H)-yl)-N-(pyridin-4-yl)pyridine-3-sulfonamide;
• 2-{5-[cyclopentyl(ethyl)sulfamoyl]pyridin-2-yl}-2,6-dihydro-4H-thieno[3,4-c]pyrazol-3-olate de sodium;
• 2-[4-(pyridin-3-ylmethoxy)pyridin-2-yl]-2,6-dihydro-4H-thieno[3,4-c]pyrazol-3-olate de sodium;
• 2-{5-[ethyl(phenyl)sulfamoyl]pyridin-2-yl}-3-oxo-1,2,3,4,6,7-hexahydro-5H-pyrazolo[4,3-c]pyridine-5-carboxylate de méthyle;
• cyclopentyl 6-(3-oxo-4,6-dihydro-1H-thieno[3,4-c]pyrazol-2(3H)-yl)pyridine-3-carboxylate de cyclopentyle;
• 6-(3-oxo-4,6-dihydro-1H-thieno[3,4-c]pyrazol-2(3H)-yl)pyridine-3-carboxylate de2-méthylpropyle;
• 2-[4-(propan-2-yl)pyridin-2-yl]-1,2,4,6-tetrahydro-3H-thieno[3,4-c]pyrazol-3-one;
• 2-[5-(propan-2-yl)pyridin-2-yl]-2,6-dihydro-4H-thieno[3,4-c]pyrazol-3-olate de sodium;
• methyl 2-{5-[ethyl(phenyl)sulfamoyl]pyridin-2-yl}-3-oxo-2,3,4,6-tetrahydro-1H-thieno[3,4-c]pyrazole-4-carboxylate de méthyle;
• 6-(3-oxo-4,6-dihydro-1H-thieno[3,4-c]pyrazol-2(3H)-yl)pyridine-3-carboxylate de propan-2-yle;
• 2-{5-[ethyl(phenyl)sulfamoyl]pyridin-2-yl}-6-methoxy-2,4,5,6-tetrahydrocyclopenta[c]pyrazol-3-olate de sodium;
• N-ethyl-6-(3-oxo-3,4,5,6-tetrahydrocyclopenta[c]pyrazol-2(1H)-yl)-N-phenylpyridine-3-sulfonamide;
• 2-[4-(pyridin-3-ylmethoxy)pyridin-2-yl]-2,6-dihydro-4H-thieno[3,4-c]pyrazol-3-olate de sodium;
• 6-(3-oxo-4,6-dihydro-1H-thieno[3,4-c]pyrazol-2(3H)-yl)pyridine-3-carboxylate 2,2-diméthylpropyle;
• 2-[5-(5-tert-butyl-1,2,4-oxadiazol-3-yl)pyridin-2-yl]-1,2,4,6-tetrahydro-3H-thieno[3,4-c]pyrazol-3-one;
• N-cyclopentyl-N-methyl-6-(3-oxo-4,6-dihydro-1H-thieno[3,4-c]pyrazol-2(3H)-yl)pyridine-3-sulfonamide;
• N-cyclopentyl-N-ethyl-6-(3-oxo-3,4,5,6-tetrahydrocyclopenta[c]pyrazol-2(1H)-yl)pyridine-3-sulfonamide;
• N-cyclopentyl-N-(2,3-dihydroxypropyl)-6-(3-oxo-4,6-dihydro-1H-thieno[3,4-c]pyrazol-2(3H)-yl)pyridine-3-sulfonamide;
• 6-[5-(methylsulfonyl)3-oxo-1,3,4,5,6,7-hexahydro-2H-pyrazolo[4,3-c]pyridin-2-yl]pyridine-3-carboxylate 2,2-dimethylpropyle;
• 2-[5-(3-tert-butyl-1,2,4-oxadiazol-5-yl)pyridin-2-yl]-1,2,4,6-tetrahydro-3H-thieno[3,4-c]pyrazol-3-one.

11. Procédé de préparation d'un composé de formule (I) selon l'une quelconque des revendications 1 à 10, **caractérisé en ce que** l'on fait réagir, avec une base organique, un composé de formule (IV) qui se présente sous la forme d'un composé de formule (IVa) ou (IVb) ou les deux : 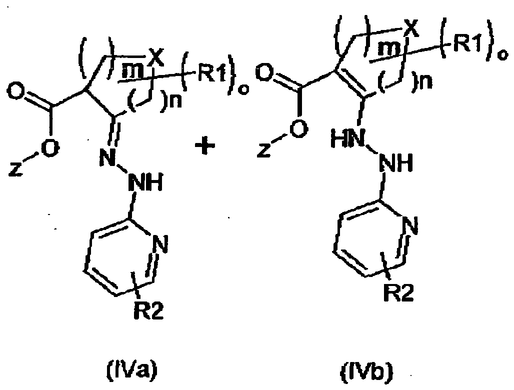 dans laquelle X, R1, R2, n, m, o sont tels que définis dans la revendication 1, et z représente un groupe alkyle.

12. Composés de formules (IVa) et (IVb) 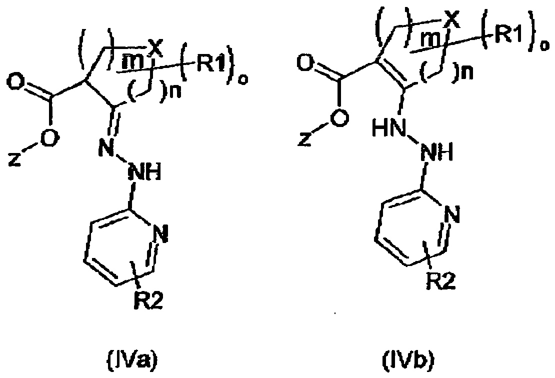 dans lesquelles R1, R2, n, m et o sont tels que définis dans la revendication 1, z représente un groupe alkyk et X représente -CH(R')-, -N(R')- ou un hétéroatome tels que R' et l'hétéroatome sont tels que définis dans la revendication 1.

13. Médicament, **caractérisé en ce qu'**il comprend un composé de formule (I) selon l'une quelconque des revendications 1 à 10, ou un sel d'addition de ce composé à un acide pharmacutiquement acceptable du composé de formule (I).

14. Composition pharmaceutique, **caractérisée en ce qu'**elle comprend un composé de formula (I) selon l'une quelconque des revendications 1 à 10, ou un sel pharmaceutiquement acceptable de ce composé, ainsi qu'au moins un excipient pharmaceutiquement acceptable.

15. Utilisation d'un composé de formule (I) selon l'une quelconque des revendications 1 à 10 pour la préparation d'un médicament destiné au traitement/la prophylaxie de maladies cardiovasculaires.

16. Utilisation d'un composé de formule (I) selon l'une quelconque des revendications 1 à 10 pour la préparation d'un médicament destiné au traitement/la prophylaxie de l'ischémie des membres inférieurs, l'insuffisance cardiaque, les maladies coronariennes d'origine ischémique comme l'angine de poitrine ou l'infarctus du myocarde, l'arterosctérose, les accidents vasculaires cérébraux d'origine ischémique, l'hypertension pulmonaire et toutes les pathologies provoquées par une occlusion vasculaire partielle ou totale.

17. Utilisation d'un composé de formule (I) selon l'une quelconque des revendications 1 à 10 pour la préparation d'un médicament destiné au traitement/la prophylaxie des glaucomes, des maladies rénales ou dans les maladies cérébrales d'origine neurodégénératives ou pas, des anémies, ou d'un médicament destiné à favoriser la cicatrisation ou agents ou à raccourcir la période de reconvalescence post-opératoire ou d'un médicament destiné au traitement des états de fatigue générale ou encore d'un médicament utilisé dans le but d'obtenir du sang dans le cadre d'autotransfusions nécessaires à la suite d'interventions chirurgicales lourdes telles que la chirurgie cranienne ou thoracique ou comme les opérations cardiaques ou au niveau carotidien ou aortique.

18. Utilisation d'un composé de formule (I) selon l'une quelconque des revendications 1 à 10 pour la préparation d'un médicament destiné au traitement/la prophylaxie de maladies cardiaques ou périphériques d'origine ischémique via une médecine régénérative utilisant des cellules souches.

19. Association d'un composé de formule (I) selon l'une quelconque des revendications 1 à 10 avec un ou des autres composé(s) actif(s) utiles dans le traitement de l'hypertension, de l'insuffisance cardiaque, du diabète et de l'anémie.

## Patentansprüche

1. Verbindung der Formel (I) : 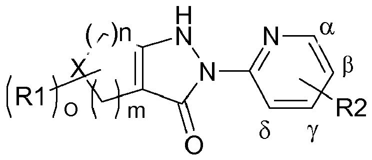 mit den folgenden Bedeutungen:
**n** ist 0, 1, 2, 3 oder 4;
**m** ist 0, 1 oder 2;
**o** ist 0 oder 1;
**X** bedeutet eine -CH₂, -CH(R')-, -N(R')- Gruppe oder ein Heteroatom, ausgewählt aus dem Sauerstoffatom und dem Schwefelatom, mit der Maßgabe, dass R' eine -(C1-C5)-Alkylgruppe, -(C1-C5)-Alkoxygruppe, -CH₂-Arylgruppe, -C(O)R5-Gruppe oder -COOR5-Gruppe, wobei R5 wie unten definiert ist, bedeutet;
**R1** eine Oxogruppe, eine -COOR5-Gruppe, eine -W-OH-Gruppe oder eine -W-NR5R6-Gruppe, wobei W, R5 und R6 wie unten definiert sind, bedeutet; und
**R2** ein Wasserstoffatom oder eine Gruppe, ausgewählt aus (i) den -(C1-C5)-Alkylgruppen, (ii) den -(C1-C5)-Alkoxygruppen, (iii) den -COOR5-Gruppen, (iv) den-NR5R6-Gruppen, (v) den -C(O)-NR5R6-Gruppen, (vi) den - SO₂-NR3R4-Gruppen, (vii) den Heteroarylgruppen, die gegebenenfalls durch eine -(C1-C5)-Alkylgruppe substituiert sind, (viii) den -W-Arylgruppen, (ix) den -W-Heteroarylgruppen, (x) den -O-W-Arylgruppen, (xi) den -0-W-Heteroarylgruppen und (xii) den -0-W-NR5R6-Gruppen, wobei W, R3, R4, R5 und R6 wie unten definiert sind, bedeutet;
mit der Maßgabe, dass:
**R3** und **R4**,
(i) die gleich oder verschieden sein können, unabhängig voneinander ein Wasserstoffatom, eine -(C1-C5)-Alkylgruppe, eine -(C3-C6)-Cycloalkylgruppe, eine Arylgruppe, eine Heteroarylgruppe, eine -CH₂-Heteroarylgruppe, eine -(C1-C5)-Alkyl-NR5R6-Gruppe, eine -W-OH-Gruppe oder eine -W-NR5R6-Gruppe bedeuten; oder
(ii) gemeinsam mit dem Stickstoffatom, von dem sie getragen werden, eine Heterocycloalkylgruppe, die gegebenenfalls durch eine oder mehrere Gruppen, ausgewählt aus -(C1-C5)-Alkylgruppen und -CH₂-Arylgruppen, substituiert ist, bilden;
**W** eine -(C1-C5)-Alkylengruppe, die gegebenenfalls durch eine oder mehrere Hydroxygruppen, substituiert ist, bedeutet; und
**R5** und **R6**, die gleich oder verschieden sein können, unabhängig voneinander ein Wasserstoffatom oder eine Gruppe, ausgewählt aus -(C1-C5)-Alkylgruppen und -(C3-C6)-Cycloalkylgruppen, bedeuten;
in Form einer Base oder eines Säureadditionssalzes.

2. Verbindung der Formel (I) nach Anspruch 1, **dadurch gekennzeichnet, dass**
• **n** 0, 1, 2, 3 oder 4 ist;
• **m** 0, 1 oder 2 ist;
• **o** 0 oder 1 ist;
• **X** eine -CH₂-, -CH(R')-, -N(R')-Gruppe oder ein Heteroatom, ausgewählt aus dem Sauerstoffatom und dem Schwefelatom, bedeutet;
• **R' eine** -(C1-C5)-Alkylgruppe, eine -(C1-C5)-Alkoxygruppe, eine -CH₂-Arylgruppe, eine -C(O)R5-Gruppe oder eine -COOR5-Gruppe bedeutet;
• **R1** eine Oxogruppe, eine -COOR5-Gruppe, eine -W-OH-Gruppe oder eine -W-NR5R6-Gruppe bedeutet;
• **R2** ein Wasserstoffatom, eine -(C1-C5)-Alkylgruppe, eine -(C1-C5)-Alkoxygruppe, eine -COOR5-Gruppe, eine - NR5R6-Gruppe, eine -C(O)-NR5R6-Gruppe oder eine -SO₂-NR3R4-Gruppe bedeutet;
• **R3** und **R4**
(i) unabhängig voneinander ein Wasserstoffatom, eine -(C1-C5)-Alkylgruppe, eine -(C3-C6)-Cycloalkylgruppe, eine Arylgruppe, eine Heteroarylgruppe, eine -CH₂-Heteroarylgruppe oder eine -(C1-C5)-Alkyl-NR5R6-Gruppe bedeuten; oder
(ii) gemeinsam mit dem Stickstoffatom, von dem sie getragen werden, eine Heterocycloalkylgruppe, die gegebenenfalls durch eine -(C1-C5)-Alkylgruppe oder eine Arylgruppe substituiert ist, bilden;
• **W** eine -(C1-C5)-Alkylengruppe, die gegebenenfalls durch eine oder mehrere Hydroxygruppen substituiert ist, bedeutet; und/oder
• **R5** und **R6** unabhängig voneinander ein Wasserstoffatom oder eine -(C1-C5)-Alkylgruppe bedeuten.

3. Verbindung der Formel (I) nach Anspruch 1, **dadurch gekennzeichnet, dass**
• **n** 0, 1, 2, 3 oder 4 ist;
• **m** 0, 1 oder 2 ist;
• **o** 0 ist;
• **R1** eine Oxogruppe, eine -CH₂-Arylgruppe, eine - C(0)R5-Gruppe oder eine -COOR5-Gruppe bedeutet, wobei die R1-Gruppe an ein Kohlenstoffatom oder ein Heteroatom gebunden sein kann, und wobei die Arylgruppe vorteilhafterweise eine Phenylgruppe bedeutet;
• **R3** und **R4**
(i) unabhängig voneinander ein Wasserstoffatom, eine - (C1-C5) -Alkylgruppe, eine - (C3-C6) - Cycloalkylgruppe, eine Arylgruppe, eine Heteroarylgruppe, eine -CH₂-Heteroarylgruppe oder eine -(C1-C5)-Alkyl-NR5R6-Gruppe bedeuten, wobei die Arylgruppe vorteilhafterweise eine Phenylgruppe bedeutet und die Heteroarylgruppe eine Pyridinylgruppe oder eine Furanylgruppe bedeutet; oder
(ii) gemeinsam mit dem Stickstoffatom, von dem sie getragen werden, eine Heterocycloalkylgruppe, die gegebenenfalls durch eine oder mehrere -(C1-C5)-Alkyl-und/oder Arylgruppe(n) substituiert ist, bilden, wobei die Heterocycloalkylgruppe vorteilhafterweise eine Piperidinylgruppe, eine Pyrrolidinylgruppe oder eine Hexamethyleniminogruppe bedeutet und die Arylgruppe eine Phenylgruppe bedeutet;
• **R5** eine -(C1-C5)-Alkyl- oder eine -(C1-C5)-Cycloalkylgruppe bedeutet; und/oder
• **R6** ein Wasserstoffatom oder eine (C1-C5)-Alkylgruppe bedeutet.

4. Verbindung der Formel (I) nach Anspruch 1, **dadurch gekennzeichnet, dass**
• **n** 0, 1, 2, 3 oder 4 ist;
• **m** 0, 1 oder 2 ist;
• **o** 0 oder 1 ist;
• **X** eine -CH₂-Gruppe, eine -CH(R')-Gruppe, eine - N(R')-Gruppe oder ein Heteroatom, ausgewählt aus dem Sauerstoffatom und dem Schwefelatom, bedeutet;
• **R'** eine -(C1-C5)Alkylgruppe, eine -(C1-C5)-Alkoxygruppe, eine -CH₂-Arylgruppe, eine -C(0)R5-Gruppe oder eine -COOR5-Gruppe bedeutet;
• **R1** eine Oxogruppe, eine COOR5-Gruppe, eine -W-OH-Gruppe oder eine -W-NR5R6-Gruppe bedeutet;
• **R2** eine -SO₂-NR3R4-Gruppe bedeutet;
• **R3** und **R4**
(i) unabhängig voneinander ein Wasserstoffatom, eine - (C1-C5) -Alkylgruppe, eine -(C3-C6)-Cycloalkylgruppe, eine Aryl-Gruppe, eine Heteroarylgruppe oder eine -CH₂-Heteroarylgruppe bedeuten, oder
(ii) gemeinsam mit dem Stickstoffatom, von dem sie getragen werden, eine Heterocycloalkylgruppe bilden; und
• **R5** und **R6** eine -(C1-C5)-Alkylgruppe bedeuten.

5. Verbindung der Formel (I) nach Anspruch 1, **dadurch gekennzeichnet, dass**
• **n** 0, 1, 2, 3 oder 4 ist;
• **m** 0, 1 oder 2 ist;
• **o** 0 oder 1 ist;
• **X** eine -CH₂-Gruppe, eine -CH(R')-Gruppe, eine - N(R')-Gruppe oder ein Heteroatom, ausgewählt aus dem Sauerstoffatom und dem Schwefelatom, bedeutet;
• R' eine -(C1-C5)Alkylgruppe, eine -(C1-C5)-Alkoxygruppe, eine -CH₂-Arylgruppe, eine -C(O)R5-Gruppe oder eine -COOR5-Gruppe bedeutet;
• **R1** eine Oxogruppe bedeutet;
• **R2** ein Wasserstoffatom, eine -(C1-C5)-Alkylgruppe, eine -(C1-C5)-Alkoxygruppe, eine -COOR5-Gruppe, eine -NR5R6-Gruppe oder eine -C(0)-NR5R6-Gruppe bedeutet; und
• **R5** und **R6** unabhängig voneinander ein Wasserstoffatom, eine -(C1-C5)-Alkylgruppe oder eine -(C1-C5)-Cycloalkylgruppe bedeuten.

6. Verbindung der Formel (I) nach Anspruch 1, **dadurch gekennzeichnet, dass R2** (i) ein Wasserstoffatom, (ii) eine -(C1-C5)-Alkylgruppe, (iii) eine -(C1-C5)-Alkoxygruppe, (iv) eine -COOR5-Gruppe, (v) eine -NR5R6-Gruppe, (vi) eine -C(0)-NR5R6-Gruppe, (vii) ein Heteroaryl, das durch eine -(C1-C5)-Alkylgruppe substituiert ist, (viii) eine -O-W-Arylgruppe oder (ix) eine -O-W-Heteroarylgruppe bedeutet.

7. Verbindung der Formel (I) nach Anspruch 1, **dadurch gekennzeichnet, dass R2** eine -SO₂-NR3R4-Gruppe bedeutet.

8. Verbindung der Formel (I) nach einem der Ansprüche 1 bis 7, **dadurch gekennzeichnet, dass R2** ein Substituent des in beta-Stellung des Pyridins gelegenen Atoms ist.

9. Verbindung der Formel (I) nach einem der Ansprüche 1 bis 7, **dadurch gekennzeichnet, dass R2** ein Substituent des in gamma-Stellung des Pyridins gelegenen Atoms ist.

10. Verbindung der Formel (I) nach einem der vorhergehenden Ansprüche, **dadurch gekennzeichnet, dass** es sich um Folgendes handelt:
• 2-[5-(Piperidin-1-ylsulfonyl)pyridin-2-yl]-1,2,4,5,6,7-hexahydro-3H-indazol-3-on;
• 6-Methyl-2-[5-(piperidin-1-ylsulfonyl)pyridin-2-yl]-1,2,4,5,6,7-hexahydro-3H-indazol-3-on;
• 2-[5-(Piperidin-1-ylsulfonyl)pyridin-2-yl]-1,4,5,6,7,8-hexahydrocyclohepta[c]pyrazol-3(2H)-on;
• N-Ethyl-6-(3-oxo-1,3,4,5,6,7-hexahydro-2H-indazol-2-yl)-N-phenylpyridin-3-sulfonamid;
• 6-(5-Benzyl-3-oxo-1,3,4,5,6,7-hexahydro-2H-pyrazolo[4,3-c]pyridin-2-yl)-N-ethyl-N-phenylpyridin-3-sulfonamid;
• (±) 2-(5-{[(3R,5S)-3,5-Dimethylpiperidin-1-yl]sulfonyl}pyridin-2-yl)-1,2,4,5,6,7-hexahydro-3H-indazol-3-on;
• 2-(4-Methoxypyridin-2-yl)-1,2,4,6-tetrahydro-3H-thieno[3,4-c]pyrazol-3-on;
• 2-(Pyridin-2-yl)-1,4,5,6-tetrahydrocyclopenta[c]pyrazol-3(2H)-on;
• (±)5-Benzyl-2-(5-{[(3R,5S)-3,5-dimethylpiperidin-1-yl]sulfonyl}pyridin-2-yl)-1,2,4,5,6,7-hexahydro-3H-pyrazolo[4,3-c]pyridin-3-on;
• (±) 2-(5-{[(3R,5S)-3,5-Dimethylpiperidin-1-yl]sulfonyl}pyridin-2-yl)-6-methyl-1,2,4,5,6,7-hexahydro-3H-indazol-3-on;
• 6-(5-Benzyl-3-oxo-1,3,4,5,6,7-hexahydro-2H-pyrazolo[4,3-c]pyridin-2-yl)-N,N-diethylpyridin-3-sulfonamid;
• N-Ethyl-6-(3-oxo-1,3,4,5,6,7,8,9-octahydro-2H-cycloocta[c]pyrazol-2-yl)-N-phenylpyridin-3-sulfonamid;
• N-Ethyl-6-(3-oxo-4,6-dihydro-1H-thieno[3,4-c]pyrazol-2(3H)-yl)-N-phenylpyridin-3-sulfonamid;
• 6-(5-Benzyl-3-oxo-1,3,4,5,6,7-hexahydro-2H-pyrazolo[4,3-c]pyridin-2-yl)-N,N-di(propan-2-yl)pyridin-3-sulfonamid;
• 6-Methoxy-2-(pyridin-2-yl)-1,4,5,6-tetrahydrocyclopenta[c]pyrazol-3(2H)-on;
• 2-(Pyridin-2-yl)-1,4,6,7-tetrahydrothiopyrano[4,3-c]pyrazol-3(2H)-on;
• N-Ethyl-6-(3-oxo-1,4,6,7-tetrahydrothiopyrano[4,3-c]pyrazol-2(3H)-yl)-N-phenylpyridin-3-sulfonamid;
• N-Ethyl-6-(3-oxo-3,5,6,7-tetrahydrothiopyrano[3,2-c]pyrazol-2(1H)-yl)-N-phenylpyridin-3-sulfonamid;
• N,N-Diethyl-6-(3-oxo-4,6-dihydro-1H-thieno[3,4-c]pyrazol-2(3H)-yl)pyridin-3-sulfonamid;
• N,N-Dimethyl-6-(3-oxo-4,6-dihydro-1H-thieno[3,4-c]pyrazol-2(3H)-yl)pyridin-3-sulfonamid;
• 2-[5-(Pyrrolidin-1-ylsulfonyl)pyridin-2-yl]-1,2,4,6-tetrahydro-3H-thieno[3,4-c]pyrazol-3-on;
• N-Cyclopropyl-6-(3-oxo-4,6-dihydro-1H-thieno[3,4-c]pyrazol-2(3H)-yl)pyridin-3-sulfonamid;
• 6-(3-Oxo-4,6-dihydro-1H-thieno[3,4-c]pyrazol-2(3H)-yl)-N-(propan-2-yl)pyridin-3-sulfonamid;
• N-tert.-Butyl-6-(3-oxo-4,6-dihydro-1H-thieno[3,4-c]pyrazol-2(3H)-yl)pyridin-3-sulfonamid;
• N-(Furan-2-ylmethyl)-6-(3-oxo-4,6-dihydro-1H-thieno[3,4-c]pyrazol-2(3H)-yl)pyridin-3-sulfonamid;
• N-Cyclopentyl-6-(3-oxo-4,6-dihydro-1H-thieno[3,4-c]pyrazol-2(3H)-yl)pyridin-3-sulfonamid;
• N-Methyl-6-(3-oxo-4,6-dihydro-1H-thieno[3,4-c]pyrazol-2(3H)-yl)-N-(pyridin-2-yl)pyridin-3-sulfonamid;
• 2-(Pyridin-2-yl)-1,2,4,6-tetrahydro-3H-thieno[3,4-c]pyrazol-3-on;
• 2-[4-(Dimethylamino)pyridin-2-yl]-1,2,4,6-tetrahydro-3H-thieno[3,4-c]pyrazol-3-on;
• 2-{5-[(4-Benzylpiperidin-1-yl)sulfonyl]pyridin-2-yl}-1,2,4,6-tetrahydro-3H-thieno[3,4-c]pyrazol-3-on;
• 6-(3-Oxo-4,6-dihydro-1H-thieno[3,4-c]pyrazol-2(3H)-yl)-N-(pyridin-2-yl)pyridin-3-sulfonamid;
• N-Ethyl-6-(3-oxo-1,4,6,7-tetrahydropyrano[4,3-c]pyrazol-2(3H)-yl)-N-phenylpyridin-3-sulfonamid;
• Natrium-2-(4-ethylpyridin-2-yl)-2,6-dihydro-4H-thieno[3,4-c]pyrazol-3-olat;
• 2-[5-(Azepan-1-ylsulfonyl)pyridin-2-yl]-1,2,4,6-tetrahydro-3H-thieno[3,4-c]pyrazol-3-on;
• Natrium-4-benzyl-2-{5-[ethyl(phenyl)sulfamoyl]pyridin-2-yl}-5-oxo-4,5,6,7-tetrahydro-2H-pyrazolo[4,3-b]pyridin-3-olat;
• N-Methyl-6-(3-oxo-4,6-dihydro-1H-thieno[3,4-c]pyrazol-2(3H)-yl)-N-(pyridin-2-ylmethyl)pyridin-3-sulfonamid;
• tert.-Butyl-2-{5-[ethyl(phenyl)sulfamoyl]pyridin-2-yl}-3-oxo-1,2,3,4,6,7-hexahydro-5H-pyrazolo[4,3-c]pyridin-5-carboxylat;
• 6-(5-Acetyl-3-oxo-1,3,4,5,6,7-hexahydro-2H-pyrazolo[4,3-c]pyridin-2-yl)-N-ethyl-N-phenylpyridin-3-sulfonamid;
• tert.-Butyl-6-(3-oxo-4,6-dihydro-1H-thieno[3,4-c]pyrazol-2(3H)-yl)pyridin-3-carboxylat;
• Natrium-2-(4-methylpyridin-2-yl)-2,6-dihydro-4H-thieno[3,4-c]pyrazol-3-olat;
• Natrium-2-{5-[tert.-butyl(methyl)sulfamoyl]pyridin-2-yl}-2,6-dihydro-4H-thieno[3,4-c]pyrazol-3-olat;
• Natrium-2-{5-[tert.-butyl(ethyl)sulfamoyl]pyridin-2-yl}-2,6-dihydro-4H-thieno[3,4-c]pyrazol-3-olat;
• Natrium-2-(5-methylpyridin-2-yl)-2,6-dihydro-4H-thieno[3,4-c]pyrazol-3-olat;
• Natrium-2-[5-(tert.-butylcarbamoyl)pyridin-2-yl]-2,6-dihydro-4H-thieno[3,4-c]pyrazol-3-olat;
• Natrium-2-(5-methoxypyridin-2-yl)-2,6-dihydro-4H-thieno[3,4-c]pyrazol-3-olat;
• N-Methyl-6-(3-oxo-4,6-dihydro-1H-thieno[3,4-c]pyrazol-2(3H)-yl)-N-(pyridin-4-yl)pyridin-3-sulfonamid;
• Natrium-2-{5-[cyclopentyl(ethyl)sulfamoyl]pyridin-2-yl}-2,6-dihydro-4H-thieno[3,4-c]pyrazol-3-olat;
• Natrium-2-[4-(pyridin-3-ylmethoxy)pyridin-2-yl]-2,6-dihydro-4H-thieno[3,4-c]pyrazol-3-olat;
• Methyl-2-{5-[ethyl(phenyl)sulfamoyl]pyridin-2-yl}-3-oxo-1,2,3,4,6,7-hexahydro-5H-pyrazolo[4,3-c]pyridin-5-carboxylat;
• Cyclopentyl-6-(3-oxo-4,6-dihydro-1H-thieno[3,4-c]pyrazol-2(3H)-yl)pyridin-3-carboxylat;
• 2-Methylpropyl-6-(3-oxo-4,6-dihydro-1H-thieno[3,4-c]pyrazol-2(3H)-yl)pyridin-3-carboxylat;
• 2-[4-(Propan-2-yl)pyridin-2-yl]-1,2,4,6-tetrahydro-3H-thieno[3,4-c]pyrazol-3-on;
• Natrium-2-[5-(propan-2-yl)pyridin-2-yl]-2,6-dihydro-4H-thieno[3,4-c]pyrazol-3-olat;
• Methyl-2-{5-[ethyl(phenyl)sulfamoyl]pyridin-2-yl}-3-oxo-2,3,4,6-tetrahydro-1H-thieno[3,4-c]pyrazol-4-carboxylat;
• Propan-2-yl-6-(3-oxo-4,6-dihydro-1H-thieno[3,4-c]pyrazol-2(3H)-yl)pyridin-3-carboxylat;
• Natrium-2-{5-[ethyl(phenyl)sulfamoyl]pyridin-2-yl}-6-methoxy-2,4,5,6-tetrahydrocyclopenta[c]pyrazol-3-olat;
• N-Ethyl-6-(3-oxo-3,4,5,6-tetrahydrocyclopenta[c]pyrazol-2(1H)-yl)-N-phenylpyridin-3-sulfonamid;
• Natrium-2-[4-(pyridin-3-ylmethoxy)pyridin-2-yl]-2,6-dihydro-4H-thieno[3,4-c]pyrazol-3-olat;
• 2,2-Dimethylpropyl 6-(3-oxo-4,6-dihydro-1H-thieno[3,4-c]pyrazol-2(3H)-yl)pyridin-3-carboxylat;
• 2-[5-(5-tert.-Butyl-1,2,4-oxadiazol-3-yl)pyridin-2-yl]-1,2,4,6-tetrahydro-3H-thieno[3,4-c]pyrazol-3-on;
• N-Cyclopentyl-N-methyl-6-(3-oxo-4,6-dihydro-1H-thieno[3,4-c]pyrazol-2(3H)-yl)pyridin-3-sulfonamid;
• N-Cyclopentyl-N-ethyl-6-(3-oxo-3,4,5,6-tetrahydrocyclopenta[c]pyrazol-2(1H)-yl)pyridin-3-sulfonamid;
• N-Cyclopentyl-N-(2,3-dihydroxypropyl)-6-(3-oxo-4,6-dihydro-1H-thieno[3,4-c]pyrazol-2(3H)-yl)pyridin-3-sulfonamid;
• 2,2-Dimethylpropyl-6-[5-(methylsulfonyl)-3-oxo-1,3,4,5,6,7-hexahydro-2H-pyrazolo[4,3-c]pyridin-2-yl]pyridin-3-carboxylat;
• 2-[5-(3-tert.-Butyl-1,2,4-oxadiazol-5-yl)pyridin-2-yl]-1,2,4,6-tetrahydro-3H-thieno[3,4-c]pyrazol-3-on.

11. Verfahren zur Herstellung einer Verbindung der Formel (I) nach einem der Ansprüche 1 bis 10, **dadurch gekennzeichnet, dass** man eine Verbindung der Formel (IV), die in Form einer Verbindung der Formel (IVa) oder (IVb) oder beiden vorliegt, 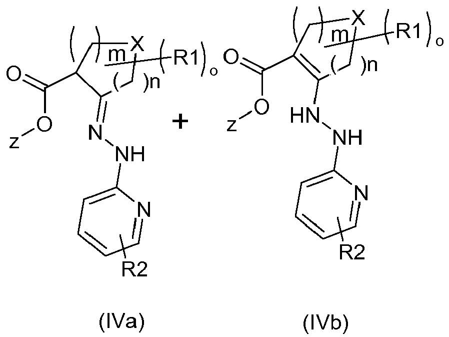 worin X, R1, R2, n, m, o wie in Anspruch 1 definiert sind und z eine Alkylgruppe bedeutet, mit einer organischen Base umsetzt.

12. Verbindungen der Formeln (IVa) und (IVb) 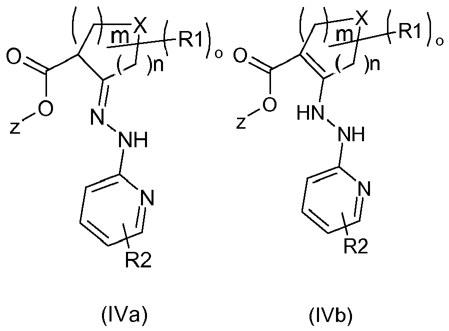 worin R1, R2, n, m und o wie in Anspruch 1 definiert sind, z eine Alkylgruppe bedeutet und X -CH(R')-, -N(R')-oder ein Heteroatom bedeutet, wobei R' und das Heteroatom wie in Anspruch 1 definiert sind.

13. Arzneimittel, **dadurch gekennzeichnet, dass** es eine Verbindung der Formel (I) nach einem der Ansprüche 1 bis 10 oder ein Additionssalz dieser Verbindung mit einer pharmazeutisch unbedenklichen Säure der Verbindung der Formel (I) umfasst.

14. Pharmazeutische Zusammensetzung, **dadurch gekennzeichnet, dass** sie eine Verbindung der Formel (I) nach einem der Ansprüche 1 bis 10 oder ein pharmazeutisch unbedenkliches Salz der Verbindung sowie mindestens einen pharmazeutisch unbedenklichen Grundstoff umfasst.

15. Verwendung einer Verbindung der Formel (I) nach einem der Ansprüche 1 bis 10 für die Herstellung eines Arzneimittels zur Behandlung/Prophylaxe von Herz-Kreislauf-Erkrankungen.

16. Verwendung einer Verbindung der Formel (I) nach einem der Ansprüche 1 bis 10 für die Herstellung eines Arzneimittels zur Behandlung/Prophylaxe von Ischämie der unteren Gliedmaßen, Herzinsuffizienz, Koronarerkrankungen ischämischen Ursprungs, zum Beispiel Angina pectoris oder Myokardinfarkt, Arteriosklerose, Schlaganfällen ischämischen Ursprungs, pulmonaler Hypertonie und allen durch teilweisen oder vollständigen Gefäßverschluss verursachten Pathologien.

17. Verwendung einer Verbindung der Formel (I) nach einem der Ansprüche 1 bis 10 für die Herstellung eines Arzneimittels zur Behandlung/Prophylaxe von Glaukomen, Nierenerkrankungen oder Hirnerkrankungen neurodegenerativen oder anderen Ursprungs, Anämien, oder eines Medikaments zur Förderung der Vernarbung oder Mitteln zur Verkürzung des Zeitraums der postoperativen Genesung oder eines Medikaments zur Behandlung von allgemeinen Erschöpfungszuständen oder auch eines Medikaments zum Erhalt von Blut im Rahmen von Autotransfusionen, die nach schweren chirurgischen Eingriffen wie Schädel- oder Thoraxchirurgie oder bei Herz-, Karotis- oder Aortaoperationen notwendig sind.

18. Verwendung einer Verbindung der Formel (I) nach einem der Ansprüche 1 bis 10 für die Herstellung eines Arzneimittels zur Behandlung/Prophylaxe von Herzerkrankungen oder peripheren Erkrankungen ischämischen Ursprungs mit einer regenerativen Medizin unter Verwendung von Stammzellen.

19. Kombination einer Verbindung der Formel (I) nach einem der Ansprüche 1 bis 10 mit einem oder mehreren anderen Wirkstoff(en) der bzw. die bei der Behandlung von Hypertonie, Herzinsuffizienz, Diabetes und Anämie verwendbar ist/sind.

## Claims

1. Compound of formula (I): 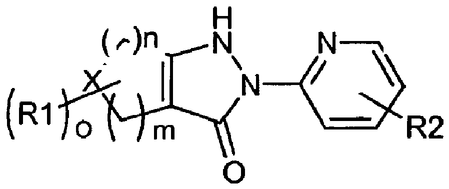 in which
**n** is equal to 0, 1, 2, 3 or 4;
**m** is equal to 0, 1 or 2;
**o** is equal to 0 or 1;
**X** represents a group -CH₂, -CH(R')-, -N(R')- or a heteroatom chosen from an oxygen atom and a sulfur atom, it being understood that R' represents a group -(C1-C5)alkyl, -(C1-C5)alkoxy, -CH₂-aryl, -C(O)R5 or -COOR5 with R5 as defined below;
**R1** represents an oxo group, a group -COOR5, a group -W-OH or a group -W-NR5R6, with W, R5 and R6 as defined below; and
**R2** represents a hydrogen atom or a group chosen from (i) groups -(C1-C5)alkyl, (ii) groups -(C1-C5)alkoxy, (iii) groups -COOR5, (iv) groups -NR5R6, (v) groups -C(O)-NR5R6, (vi) groups -SO₂-NR3R4, (vii) heteroaryl groups optionally substituted with a group -(C1-C5)alkyl, (viii) groups -W-aryl, (ix) groups -W-heteroaryl, (x) groups -O-W-aryl, (xi) groups -0-W-heteroaryl and (xii) groups -O-W-NR5R6, with W, R3, R4, R5 and R6 as defined below;
it being understood that:
**R3** and **R4,**
(i) which may be identical or different, represent, independently of each other, a hydrogen atom, a group -(C1-C5)alkyl, a group -(C3-C6)cycloalkyl, an aryl group, a heteroaryl group, a group -CH₂-heteroaryl, a group -(C1-C5)alkyl-NR5R6, a group -W-OH or a group -W-NR5R6; or
(ii) form, together with the nitrogen atom that bears them, a heterocycloalkyl group optionally substituted with one or more groups chosen from groups -(C1-C5)alkyl and groups -CH₂-aryl;
**W** is a group -(C1-C5)alkylene, optionally substituted with one or more hydroxyl groups; and
**R5** and **R6,** which may be identical or different, represent, independently of each other, a hydrogen atom or a group chosen from groups -(C1-C5)alkyl and groups -(C3-C6)cycloalkyl;
in the form of the base or of an acid-addition salt.

2. Compound of formula (I) according to Claim 1, **characterized in that**
• **n** is equal to 0, 1, 2, 3 or 4;
• **m** is equal to 0, 1 or 2;
• **o** is equal to 0 or 1;
• **X** represents a group -CH₂-, -CH(R')-, -N(R')- or a heteroatom chosen from an oxygen atom and a sulfur atom;
• **R'** represents a group -(C1-C5)alkyl, a group -(C1-C5)alkoxy, a group -CH₂-aryl, a group -C(O)R5 or a group -COOR5;
• **R1** represents an oxo group, a group -COOR5, a group -W-OH or a group -W-NR5R6;
• **R2** represents a hydrogen atom, a group -(C1-C5)alkyl, a group -(C1-C5)alkoxy, a group -COOR5, a group -NR5R6, a group -C(O)-NR5R6 or a group -SO₂-NR3R4;
• **R3** and **R4**
(i) represent, independently of each other, a hydrogen atom, a group -(C1-C5)alkyl, a group -(C3-C6)cycloalkyl, an aryl group, a heteroaryl group, a group -CH₂-heteroaryl or a group -(C1-C5))alkyl-NR5R6; or
(ii) form, together with the nitrogen atom that bears them, a heterocycloalkyl group optionally substituted with a group -(C1-C5)alkyl or with an aryl group;
• **W** represents a group -(C1-C5)alkylene, optionally substituted with one or more hydroxyl groups; and/or
• **R5** and **R6** represent, independently of each other, a hydrogen atom or a group -(C1-C5)alkyl.

3. Compound of formula (I) according to Claim 1, **characterized in that**
• **n** is equal to 0, 1, 2, 3 or 4;
• **m** is equal to 0, 1 or 2;
• **o** is equal to 0;
• **R1** represents an oxo group, a group -CH₂-aryl, a group -C(O)R5- or a group -COOR5, the said group R1 possibly being linked to a carbon atom or a heteroatom, the said aryl group advantageously representing a phenyl group;
• **R3** and **R4**
(i) represent, independently of each other, a hydrogen atom, a group -(C1-C5)alkyl, a group -(C3-C6)cycloalkyl, an aryl group, a heteroaryl group, a group -CH₂-heteroaryl or a group -(C1-C5)alkyl-NR5R6, advantageously, the said aryl group representing a phenyl group and the said heteroaryl group representing a pyridyl group or a furyl group; or
(ii) form, together with the nitrogen atom that bears them, a heterocycloalkyl group optionally substituted with one or more group(s) -(C1-C5)alkyl and/or aryl, advantageously, the said heterocycloalkyl group representing a piperidyl group or a hexamethyleneimino group and the said aryl group representing a phenyl group;
• **R5** represents a group -(C1-C5)alkyl or a group -(C1-C5)cycloalkyl; and/or
• **R6** represents a hydrogen atom or a group (C1-C5)alkyl.

4. Compound of formula (I) according to Claim 1, **characterized in that**
• **n** is equal to 1, 2, 3 or 4;
• **m** is equal to 0, 1 or 2;
• **o** is equal to 0 or 1;
• **X** represents a group -CH₂-, a group -CH(R')-, a group -N(R')- or a heteroatom chosen from an oxygen atom and a sulfur atom;
• **R'** represents a group -(C1-C5)alkyl, a group - (C1-C5) alkoxy, a group -CH₂-aryl, a group -C(O)R5 or a group -COOR5;
• **R1** represents an oxo group, a group COOR5, a group -W-OH or a group -W-NR5R6;
• **R2** represents a group -SO₂-NR3R4;
• **R3** and **R4**
(i) represent, independently of each other, a hydrogen atom, a group -(C1-C5)alkyl, a group -(C3-C6)cycloalkyl, an aryl group, a heteroaryl group or a group -CH₂-heteroaryl, or
(ii) form, together with the nitrogen atom that bears them, a heterocycloalkyl group; and
• **R5** and **R6** represent a group -(C1-C5)alkyl.

5. Compound of formula (I) according to Claim 1, **characterized in that**
• **n** is equal to 1, 2, 3 or 4;
• **m** is equal to 0, 1 or 2;
• **o** is equal to 0 or 1;
• **X** represents a group -CH₂-, a group -CH(R')-, a group -N(R')- or a heteroatom chosen from an oxygen atom and a sulfur atom;
• **R'** represents a group -(C1-C5)alkyl, a group - (C1-C5) alkoxy, a group -CH₂-aryl, a group -C(O)R5 or a group -COOR5;
• **R1** represents an oxo group;
• **R2** represents a hydrogen atom, a group -(C1-C5)alkyl, a group -(C1-C5)alkoxy, a group -COOR5, a group -NR5R6 or -C(O)-NR5R6; and
• **R5** and **R6** represent, independently of each other, a hydrogen atom, a group -(C1-C5)alkyl or a group -(C1-C5)cycloalkyl.

6. Compound of formula (I) according to Claim 1, **characterized in that R2** represents (i) a hydrogen atom, (ii) a group -(C1-C5)alkyl, (iii) a group -(C1-C5)alkoxy, (iv) a group -COOR5, (v) a group -NR5R6, (vi) a group -C(O)-NR5R6, (vii) a heteroaryl substituted with a group -(C1-C5) alkyl, (viii) a group -O-W-aryl or (ix) a group -0-W-heteroaryl.

7. Compound of formula (I) according to Claim 1, **characterized in that R2** represents a group -SO₂-NR3R4.

8. Compound of formula (I) according to any one of Claims 1 to 7, **characterized in that R2** is a substituent on the atom in the beta position of pyridine.

9. Compound of formula (I) according to any one of Claims 1 to 7, **characterized in that R2** is a substituent on the atom in the gamma position of pyridine.

10. Compound of formula (I) according to any one of the preceding claims, **characterized in that** it is:
• 2-[5-(piperidin-1-ylsulfonyl)pyridin-2-yl]-1,2,4,5,6,7-hexahydro-3H-indazol-3-one;
• 6-methyl-2-[5-(piperidin-1-ylsulfonyl)pyridin-2-yl]-1,2,4,5,6,7-hexahydro-3H-indazol-3-one;
• 2-[5-(piperidin-1-ylsulfonyl)pyridin-2-yl]-1,4,5,6,7,8-hexahydrocyclohepta[c]pyrazol-3(2H)-one;
• N-ethyl-6-(3-oxo-1,3,4,5,6,7-hexahydro-2H-indazol-2-yl)-N-phenylpyridine-3-sulfonamide;
• 6-(5-benzyl-3-oxo-1,3,4,5,6,7-hexahydro-2H-pyrazolo[4,3-c]pyridin-2-yl)-N-ethyl-N-phenylpyridine-3-sulfonamide;
• (±) 2-(5-{[(3R,5S)-3,5-dimethylpiperidin-1-yl]sulfonyl}pyridin-2-yl)-1,2,4,5,6,7-hexahydro-3H-indazol-3-one;
• 2-(4-methoxypyridin-2-yl)-1,2,4,6-tetrahydro-3H-thieno[3,4-c]pyrazol-3-one;
• 2-(pyridin-2-yl)-1,4,5,6-tetrahydrocyclopenta[c]pyrazol-3(2H)-one;
• (±)5-benzyl-2-(5-{[(3R,5S)-3,5-dimethylpiperidin-1-yl]sulfonyl}pyridin-2-yl)-1,2,4,5,6,7-hexahydro-3H-pyrazolo[4,3-c]pyridin-3-one;
• (±) 2-(5-{[(3R,5S)-3,5-dimethylpiperidin-1-yl]sulfonyl}pyridin-2-yl)-6-methyl-1,2,4,5,6,7-hexahydro-3H-indazol-3-one;
• 6-(5-benzyl-3-oxo-1,3,4,5,6,7-hexahydro-2H-pyrazolo[4,3-c]pyridin-2-yl)-N,N-diethylpyridine-3-sulfonamide;
• N-ethyl-6-(3-oxo-1,3,4,5,6,7,8,9-octahydro-2H-cycloocta[c]pyrazol-2-yl)-N-phenylpyridine-3-sulfonamide;
• N-ethyl-6-(3-oxo-4,6-dihydro-1H-thieno[3,4-c]pyrazol-2(3H)-yl)-N-phenylpyridine-3-sulfonamide;
• 6-(5-benzyl-3-oxo-1,3,4,5,6,7-hexahydro-2H-pyrazolo[4,3-c]pyridin-2-yl)-N,N-di(propan-2-yl)pyridine-3-sulfonamide;
• 6-methoxy-2-(pyridin-2-yl)-1,4,5,6-tetrahydrocyclopenta[c]pyrazol-3(2H)-one;
• 2-(pyridin-2-yl)-1,4,6,7-tetrahydrothiopyrano[4,3-c] pyrazol-3 (2H) -one;
• N-ethyl-6-(3-oxo-1,4,6,7-tetrahydrothiopyrano[4,3-c]pyrazol-2(3H)-yl)-N-phenylpyridine-3-sulfonamide;
• N-ethyl-6-(3-oxo-3,5,6,7-tetrahydrothiopyrano[3,2-c]pyrazol-2(1H)-yl)-N-phenylpyridine-3-sulfonamide;
• N,N-diethyl-6-(3-oxo-4,6-dihydro-1H-thieno[3,4-c]pyrazol-2(3H)-yl)pyridine-3-sulfonamide;
• N,N-dimethyl-6-(3-oxo-4,6-dihydro-1H-thieno[3,4-c]pyrazol-2(3H)-yl)pyridine-3-sulfonamide;
• 2-[5-(pyrrolidin-1-ylsulfonyl)pyridin-2-yl]-1,2,4,6-tetrahydro-3H-thieno[3,4-c]pyrazol-3-one;
• N-cyclopropyl-6-(3-oxo-4,6-dihydro-1H-thieno[3,4-c]pyrazol-2(3H)-yl)pyridine-3-sulfonamide;
• 6-(3-oxo-4,6-dihydro-1H-thieno[3,4-c]pyrazol-2(3H)-yl)-N-(propan-2-yl)pyridine-3-sulfonamide;
• N-tert-butyl-6-(3-oxo-4,6-dihydro-1H-thieno[3,4-c]pyrazol-2(3H)-yl)pyridine-3-sulfonamide;
• N-(furan-2-ylmethyl)-6-(3-oxo-4,6-dihydro-1H-thieno[3,4-c]pyrazol-2(3H)-yl)pyridine-3-sulfonamide;
• N-cyclopentyl-6-(3-oxo-4,6-dihydro-1H-thieno[3,4-c]pyrazol-2(3H)-yl)pyridine-3-sulfonamide;
• N-methyl-6-(3-oxo-4,6-dihydro-1H-thieno[3,4-c]pyrazol-2(3H)-yl)-N-(pyridin-2-yl)pyridine-3-sulfonamide;
• 2-(pyridin-2-yl)-1,2,4,6-tetrahydro-3H-thieno[3,4-c]pyrazol-3-one;
• 2-[4-(dimethylamino)pyridin-2-yl]-1,2,4,6-tetrahydro-3H-thieno[3,4-c]pyrazol-3-one;
• 2-{5-[(4-benzylpiperidin-1-yl)sulfonyl]pyridin-2-yl}-1,2,4,6-tetrahydro-3H-thieno[3,4-c]pyrazol-3-one;
• 6-(3-oxo-4,6-dihydro-1H-thieno[3,4-c]pyrazol-2(3H)-yl)-N-(pyridin-2-yl)pyridine-3-sulfonamide;
• N-ethyl-6-(3-oxo-1,4,6,7-tetrahydropyrano[4,3-c]pyrazol-2(3H)-yl)-N-phenylpyridine-3-sulfonamide;
• sodium 2-(4-ethylpyridin-2-yl)-2,6-dihydro-4H-thieno[3,4-c]pyrazol-3-olate;
• 2-[5-(azepan-1-ylsulfonyl)pyridin-2-yl]-1,2,4,6-tetrahydro-3H-thieno[3,4-c]pyrazol-3-one;
• sodium 4-benzyl-2-{5-[ethyl(phenyl)sulfamoyl]pyridin-2-yl}-5-oxo-4,5,6,7-tetrahydro-2H-pyrazolo[4,3-b]pyridin-3-olate;
• N-methyl-6-(3-oxo-4,6-dihydro-1H-thieno[3,4-c] pyrazol-2 (3H) -yl) -N- (pyridin-2-ylmethyl)pyridine-3-sulfonamide;
• tert-butyl 2-{5-[ethyl(phenyl)sulfamoyl]pyridin-2-yl}-3-oxo-1,2,3,4,6,7-hexahydro-5H-pyrazolo[4,3-c]pyridine-5-carboxylate;
• 6-(5-acetyl-3-oxo-1,3,4,5,6,7-hexahydro-2H-pyrazolo[4,3-c]pyridin-2-yl)-N-ethyl-N-phenylpyridine-3-sulfonamide;
• tert-butyl 6-(3-oxo-4,6-dihydro-1H-thieno[3,4-c]pyrazol-2(3H)-yl)pyridine-3-carboxylate;
• sodium 2-(4-methylpyridin-2-yl)-2,6-dihydro-4H-thieno[3,4-c]pyrazol-3-olate;
• sodium 2-{5-[tert-butyl(methyl)sulfamoyl]pyridin-2-yl}-2,6-dihydro-4H-thieno[3,4-c]pyrazol-3-olate;
• sodium 2-{5-[tert-butyl(ethyl)sulfamoyl]pyridin-2-yl}-2,6-dihydro-4H-thieno[3,4-c]pyrazol-3-olate;
• sodium 2-(5-methylpyridin-2-yl)-2,6-dihydro-4H-thieno[3,4-c]pyrazol-3-olate;
• sodium 2-[5-(tert-butylcarbamoyl)pyridin-2-yl]-2,6-dihydro-4H-thieno[3,4-c]pyrazol-3-olate;
• sodium 2-(5-methoxypyridin-2-yl)-2,6-dihydro-4H-thieno[3,4-c]pyrazol-3-olate;
• N-methyl-6-(3-oxo-4,6-dihydro-1H-thieno[3,4-c]pyrazol-2(3H)-yl)-N-(pyridin-4-yl)pyridine-3-sulfonamide;
• sodium 2-{5-[cyclopentyl(ethyl)sulfamoyl]pyridin-2-yl}-2,6-dihydro-4H-thieno[3,4-c]pyrazol-3-olate;
• sodium 2-[4-(pyridin-3-ylmethoxy)pyridin-2-yl]-2,6-dihydro-4H-thieno[3,4-c]pyrazol-3-olate;
• methyl 2-{5-[ethyl(phenyl)sulfamoyl]pyridin-2-yl}-3-oxo-1,2,3,4,6,7-hexahydro-5H-pyrazolo[4,3-c]pyridine-5-carboxylate;
• cyclopentyl 6-(3-oxo-4,6-dihydro-1H-thieno[3,4-c]pyrazol-2(3H)-yl)pyridine-3-carboxylate;
• 2-methylpropyl 6-(3-oxo-4,6-dihydro-1H-thieno[3,4-c]pyrazol-2(3H)-yl)pyridine-3-carboxylate;
• 2-[4-(propan-2-yl)pyridin-2-yl]-1,2,4,6-tetrahydro-3H-thieno[3,4-c]pyrazol-3-one;
• sodium 2-[5-(propan-2-yl)pyridin-2-yl]-2,6-dihydro-4H-thieno[3,4-c]pyrazol-3-olate;
• methyl 2-{5-[ethyl(phenyl)sulfamoyl]pyridin-2-yl}-3-oxo-2,3,4,6-tetrahydro-1H-thieno[3,4-c]pyrazole-4-carboxylate;
• propan-2-yl 6-(3-oxo-4,6-dihydro-1H-thieno[3,4-c]pyrazol-2(3H)-yl)pyridine-3-carboxylate;
• sodium 2-{5-[ethyl(phenyl)sulfamoyl]pyridin-2-yl}-6-methoxy-2,4,5,6-tetrahydrocyclopenta[c]pyrazol-3-olate;
• N-ethyl-6-(3-oxo-3,4,5,6-tetrahydrocyclopenta[c]pyrazol-2(1H)-yl)-N-phenylpyridine-3-sulfonamide;
• sodium 2-[4-(pyridin-3-ylmethoxy)pyridin-2-yl]-2,6-dihydro-4H-thieno[3,4-c]pyrazol-3-olate;
• 2,2-dimethylpropyl 6-(3-oxo-4,6-dihydro-1H-thieno[3,4-c]pyrazol-2(3H)-yl)pyridine-3-carboxylate;
• 2-[5-(5-tert-butyl-1,2,4-oxadiazol-3-yl)pyridin-2-yl]-1,2,4,6-tetrahydro-3H-thieno[3,4-c]pyrazol-3-one;
• N-cyclopentyl-N-methyl-6-(3-oxo-4,6-dihydro-1H-thieno[3,4-c]pyrazol-2(3H)-yl)pyridine-3-sulfonamide;
• N-cyclopentyl-N-ethyl-6-(3-oxo-3,4,5,6-tetrahydrocyclopenta[c]pyrazol-2(1H)-yl)pyridine-3-sulfonamide;
• N-cyclopentyl-N-(2,3-dihydroxypropyl)-6-(3-oxo-4,6-dihydro-1H-thieno[3,4-c]pyrazol-2(3H)-yl)pyridine-3-sulfonamide;
• 2,2-dimethylpropyl 6-[5-(methylsulfonyl)-3-oxo-1,3,4,5,6,7-hexahydro-2H-pyrazolo[4,3-c]pyridin-2-yl]pyridine-3-carboxylate;
• 2-[5-(3-tert-butyl-1,2,4-oxadiazol-5-yl)pyridin-2-yl]-1,2,4,6-tetrahydro-3H-thieno[3,4-c]pyrazol-3-one.

11. Process for preparing a compound of formula (I) according to any one of Claims 1 to 10, **characterized in that** a compound of formula (IV), which is in the form of a compound of formula (IVa) or (IVb) or both: 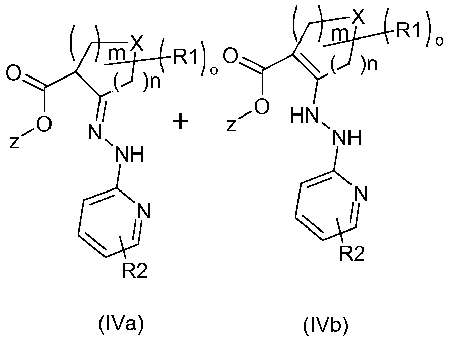 in which X, R1, R2, n, m and are as defined in Claim 1, and z represents an alkyl group,
is reacted with an organic base.

12. Compounds of formulae (IVa) and (IVb) 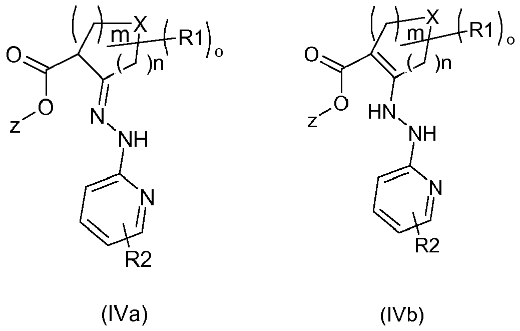 in which R1, R2, n, m and o are as defined in Claim 1, z represents an alkyl group and x represents -CH(R')--N(R')- or a heteroatom such that R' and the heteroatom are as defined in Claim 1.

13. Medicament, **characterized in that** it comprises a compound of formula (I) according to any one of Claims 1 to 10, or an addition salt of this compound with a pharmaceutically acceptable acid of the compound of formula (I).

14. Pharmaceutical composition, **characterized in that** it comprises a compound of formula (I) according to any one of Claims 1 to 10, or a pharmaceutically acceptable salt of this compound, and also at least one pharmaceutically acceptable excipient.

15. Use of a compound of formula (I) according to any one of Claims 1 to 10, for the preparation of a medicament for the treatment/prophylaxis of cardiovascular diseases.

16. Use of a compound of formula (I) according to any one of Claims 1 to 10, for the preparation of a medicament for the treatment/prophylaxis of ischaemia of the lower limbs, cardiac insufficiency, coronary diseases of ischaemic origin, for instance angina pectoris or myocardial infarction, arteriosclerosis, strokes of ischaemic origin, pulmonary hypertension and any pathology caused by partial or total vascular occlusion.

17. Use of a compound of formula (I) according to any one of Claims 1 to 10, for the preparation of a medicament for the treatment/prophylaxis of glaucoma, renal diseases or cerebral diseases of neurodegenerative origin or otherwise, anaemia, or a medicament for promoting cicatrization, or agents for shortening the post-operative convalescence period, or a medicament for treating general fatigue conditions, or alternatively a medicament used for the purpose of obtaining blood in the context of autotransfusions necessary following major surgical interventions such as cranial or chest surgery, or heart, carotid or aortic operations.

18. Use of a compound of formula (I) according to any one of Claims 1 to 10, for the preparation of a medicament for the treatment/prophylaxis of cardiac or peripheral diseases of ischaemic origin via regenerative medicine using stem cells.

19. Combination of a compound of formula (I) according to any one of Claims 1 to 10 with one or more active compound(s) that is (are) useful in the treatment of hypertension, cardiac insufficiency, diabetes and anaemia.
